# EUROPEAN PATENT APPLICATION

(11) **EP 4 393 909 A1**
(43) Date of publication of application: **03.07.2024**
(21) Application number: 22921723.7
(22) Date of filing: 23.12.2022
(51) Int. Cl.: C07D 233/64, A61K 31/4174, A61P 3/00, A61P 9/00, A61P 37/00

(54) **HYDANTOIN COMPOUND AND MEDICAL USE THEREOF**

(30) Priority: 20.01.2022 CN 202210067669; 16.12.2022 CN 202211624621
(71) Applicant: Harbin Medisan Pharmaceutical Co., LTD., Harbin, Heilongjiang 150025 (CN)
(72) Inventor: DAI, Liang, Nanjing, Jiangsu 211198 (CN); SUN, Hongbin, Nanjing, Jiangsu 211198 (CN); XIANG, Jiehao, Nanjing, Jiangsu 211198 (CN); FENG, Zhiqi, Nanjing, Jiangsu 211198 (CN); LIU, Hui, Nanjing, Jiangsu 211198 (CN); XU, Xiangrui, Nanjing, Jiangsu 211198 (CN); YUN, Yinling, Nanjing, Jiangsu 211198 (CN); SUN, Gang, Nanjing, Jiangsu 211198 (CN); LIU, Xin, Nanjing, Jiangsu 211198 (CN); WANG, Yanyan, Nanjing, Jiangsu 211198 (CN); YUAN, Haoliang, Nanjing, Jiangsu 211198 (CN)
(74) Representative: Sun, Yiming
(86) International application number: PCT/CN2022/141359
(87) International publication number: WO 2023/138302

(57) **Abstract**

Provided are a hydantoin compound as represented by formula (I) or a pharmaceutically acceptable salt thereof and the medical use thereof. The compound has a strong agonistic action on PPARα and PPARδ, has a good selectivity to PPARγ, and has good pharmacokinetic properties. The compound or a pharmaceutically acceptable salt, prodrug, deuterated compound or solvate thereof can be used for preparing a PPARα/δ dual agonist, and is used for preparing a drug for preventing or treating diseases mediated by PPARα and/or PPARδ.

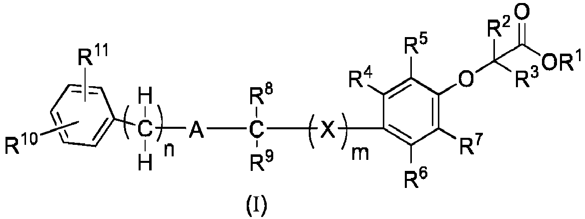

## Description

### TECHNICAL FIELD

The present invention belongs to the field of biomedicines, and particularly relates to a hydantoin compound with dual agonistic activity for PPARα/δ and medical use thereof as a PPARα/δ dual agonist.

### BACKGROUND

Peroxisome proliferator-activated receptors (PPARs) are a family of nuclear receptors, including three subtypes, PPARα, PPAR6, and PPARγ. Studies have shown that activation of PPARs plays a positive role in the improvement of metabolic diseases, cardiovascular and cerebrovascular diseases, inflammatory diseases, autoimmune diseases, neurodegenerative diseases, organ regeneration, retinopathy, or tumors (Mol. Cells., 2012, 33, 217; J. Biomed. Sci., 2017, 24, 5; J. Med. Chem., 2017, 55, 4027; Endocr. J., 2007, 54, 347). The development and application of PPAR agonists is a potential therapeutic strategy for intervention in the diseases described above. However, PPARγ agonists have been shown to have risks of weight gain, edema, fractures, and potential heart failure. Therefore, the development of selective PPARα/δ dual agonists may provide safe and effective new approaches for the treatment of the diseases described above.

Currently, the clinically researched PPARα/δ dual agonist is GFT505 (Elafibranor) developed by Genfit, France. GFT505 was tested in multiple clinical trials in nonalcoholic fatty liver disease, cholestatic cholangitis, and kidney diseases. Unfortunately, the interim analysis results of a phase III clinical trial against nonalcoholic steatohepatitis (NASH) show that GFT505 was substantially ineffective (NCT02704403), and the reason for the poor clinical trial effect may be related to the poor agonistic activity for PPARα/δ, poor metabolic stability, and short halflife of GFT505.

In conclusion, there is an urgent need clinically to develop a PPARα/δ dual agonist with high activity and excellent pharmacokinetic properties.

### SUMMARY

Objective: in order to solve the problems in the existing PPAR agonists, the present invention provides a novel hydantoin compound. The hydantoin compound of the present invention has a potent agonistic effect on PPARα and PPARδ, and has very weak agonistic activity for PPARγ, thereby having a good selectivity and good pharmacokinetic properties. Therefore, the compound and a pharmaceutically acceptable salt, a prodrug, a deuterated compound, or a solvate thereof may be used for preparing a PPARα/δ dual agonist.

Another objective of the present invention is to provide medical use of the hydantoin compound as a PPARα/δ dual agonist. The compound and the pharmaceutically acceptable salt, the prodrug, the deuterated compound, or the solvate thereof may be used for preparing a medicament for the prevention or treatment of a disease mediated by PPARα and/or PPARδ.

Technical scheme: in order to achieve the objectives described above, the present invention provides a hydantoin compound of formula (I) or a pharmaceutically acceptable salt thereof: 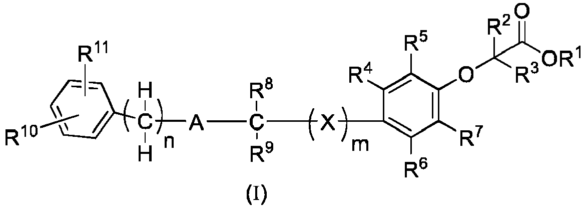
A is selected from 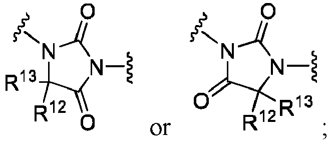
R¹ is selected from H, linear or branched C1-C6 alkyl, C3-C6 cycloalkyl, (CH₂)ₚOR¹⁴, or (CH₂)_{q}NR¹⁵, wherein p = any integer from 2 to 6; q = any integer from 2 to 6; R¹⁴ and R¹⁵ are each independently H, R¹⁶, or C(O)R¹⁷, wherein R¹⁶ and R¹⁷ are each independently linear or branched C1-C6 alkyl or C3-C6 cycloalkyl;
R² and R³ are each independently selected from H or linear or branched C1-C4 alkyl; or R² and R³, together with the carbon atom to which they are bonded, form a 3- to 6-membered cycloalkyl ring;
R⁴, R⁵, R⁶, and R⁷ are each independently selected from H, halogen, OR¹⁸, hydroxy, linear or branched C1-C4 alkyl, trifluoromethyl, methylthio, trifluoromethoxy, trifluoromethylthio, C3-C6 cycloalkyl, cycloalkenyl, heterocycloalkyl, heterocycloalkenyl, alkynyl, phenyl, substituted phenyl, heteroaryl, substituted heteroaryl, fused aryl, or substituted fused aryl; or at least two of substituents in R⁴, R⁵, R⁶, and R⁷, together with the atoms to which they are linked, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹⁸ is selected from linear or branched C1-C4 alkyl, hydroxyalkyl, alkoxyalkyl, alkoxyalkoxyalkyl, C3-C6 cycloalkyl, or alkynylalkoxyalkyl;
X is selected from CH₂, O, or S;
m is selected from any integer from 0 to 4;
n is selected from any integer from 0 to 2;
R⁸ and R⁹ are each independently selected from H, deuterium, linear or branched C1-C4 alkyl, or halogen; or R⁸ and R⁹, together with the carbon atom to which they are bonded, form a 3- to 6-membered cycloalkyl ring;
R¹⁰ and R¹¹ are independently selected from H, hydroxy, halogen, cyano, linear or branched C1-C4 alkyl, trifluoromethyl, methylthio, trifluoromethoxy, trifluoromethylthio, alkylsulfonyl, alkoxy, cycloalkyl, cycloalkenyl, heterocycloalkyl, heterocycloalkenyl, alkynyl, phenyl, substituted phenyl, phenoxy, substituted phenyloxy, heteroaryl, substituted heteroaryl, fused aryl, or substituted fused aryl, wherein the substituted phenyl, substituted phenyloxy, substituted heteroaryl, or substituted fused aryl may be independently substituted with 1 to 2 of the following substituents: halogen, hydroxy, cyano, linear or branched C1-C4 alkyl, trifluoromethyl, methylthio, trifluoromethoxy, trifluoromethylthio, or alkylsulfonyl; or R¹⁰ and R¹¹, together with the atoms to which they are linked, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹² and R¹³ are each independently selected from H, deuterium, and linear or branched C1-C4 alkyl; or R¹² and R¹³, together with the carbon atom to which they are bonded, form a 3- to 6-membered cycloalkyl ring.

Preferably, in the hydantoin compound of formula (I) or the pharmaceutically acceptable salt thereof,
A is selected from 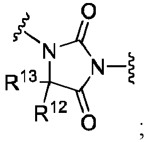
R¹ is selected from H, linear or branched C1-C4 alkyl, acetamidoethyl, or (CH₂)ₚOR¹⁴, wherein p = any integer from 2 to 6; R¹⁴ is selected from linear or branched C1-C4 alkyl;
R² and R³ are each independently selected from H or linear or branched C1-C4 alkyl; or R² and R³, together with the carbon atom to which they are bonded, form a 3- to 6-membered cycloalkyl ring;
R⁴, R⁵, R⁶, and R⁷ are each independently selected from H, halogen, trifluoromethyl, trifluoromethoxy, trifluoromethylthio, OR¹⁸, and linear or branched C1-C4 alkyl;
R¹⁸ is selected from linear or branched C1-C4 alkyl;
X is selected from CH₂;
m is selected from any integer from 0 to 2;
n is selected from 0 or 1;
R⁸ and R⁹ are each independently selected from H, deuterium, linear or branched C1-C4 alkyl, and halogen; or R⁸ and R⁹, together with the carbon atom to which they are bonded, form a 3-to 6-membered cycloalkyl ring;
R¹⁰ and R¹¹ are independently selected from H, halogen, cyano, linear or branched C1-C4 alkyl, trifluoromethyl, methylthio, trifluoromethoxy, trifluoromethylthio, methylsulfonyl, ethylsulfonyl, linear or branched C1-C4 alkoxy, C3-C6 cycloalkyloxy, C3-C6 cycloalkyl, phenyl, substituted phenyl, phenoxy, and substituted phenyloxy, wherein the substituted phenyl or substituted phenyloxy may independently be substituted with 1 to 2 of the following substituents: halogen, cyano, linear or branched C1-C4 alkyl, trifluoromethyl, methylthio, trifluoromethoxy, trifluoromethylthio, or methylsulfonyl;
R¹² and R¹³ are each independently selected from H, deuterium, and linear or branched C1-C4 alkyl.

Further, the hydantoin compound further includes prodrugs, deuterated compounds, or solvates thereof.

In certain more preferred embodiments, the hydantoin compound or the pharmaceutically acceptable salt thereof of the present invention is any one of the compounds shown in Table 1 below:

**Table 1. Structure and nomenclature of compounds**

| Compound No. | Structure | Name |
|---|---|---|
| 1 | 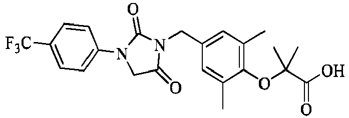 | 2-(4-((2,5-Dioxo-3-(4- (trifluoromethyl)phenyl)imid azolidin-1-yl)methyl)-2,6- dimethylphenoxy)-2- methylpropionic acid |
| 2 | 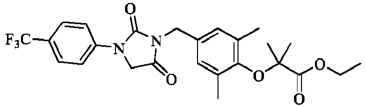 | Ethyl 2-(4-((2,5-dioxo-3-(4- (trifluoromethyl)phenyl)imid azolidin-1-yl)methyl)-2,6- dimethylphenoxy)-2-methylpropionate |
| 3 | 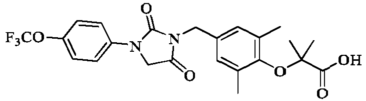 | 2-(4-((2,5-Dioxo-3-(4-(trifluoromethoxy)phenyl)im idazolidin-1-yl)methyl)-2,6-dimethylphenoxy)-2-methylpropionic acid |
| 4 | 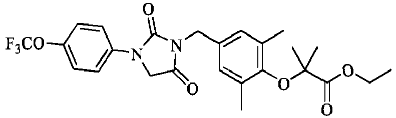 | Ethyl 2-(4-((2,5-dioxo-3-(4-(trifluoromethoxy)phenyl)im idazolidin-1-yl)methyl)-2,6-dimethylphenoxy)-2-methylpropionate |
| 5 | 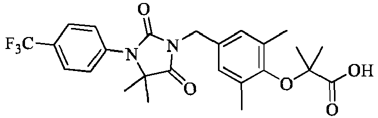 | 2-(4-((4,4-Dimethyl-2,5-dioxo-3-(4-(trifluoromethyl)phenyl)imid azolin-1-yl)methyl)-2,6-dimethylphenoxy)-2-methylpropionic acid |
| 6 | 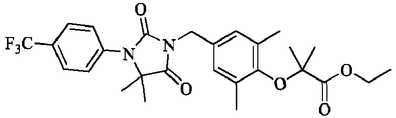 | Ethyl 2-(4-((4,4-dimethyl-2,5-dioxo-3-(4-(trifluoromethyl)phenyl)imid azolin-1-yl)methyl)-2,6-dimethylphenoxy)-2-methylpropionate |
| 7 | 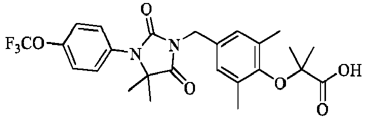 | 2-(4-((4,4-Dimethyl-2, 5-dioxo-3-(4-(trifluoromethyl)phenyl)imid azolin-1-yl)methyl)-2,6-dimethylphenoxy)-2-methylpropionic acid |
| 8 | 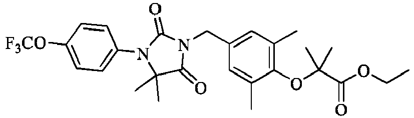 | Ethyl 2-(4-((4,4-dimethyl-2,5-dioxo-3-(4-(trifluoromethoxy)phenyl)im idazolin-1-yl)methyl)-2,6-dimethylphenoxy)-2-methylpropionate |
| 9 | 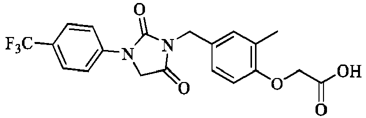 | 2-(4-((2,5-Dioxo-3-(4-(trifluoromethyl)phenyl)imid azolidin-1-yl)methyl)-2-methylphenoxy)acetic acid |
| 10 | 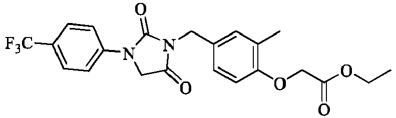 | Ethyl 2-(4-((2,5-dioxo-3-(4-(trifluoromethyl)phenyl)imid azolidin-1-yl)methyl)-2-methylphenoxy)acetate |
| 11 | 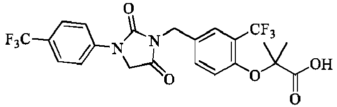 | 2-(4-((2,5-Dioxo-3-(4-(trifluoromethyl)phenyl)imid azolidin-1-yl)methyl)-2-(trifluoromethyl)phenoxy)-2-methylpropionic acid |
| 12 | 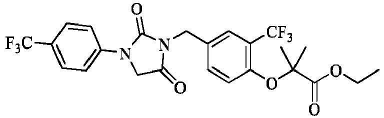 | Ethyl 2-(4-((2,5-dioxo-3-(4-(trifluoromethyl)phenyl)imid azolidin-1-yl)methyl)-2-(trifluoromethyl)phenoxy)-2-methylpropionate |
| 13 | 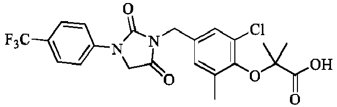 | 2-(2-Chloro-4-((2, 5-dioxo-3 - (4-(trifluoromethyl)phenyl)imid azolidin-1-yl)methyl)-6-methylphenoxy)-2-methylpropionic acid |
| 14 | 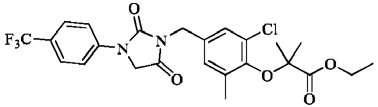 | Ethyl 2-(2-chloro-4-((2,5-dioxo-3-(4-(trifluoromethyl)phenyl)imid azolidin-1-yl)methyl)-6-methylphenoxy)-2-methylpropionate |
| 15 | 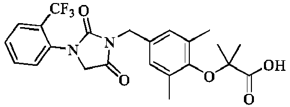 | 2-(4-((2,5-Dioxo-3-(2-(trifluoromethyl)phenyl)imid azolidin-1-yl)methyl)-2,6-dimethylphenoxy)-2-methylpropionic acid |
| 16 | 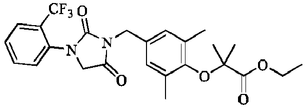 | Ethyl 2-(4-((2,5-dioxo-3-(2-(trifluoromethyl)phenyl)imid azolidin-1-yl)methyl)-2,6-dimethylphenoxy)-2-methylpropionate |
| 17 | 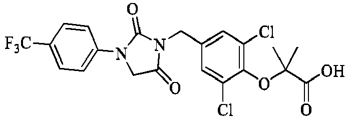 | 2-(2,6-Dichloro-4-((2,5-dioxo-3-(4- (trifluoromethyl)phenyl)imid azolidin-1- yl)methyl)phenoxy)-2-methylpropionic acid |
| 18 | 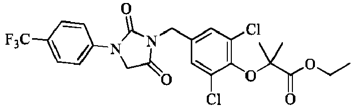 | Ethyl 2-(2,6-dichloro-4-((2,5-dioxo-3-(4-(trifluoromethyl)phenyl)imid azolidin-1-yl)methyl)phenoxy)-2-methylpropionate |
| 19 | 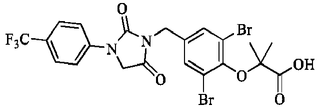 | 2-(2,6-Dibromo-4-((2,5-dioxo-3-(4-(trifluoromethyl)phenyl)imid azolidin-1-yl)methyl)phenoxy)-2-methylpropionic acid |
| 20 | 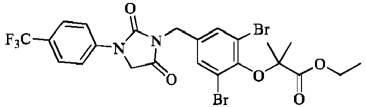 | Ethyl 2-(2,6-dibromo-4-((2,5-dioxo-3-(4-(trifluoromethyl)phenyl)imid azolidin-1-yl)methyl)phenoxy)-2-methylpropionate |
| 21 | 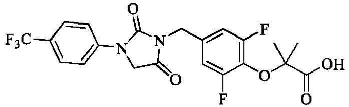 | 2-(4-((2,5-Dioxo-3-(4-(trifluoromethyl)phenyl)imid azolin-1-yl)methyl)-2,6-difluorophenoxy)-2-methylpropionic acid |
| 22 | 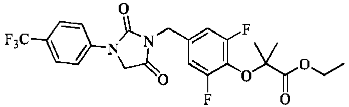 | Ethyl 2-(4-((2,5-dioxo-3-(4-(trifluoromethyl)phenyl)imid azolin-1-yl)methyl)-2,6-difluorophenoxy)-2-methylpropionate |
| 23 | 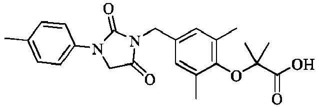 | 2-(4-((2,5-Dioxo-3-(p-tolyl)imidazolin-1-yl)methyl)-2,6-dimethylphenoxy)-2-methylpropionic acid |
| 24 | 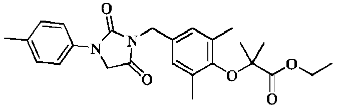 | Ethyl 2-(4-((2,5-dioxo-3-(p-tolyl)imidazolin-1-yl)methyl)-2,6-dimethylphenoxy)-2-methylpropionate |
| 25 | 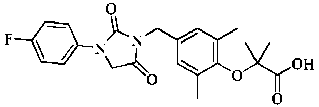 | 2-(4-((3-(4-Fluorophenyl)-2,5-dioxoimidazolin-1-yl)methyl)-2,6-dimethylphenoxy)-2-methylpropionic acid |
| 26 | 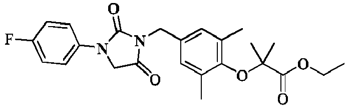 | Ethyl 2-(4-((3-(4-fluorophenyl)-2,5-dioxoimidazolin-1-yl)methyl)-2,6-dimethylphenoxy)-2-methylpropionate |
| 27 | 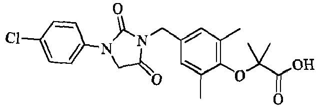 | 2-(4-((3 -(4-Chlorophenyl)-2,5-dioxoimidazolin-1- yl)methyl)-2,6-dimethylphenoxy)-2-methylpropionic acid |
| 28 | 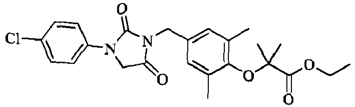 | Ethyl 2-(4-((3-(4-chlorophenyl)-2,5-dioxoimidazolin-1-yl)methyl)-2,6-dimethylphenoxy)-2-methylpropionate |
| 29 | 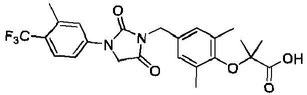 | 2-(2,6-Dimethyl-4-((3-(3-methyl-4-(trifluoromethyl)phenyl)-2,5-dioxoimidazolin-1-yl)methyl)phenoxy)-2-methylpropionic acid |
| 30 | 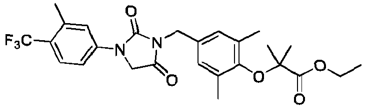 | Ethyl 2-(2,6-dimethyl-4-((3-(3-methyl-4-(trifluoromethyl)phenyl)-2,5-dioxoimidazolin-1-yl)methyl)phenoxy)-2-methylpropionate |
| 31 | 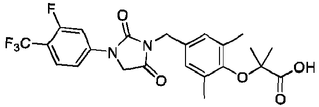 | 2-(2,6-Dimethyl-4-((3-(3-fluoro-4-(trifluoromethyl)phenyl)-2,5-dioxoimidazolin-1-yl)methyl)phenoxy)-2-methylpropionic acid |
| 32 | 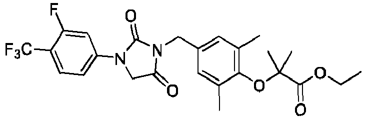 | Ethyl 2-(2,6-dimethyl-4-((3-(3-chloro-4-(trifluoromethyl)phenyl)-2,5-dioxoimidazolin-1-yl)methyl)phenoxy)-2-methylpropionate |
| 33 | 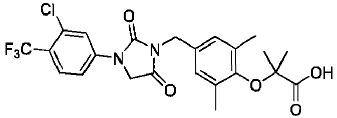 | 2-(2,6-Dimethyl-4-((3-(3-chloro-4-(trifluoromethyl)phenyl)-2,5-dioxoimidazolin-1-yl)methyl)phenoxy)-2-methylpropionic acid |
| 34 | 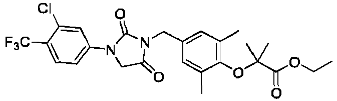 | Ethyl 2-(2,6-dimethyl-4-((3-(3-chloro-4-(trifluoromethyl)phenyl)-2,5-dioxoimidazolin-1-yl)methyl)phenoxy)-2-methylpropionate |
| 35 | 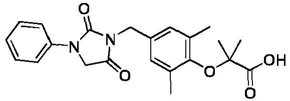 | 2-(4-((2,5-Dioxo-3- phenylimidazolin-1-yl)methyl)-2,6-dimethylphenoxy)-2-methylpropionic acid |
| 36 | 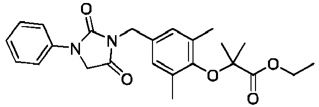 | Ethyl 2-(4-((2,5-dioxo-3-phenylimidazolin-1-yl)methyl)-2,6-dimethylphenoxy)-2-methylpropionate |
| 37 | 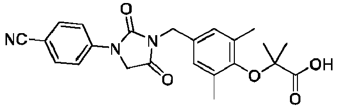 | 2-(4-((3 -(4-Cyanobenzene)-2,5-dioxoimidazolin-1-yl)methyl)-2,6-dimethylphenoxy)-2-methylpropionic acid |
| 38 | 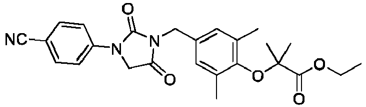 | Ethyl 2-(4-((3-(4-cyanobenzene)-2,5-dioxoimidazolin-1-yl)methyl)-2,6-dimethylphenoxy)-2-methylpropionate |
| 39 | 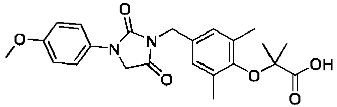 | 2-(4-((3-(4-Methoxyphenyl)-2,5-dioxoimidazolin-1-yl)methyl)-2,6-dimethylphenoxy)-2-methylpropionic acid |
| 40 | 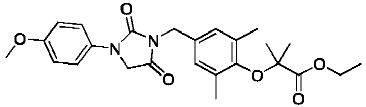 | Ethyl 2-(4-((3-(4-methoxyphenyl)-2,5-dioxoimidazolin-1-yl)methyl)-2,6-dimethylphenoxy)-2-methylpropionate |
| 41 | 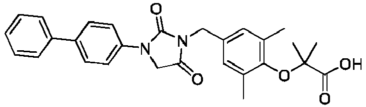 | 2-(4-((3-(4-Biphenyl-2,5-dioxoimidazolin-1-yl)methyl)-2,6-dimethylphenoxy)-2-methylpropionic acid |
| 42 | 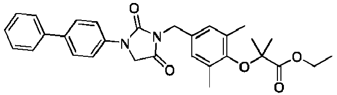 | Ethyl 2-(4-((3-(4-biphenyl-2,5-dioxoimidazolin-1-yl)methyl)-2,6-dimethylphenoxy)-2-methylpropionate |
| 43 | 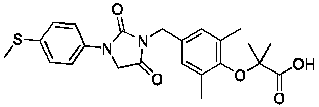 | 2-(4-((3-(4-Methylthiophenyl)-2,5-dioxoimidazolin-1-yl)methyl)-2,6-dimethylphenoxy)-2-methylpropionic acid |
| 44 | 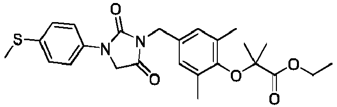 | Ethyl 2-(4-((3-(4-methylthiophenyl)-2,5-dioxoimidazolin-1-yl)methyl)-2,6-dimethylphenoxy)-2-methylpropionate |
| 45 | 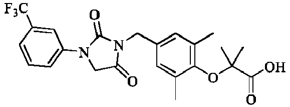 | 2-(4-((2,5-Dioxo-3-(3-(trifluoromethyl)phenyl)imid azolin-1-yl)methyl)-2,6-dimethylphenoxy)-2-methylpropionic acid |
| 46 | 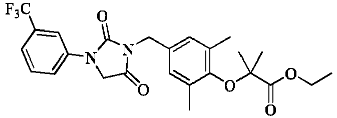 | Ethyl 2-(4-((2,5-dioxo-3-(3-(trifluoromethyl)phenyl)imid azolin-1-yl)methyl)-2,6-dimethylphenoxy)-2-methylpropionate |
| 47 | 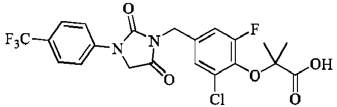 | 2-(2-Chloro-4-((2, 5-dioxo-3- (4-(trifluoromethyl)phenyl)imid azolin-1-yl)methyl)-6-fluorophenoxy)-2-methylpropionic acid |
| 48 | 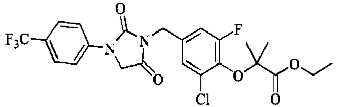 | Ethyl 2-(2-chloro-4-((2,5-dioxo-3-(4-(trifluoromethyl)phenyl)imid azolin-1-yl)methyl)-6-fluorophenoxy)-2-methylpropionate |
| 49 | 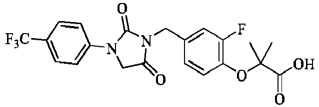 | 2-(4-((2,5-Dioxo-3-(4-(trifluoromethyl)phenyl)imid azolin-1-yl)methyl)-2-fluorophenoxy)-2-methylpropionic acid |
| 50 | 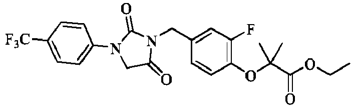 | Ethyl 2-(4-((2,5-dioxo-3-(4-(trifluoromethyl)phenyl)imid azolin-1-yl)methyl)-2-fluorophenoxy)-2-methylpropionate |
| 51 | 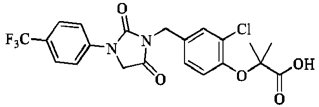 | 2-(4-((2,5-Dioxo-3-(4-(trifluoromethyl)phenyl)imid azolin-1-yl)methyl)-2-chlorophenoxy)-2-methylpropionic acid |
| 52 | 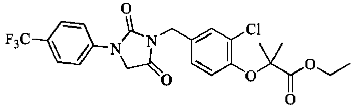 | Ethyl 2-(4-((2,5-dioxo-3-(4-(trifluoromethyl)phenyl)imid azolin-1-yl)methyl)-2-chlorophenoxy)-2-methylpropionate |
| 53 | 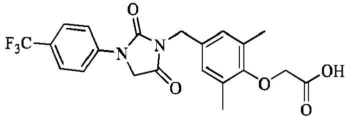 | 2-(4-((2,5-Dioxo-3-(4-(trifluoromethyl)phenyl)imid azolin-1-yl)methyl)-2,6-dimethylphenoxy)acetic acid |
| 54 | 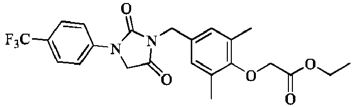 | Ethyl 2-(4-((2,5-dioxo-3-(4-(trifluoromethyl)phenyl)imid azolin-1-yl)methyl)-2,6-dimethylphenoxy)acetate |
| 55 | 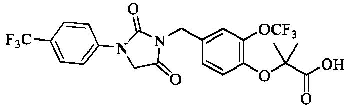 | 2-(4-((2,5-Dioxo-3-(4-(trifluoromethyl)phenyl)imid azolin-1-yl)methyl)-2-(trifluoromethoxy)phenoxy)-2-methylpropionic acid |
| 56 | 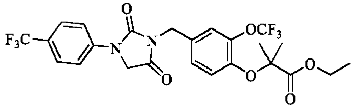 | Ethyl 2-(4-((2,5-dioxo-3-(4-(trifluoromethyl)phenyl)imid azolin-1-yl)methyl)-2- (trifluoromethoxy)phenoxy)-2-methylpropionate |
| 57 | 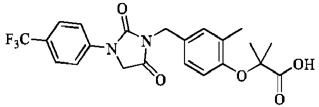 | 2-(4-((2,5-Dioxo-3-(4-(trifluoromethyl)phenyl)imid azolin-1-yl)methyl)-2-methylphenoxy)-2-methylpropionic acid |
| 58 | 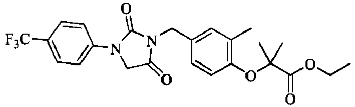 | Ethyl 2-(4-((2,5-dioxo-3-(4-(trifluoromethyl)phenyl)imid azolin-1-yl)methyl)-2-methylphenoxy)-2-methylpropionate |
| 59 | 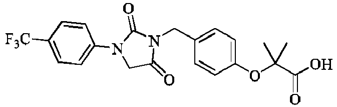 | 2-(4-((2,5-Dioxo-3-(4-(trifluoromethyl)phenyl)imid azolin-1-yl)methyl)phenoxy)-2-methylpropionic acid |
| 60 | 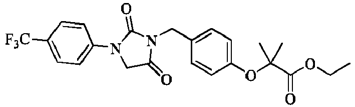 | Ethyl 2-(4-((2,5-dioxo-3-(4-(trifluoromethyl)phenyl)imid azolin-1-yl)methyl)phenoxy)-2-methylpropionate |
| 61 | 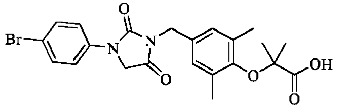 | 2-(4-((3-(4-Bromophenyl)-2,5-dioxoimidazolin-1-yl)methyl)-2,6-dimethylphenoxy)-2-methylpropionic acid |
| 62 | 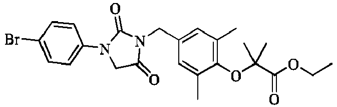 | Ethyl 2-(4-((3-(4-bromophenyl)-2,5-dioxoimidazolin-1-yl)methyl)-2,6-dimethylphenoxy)-2-methylpropionate |
| 63 | 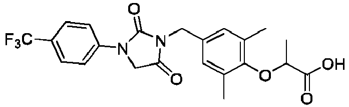 | 2-(4-((2,5-Dioxo-3-(4-(trifluoromethyl)phenyl)imid azolin-1-yl)methyl)-2,6-dimethylphenoxy)propionic acid |
| 64 | 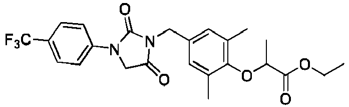 | Ethyl 2-(4-((2,5-dioxo-3-(4-(trifluoromethyl)phenyl)imid azolin-1-yl)methyl)-2,6-dimethylphenoxy)propionate |
| 65 | 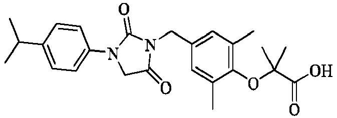 | 2-(4-((3-(4-Isopropylphenyl)-2,5-dioxoimidazolin-1-yl)methyl)-2,6-dimethylphenoxy)-2-methylpropionic acid |
| 66 | 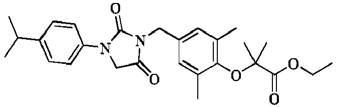 | Ethyl 2-(4-((3-(4-isopropylphenyl)-2,5- dioxoimidazolin-1-yl)methyl)-2,6-dimethylphenoxy)-2-methylpropionate |
| 67 | 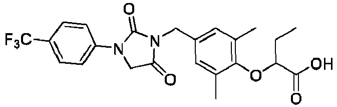 | 2-(4-((2,5-Dioxo-3-(4-(trifluoromethyl)phenyl)imid azolin-1-yl)methyl)-2,6-dimethylphenoxy)butyric acid |
| 68 | 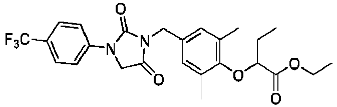 | Ethyl 2-(4-((2,5-dioxo-3-(4-(trifluoromethyl)phenyl)imid azolin-1-yl)methyl)-2,6-dimethylphenoxy)butyrate |
| 69 | 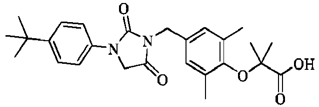 | 2-(4-((3-(4-(*tert-*Butyl)phenyl)-2,5-dioxoimidazolin-1-yl)methyl)-2,6-dimethylphenoxy)-2-methylpropionic acid |
| 70 | 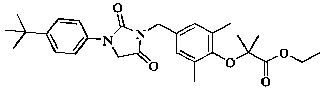 | Ethyl 2-(4-((3-(4-(*tert-*butyl)phenyl)-2,5-dioxoimidazolin-1-yl)methyl)-2,6-dimethylphenoxy)-2-methylpropionate |
| 71 | 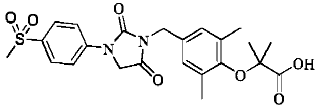 | 2-(2,6-Dimethyl-4-((3-(4-(methylsulfonyl)phenyl)-2,5-dioxoimidazolin-1-yl)methyl)phenoxy)-2-methylpropionic acid |
| 72 | 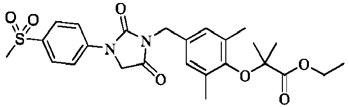 | Ethyl 2-(2,6-dimethyl-4-((3-(4-(methylsulfonyl)phenyl)-2,5-dioxoimidazolin-1-yl)methyl)phenoxy)-2-methylpropionate |
| 73 | 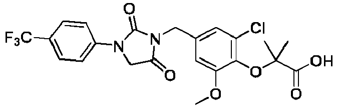 | 2-(2-Chloro-4-((2, 5-dioxo-3- (4- (trifluoromethyl)phenyl)imid azolin-1-yl)methyl)-6- methoxyphenoxy)-2- methylpropionic acid |
| 74 | 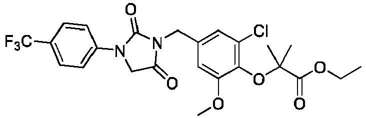 | Ethyl 2-(2-chloro-4-((2,5- dioxo-3-(4- (trifluoromethyl)phenyl)imid azolin-1-yl)methyl)-6- methoxyphenoxy)-2- methylpropionate |
| 75 | 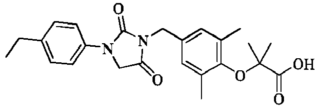 | 2-(4-(3-(4-Ethylphenyl)-2,5- dioxoimidazolidin-1- yl)methyl)-2,6- dimethylphenoxy-2- methylpropionic acid |
| 76 | 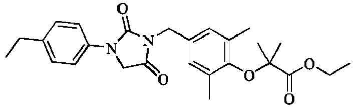 | Ethyl 2-(4-(3-(4- ethylphenyl)-2,5- dioxoimidazolidin-1- yl)methyl)-2,6- dimethylphenoxy-2-methylpropionate |
| 77 | 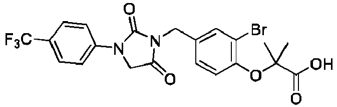 | 2-(2-Bromo-4-((2,5-dioxo-3-(4-trifluoromethyl)phenyl)imid azolidin-1-yl)methylphenoxy)-2-methylpropionic acid |
| 78 | 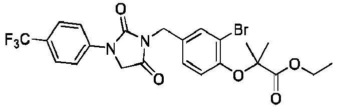 | Ethyl 2-(2-bromo-4-((2,5-dioxo-3-(4-trifluoromethyl)phenyl)imid azolidin-1-yl)methylphenoxy)-2-methylpropionate |
| 79 | 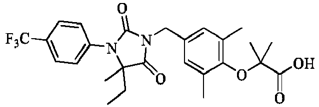 | 2-(4-((4-Ethyl-4-methyl-2,5-dioxo-3-(4-(trifluoromethyl)phenyl)imid azolin-1-yl)methyl)-2,6-dimethylphenoxy)-2-methylpropionic acid |
| 80 | 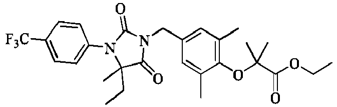 | Ethyl 2-(4-((4-ethyl-4-methyl-2,5-dioxo-3-(4-(trifluoromethyl)phenyl)imid azolin-1-yl)methyl)-2,6-dimethylphenoxy)-2-methylpropionate |
| 81 | 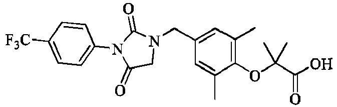 | 2-(4-((2,4-Dioxo-3-(4-(trifluoromethyl)phenyl)imid azolidin-1-yl)methyl)-2,6-dimethylphenoxy)-2-methylpropionic acid |
| 82 | 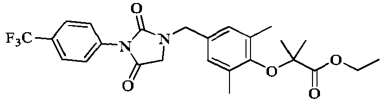 | Ethyl 2-(4-((2,4-dioxo-3-(4-(trifluoromethyl)phenyl)imid azolidin-1-yl)methyl)-2,6-dimethylphenoxy)-2-methylpropionate |
| 93 | 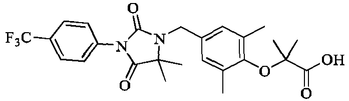 | 2-(4-((4,4-Dimethyl-2,5-dioxo-3-(4-(trifluoromethoxy)phenyl)im idazolidin-1-yl)methyl)-2,6-dimethylphenoxy)-2-methylpropionic acid |
| 84 | 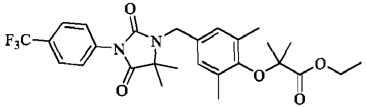 | Ethyl 2-(4-((4,4-dimethyl-2,5-dioxo-3-(4-(trifluoromethoxy)phenyl)im idazolidin-1-yl)methyl)-2,6-dimethylphenoxy)-2-methylpropionate |
| 85 | 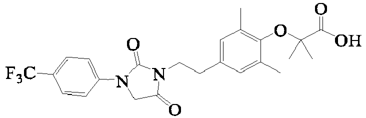 | 2-(4-(2-(2,5-Dioxo-3-(4-(trifluoromethyl)phenyl)imid azolidin-1-yl)ethyl)-2,6-dimethylphenoxy)-2-methylpropionic acid |
| 86 | 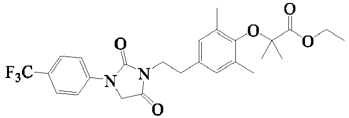 | Ethyl 2-(4-(2-(2,5-dioxo-3- (4- (trifluoromethyl)phenyl)imid azolidin-1-yl)ethyl)-2,6-dimethylphenoxy)-2-methylpropionate |
| 87 | 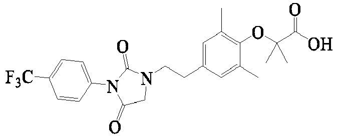 | 2-(4-(2-(2,4-Dioxo-3-(4-(trifluoromethyl)phenyl)imid azolidin-1-yl)ethyl)-2,6-dimethylphenoxy)-2-methylpropionic acid |
| 88 | 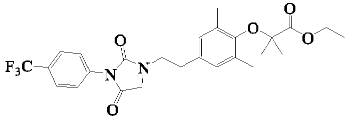 | Ethyl 2-(4-(2-(2,4-dioxo-3-(4- (trifluoromethyl)phenyl)imid azolidin-1-yl)ethyl)-2,6-dimethylphenoxy)-2-methylpropionate |
| 89 | 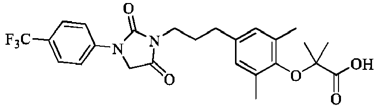 | 2-(4-(3-(2,5-Dioxo-3-(4- (trifluoromethyl)phenyl)imid azolidin-1-yl)propyl)-2,6-dimethylphenoxy)-2-methylpropionic acid |
| 90 | 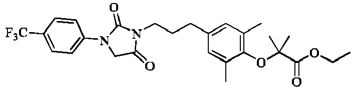 | Ethyl 2-(4-(3-(2,5-dioxo-3-(4- (trifluoromethyl)phenyl)imid azolidin-1-yl)propyl)-2,6-dimethylphenoxy)-2-methylpropionate |
| 91 | 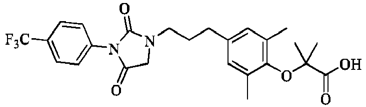 | 2-(4-(3-(2,4-Dioxo-3-(4- (trifluoromethyl)phenyl)imid azolidin-1-yl)propyl)-2,6- dimethylphenoxy)-2-methylpropionic acid |
| 92 | 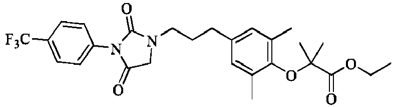 | Ethyl 2-(4-(3-(2,4-dioxo-3-(4- (trifluoromethyl)phenyl)imid azolidin-1-yl)propyl)-2,6-dimethylphenoxy)-2-methylpropionate |
| 93 | 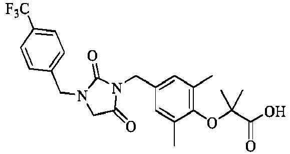 | 2-(4-((2,5-Dioxo-3-(4-(trifluoromethyl)phenyl)imid azolidin-1-yl)methyl)-2,6-dimethylphenoxy)-2-methylpropionic acid |
| 94 | 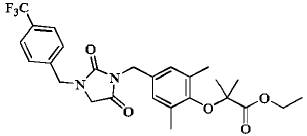 | Ethyl 2-(4-((2,5-dioxo-3-(4- (trifluoromethyl)phenyl)imid azolidin-1-yl)methyl)-2,6- dimethylphenoxy)-2- methylpropionate |
| 95 | 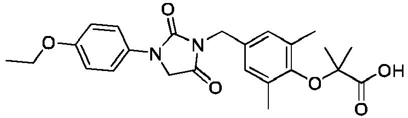 | 2-(4-(3-(4-Ethoxyphenyl)-2,5-dioxoimidazolidin-1-yl)methyl)-2,6- dimethylphenoxy)-2-methylpropionic acid |
| 96 | 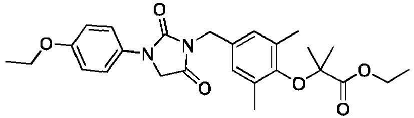 | Ethyl 2-(4-(3-(4-ethoxyphenyl)-2,5- dioxoimidazolidin-1- yl)methyl)-2,6-dimethylphenoxy)-2- methylpropionate |
| 97 | 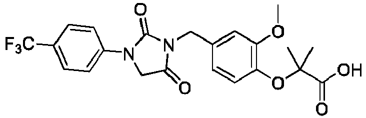 | 2-(4-(2,5-Dioxo-3-(4- trifluoromethylphenyl)imida zolidin-1-yl)methyl)-2- methoxyphenoxy)-2- methylpropionic acid |
| 98 | 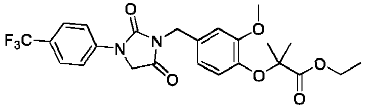 | Ethyl 2-(4-(2,5-dioxo-3-(4- trifluoromethylphenyl)imida zolidin-1-yl)methyl)-2- methoxyphenoxy)-2- methylpropionate |
| 99 | 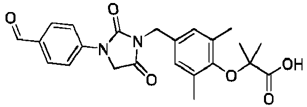 | 2-(4-(3-(4-Formylphenyl)- 2,5-dioxoimidazolidin-1- yl)methyl)-2,6- dimethylphenoxy)-2- methylpropionic acid |
| 100 | 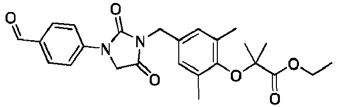 | Ethyl 2-(4-(3-(4- formylphenyl)-2,5- dioxoimidazolidin-1- yl)methyl)-2,6- dimethylphenoxy)-2-methylpropionate |
| 101 | 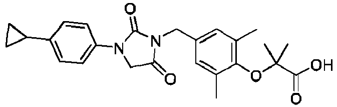 | 2-(3-(4-(Cyclopropylphenyl)-2,5-dioxoimidazolidin-1-yl)methyl)-2,6-dimethylphenoxy)-2-methylpropionic acid |
| 102 | 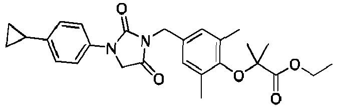 | Ethyl 2-(4-(3-(4-cyclopropylphenyl)-2,5-dioxoimidazolidin-1-yl)methyl)-2,6-dimethylphenoxy)-2-methylpropionate |
| 103 | 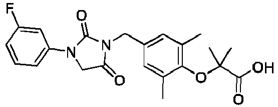 | 2-(4-(3-Fluorophenyl)-2,5-dioxoimidazolidin-1-yl)methyl)-2,6-dimethylphenoxy-2-methylpropionic acid |
| 104 | 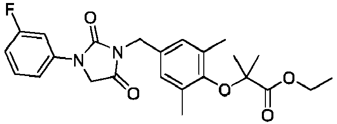 | Ethyl 2-(4-(3-(3-fluorophenyl)-2,5-dioxoimidazolidin-1-yl)methyl)-2,6-dimethylphenoxy)-2-methylpropionate |
| 105 | 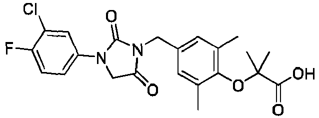 | 2-(3-Chloro-4-fluorophenyl)-2,5-dioxoimidazolidin-1-yl)methyl-2,6-dimethylphenoxy-2-methylpropionic acid |
| 106 | 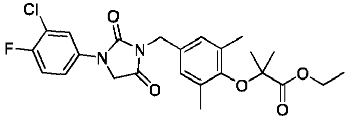 | Ethyl 2-(4-(3-chloro-4-fluorophenyl)-2,5-dioxoimidazolin-1-yl)methyl)-2,6-dimethylphenoxy-2-methylpropionate |
| 107 | 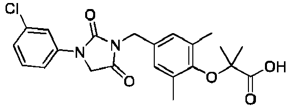 | 2-(4-((3-(3-Chlorophenyl)-2,5-dioxoimidazolidin-1-yl)methyl)-2,6-dimethylphenoxy-2-methylpropionic acid |
| 108 | 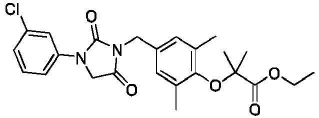 | Ethyl 2-(4-(3-chlorophenyl)-2,5-dioxoimidazolidin-1-yl)methyl)-2,6-dimethylphenoxy)-2-methylpropionate |
| 109 | 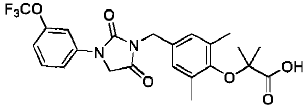 | 2-(4-(2,5-Dioxo-3-(3- trifluoromethoxyphenyl)imi dazolidin-1-yl)methyl)-2,6-dimethylphenoxy)-2-methylpropionic acid |
| 110 | 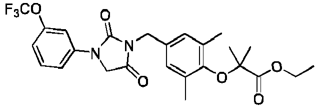 | Ethyl 2-(4-(2,5-dioxo-3-(3-(trifluoromethoxyphenyl)imi dazolidin-1-yl)methyl)-2,6-dimethylphenoxy)-2-methylpropionate |
| 111 | 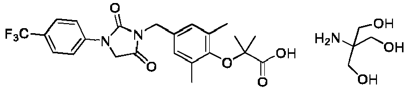 | Tromethamine 2-(4-(2,5-Dioxo-3-(4-trifluoromethylphenyl)imida zolidin-1-yl)methyl)-2,6-dimethylphenoxy)-2-methylpropionate |
| 112 | 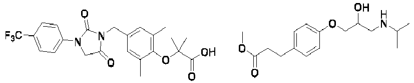 | Esmolol 2-(4-(2,5-dioxo-3-(4-(trifluoromethyl)phenyl)imid azolidin-1-yl)methyl)-2,6-dimethylphenoxy)-2-methylpropionate |
| 113 | 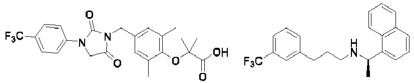 | Cinacalcet 2-(4-(2,5-dioxo-3-(4-trifluoromethylphenyl)imida zolidin-1-yl)methyl)-2,6-dimethylphenoxy)-2-methylpropionate |
| 114 | 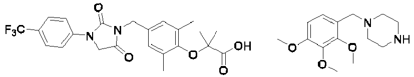 | Trimetazidine 2-(4-(2,5-dioxo-3-(4-trifluoromethylphenyl)imida zolin-1-yl)methyl)-2,6-dimethylphenoxy)-2-methylpropionate |
| 115 | 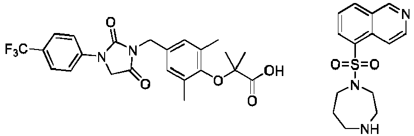 | Fasudil 2-(4-((2,5-dioxo-3-(4-(trifluoromethyl)phenyl)imid azolidinoxy-1-yl)methyl)-2,6-dimethylphenoxy)-2-methylpropionate |
| 116 | 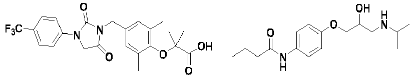 | Acebutolol 2-(2,5-dioxo-3-(4-(trifluoromethyl)phenyl)imid azolidin-1-yl)methyl)-2,6-dimethylphenoxy)-2-methylpropionate |
| 117 | 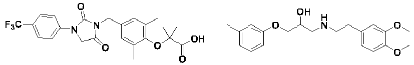 | Bevantolol 2-(4-(2,5-dioxo-3-(4-(trifluoromethyl)phenyl)imid azolidin-1-yl)methyl)-2,6-dimethylphenoxy)-2-methylpropionate |
| 118 | 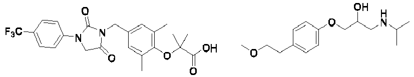 | Metoprolol 2-(4-(2,5-dioxo-3-(4-(trifluoromethyl)phenyl)imid azolidin-1-yl)methyl)-2,6-dimethylphenoxy)-2-methylpropionate |
| 119 | 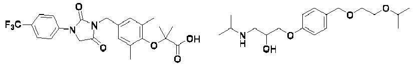 | Bisoprolol 2-(4-((2,5-dioxo-3-(4-(trifluoromethyl)phenyl))imi dazolin-1-yl)methyl)-2,6-dimethylphenoxy)-2-methylpropionate |
| 120 | 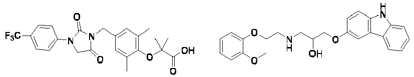 | Carvedilol 2-(4-(2,5-dioxo-3-(4-(trifluoromethyl)phenyl)imid azolidin-1-yl)methyl)-2,6-dimethylphenoxy)-2-methylpropionate |
| 121 | 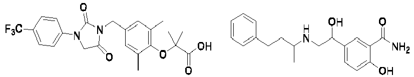 | Labetalol 2-(4-(2,5-dioxo-3-(4-(trifluoromethyl)phenyl)imid azolidin-1-yl)methyl)-2,6-dimethylphenoxy)-2-methylpropionate |
| 122 | 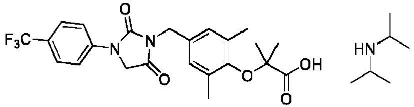 | Diisopropylamine 2-(4-(2,5-dioxo-3-(4-(trifluoromethyl)phenyl)imid azolidin-1-yl)methyl)-2,6-dimethylphenoxy-2-methylpropionate |
| 123 | 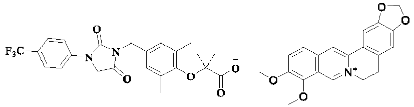 | Berberine 2-(4,5-dioxo-3-(4-(trifluoromethylphenyl)imid azolidin-1-yl)methyl)-2,6-dimethylphenoxy)-2-methylpropionate |

The hydantoin compound or the pharmaceutically acceptable salt, the prodrug, the deuterated compound, or the solvate thereof described herein has a potent PPARα/δ dual agonistic effect, so that it may be used for preparing a PPARα/δ dual agonist.

The hydantoin compound or the pharmaceutically acceptable salt, the prodrug, the deuterated compound, or the solvate thereof described herein may be used for preparing a medicament for the prevention or treatment of a disease mediated by PPARα and/or PPARδ.

Specifically, the compound of the present invention may be used for preparing a medicament for the prevention and treatment of the following diseases mediated by PPARα and/or PPARδ.

The compound of the present invention may be used for preventing and treating metabolic diseases and cardiovascular and cerebrovascular diseases, including: insulin resistance, metabolic syndrome, type 1 or type 2 diabetes, hyperlipidemia, obesity, lipoma, painful lipomatosis, arteriosclerosis, myocardial ischemia, myocardial infarction, arrhythmia, coronary heart disease, hypertension, heart failure, myocardial hypertrophy, myocarditis, diabetic complications (including diabetic cardiomyopathy, diabetic nephropathy, diabetic ulcer, retinopathy, neuropathy, and the like), nonalcoholic fatty liver, nonalcoholic steatohepatitis, alcoholic fatty liver, liver cirrhosis, hyperuricemia, gout, osteoporosis, polycystic ovary syndrome (PCOS), stroke or cerebral infarction, and the like.

The compound of the present invention may be used for preventing and treating inflammatory diseases, autoimmune diseases, organ fibrosis diseases, neurodegenerative diseases, or secondary diseases caused by pathogen infections, including: primary biliary cholangitis (PBC), primary sclerosing cholangitis (PSC), hepatic fibrosis, idiopathic pulmonary fibrosis, cystic fibrosis lung disease, interstitial pneumonia, tuberculosis, inflammatory bowel diseases (such as Crohn's disease and ulcerative colitis), Behcet's disease, asthma, chronic obstructive pulmonary disease, chronic bronchitis, emphysema, bronchiolitis obliterans, allergic rhinitis, chronic rhinitis, sinusitis, systemic lupus erythematosus, rheumatoid arthritis, spondyloarthritis, osteoarthritis, synovitis, tendonitis, thromboangiitis obliterans, phlebitis, intermittent claudication, keloid, psoriasis, ichthyosis, bullous pemphigoid, dermatitis, contact dermatitis, pancreatitis, chronic nephritis, cystitis, meningitis, gastritis, septicemia, pyoderma gangrenosum, uveitis, Parkinson's disease, Alzheimer's disease, α-synucleinopathies, depression, multiple sclerosis, amyotrophic lateral sclerosis, fibromyalgia syndrome, neuralgia, Down's syndrome, Hallervorden-Spatz disease, Huntington's chorea, Wilson disease, or the like.

The compound of the present invention may be used for treating and modulating mitochondrial dysfunction and disorder diseases, including: myasthenia, myoclonus, exercise intolerance, Kearns-Sayre syndrome, chronic fatigue syndrome, Leigh's syndrome, mitochondrial myopathy-encephalopathy-hyperlactacidemia, stroke syndrome or stroke-like episodes, Duchenne muscular dystrophy, becker muscular dystrophy, Friedreich's ataxia, or the like.

The compound of the present invention may be used for treating tumors, including: bone cancer, acute myeloid leukemia, chronic myeloid leukemia, acute lymphocytic leukemia, chronic lymphocytic leukemia, myeloproliferative diseases, multiple myeloma, myelodysplastic syndrome, Hodgkin's lymphoma, non-Hodgkin's lymphoma, hemangioma, granuloma, xanthoma, meningosarcoma, glioma, astrocytoma, medulloblastoma, ependymoma, germ cell tumor (pinealoma), glioblastoma multiforme, oligodendroglioma, schwannoma, retinoblastoma, fibroneuroma, sarcoma, esophageal cancer, gastric cancer, pancreatic cancer, colorectal cancer, colon cancer, rectal cancer, renal cancer, prostate cancer, lymphoma, testicular cancer, interstitial cell carcinoma, lung cancer, liver cancer, skin cancer, malignant melanoma, basal cell carcinoma, or the like.

In certain embodiments, the hydantoin compound of the present invention may be used as a pharmaceutically acceptable salt. The salt may be a salt formed by the compound of the present invention with a metal (including sodium, potassium, calcium, magnesium, and the like) ion or a pharmaceutically acceptable amine (including ethylenediamine, ethanolamine, tromethamine, diisopropylamine, metformin, berberine, and the like) or an ammonium ion.

The present invention also provides a pharmaceutical composition for preventing or treating a disease mediated by PPARα and/or PPAR6, wherein the pharmaceutical composition comprises a therapeutically effective amount of the hydantoin compound of formula (I) or the pharmaceutically acceptable salt, the prodrug, the deuterated compound, or the solvate thereof shown in Table 1 described herein as an active ingredient, and a pharmaceutically acceptable carrier. The carrier which may be arbitrarily mixed may be changed depending on the dosage form, administration form, and the like. Examples of carriers include excipients, binders, disintegrants, lubricants, corrigents, flavoring agents, coloring agents, sweetening agents, and the like. The pharmaceutical composition may be in a pharmaceutically conventional formulation form, including capsules, powders, tablets, granules, pills, injections, syrups, oral liquids, inhalants, ointments, suppositories, patches, and the like.

Further, the compound of the present invention may be used in combination with one or more other types of medicaments for preventing or treating the disease mediated by PPARα and/or PPARδ, including but not limited to the following combined administration cases.

Other types of prophylactic or therapeutic medicaments that may be selected for use in combination with the compound of the present invention may be one or more anti-diabetic medicaments.

Other types of prophylactic or therapeutic medicaments that may be selected for use in combination with the compound of the present invention may be one or more anti-obesity medicaments.

Other types of prophylactic or therapeutic medicaments that may be selected for use in combination with the compound of the present invention may be one or more anti-nonalcoholic fatty liver disease medicaments.

Other types of prophylactic or therapeutic medicaments that may be selected for use in combination with the compound of the present invention may be one or more anti-PBC or PSC medicaments.

Other types of prophylactic or therapeutic medicaments that may be selected for use in combination with the compound of the present invention may be one or more lipid-lowering medicaments.

The amount of the compound of formula (I) or the pharmaceutically acceptable salt, the prodrug, the deuterated compound, or the solvate thereof of the present invention may be appropriately changed depending on the age, body weight, and symptoms, administration routes of a patient. For adults, during oral administration, the lower limit of one dose is 0.01 mg (preferably 0.1 mg or 1 mg) and the upper limit of one dose is 1000 mg (preferably 500 mg); during intravenous administration, the lower limit of one dose is 0.001 mg (preferably 0.01 mg or 0.1 mg), and the upper limit of one dose is 500 mg (preferably 250 mg). Deviation from this dosage range may occur depending on the degree of diseases and dosage forms.

Considering that a "α,β-unsaturated ketone" structure in a GFT505 molecule may be the cause of poor stability of liver microsomes, the inventors replaced the "α,β-unsaturated ketone" fragment in the GFT505 molecule structure with a "hydantoin" structure fragment, designed, and synthesized the hydantoin compound of the present invention. By testing the agonistic activity of the hydantoin compound for PPARs, it was surprisingly found that: when the "hydantoin" fragment was adopted to replace the "α,β-unsaturated ketone" structure, a series of compounds with much stronger agonistic activities for PPARα and PPAR6 than those of GFT505 may be obtained, and particularly, the agonistic activities of the preferred compounds (such as compound 1) of the present invention for PPARα/δ may reach a picomolar level. It is noted that compound 1 is the first PPARα/δ dual agonist, which may achieve picomolar levels of both PPARα and PPARδ agonistic activities. In addition, the compound of the present invention has stability far superior to that of GFT505 for human liver microsomes and excellent *in-vivo* pharmacokinetic properties.

Advantages: Compared with the prior art, the present invention has the following advantages:
(1) The present invention provides novel hydantoin compounds, which have potent agonistic effects on both PPARα and PPARδ. For example, compound 1 achieves picomolar levels of EC₅₀ for both PPARα and PPARδ, and the agonistic activities are balanced. Compound 1 has been the most potent and balanced PPARα/δ dual agonist so far. Under the same test system, the activity of compound 1 is remarkably superior to that of PPARα/δ dual agonists reported in the literature, such as the medicament GFT505 in phase III clinical trials and compound H11 with the optimal activity reported in the current literature (Journal of Medicinal Chemistry 2022, 65, 2571-2592).
(2) By analyzing the eutectic structure of PPARδ and compound 1, it is found that in compound 1, besides the key interactions through hydrogen bonds between the carboxylic acid group and three key amino acid residues of PPARδ, i.e., His323, His449, and Tyr473, a plurality of special "water-bridge" interactions through hydrogen bonds exist between the hydantoin ring structure in the compound and the amino acids of PPARδ, i.e., Thr289, Thr292, and Cys285. The brandnew binding mode is probably the reason that the hydantoin derivative PPARα/δ dual agonist of the present invention has potent agonistic activity and high selectivity.
(3) Compared with the medicament GFT505 in phase III clinical trials, the compounds of the present invention have better *in-vitro* and *in-vivo* pharmacokinetic properties. Therefore, the compound or the pharmaceutically acceptable salt thereof of the present invention may be used for preparing a PPARα/δ dual agonist, and further may be used for preparing a medicament for the prevention or treatment of a disease mediated by PPARα and/or PPARδ.
(4) Compared with the activation of PPARγ, the compounds of the present invention exhibit very high selectivity for the activation of PPARα/PPARδ, and the selectivity is significantly superior to that of GFT505. Among them, the selectivity of compound 1 for PPARγ is as high as 2000 times. However, It is well known that activating PPARγ leads to risks of weight gain, edema, fractures, and heart failure (Toxicol. sci., 2006, 90, 269). Therefore, the compounds of the present invention have potential advantages in terms of safety. In addition, the compounds of the present invention have no significant agonistic effect on other nuclear receptors, indicating that the compounds have high selectivity for PPARα/δ.
(5) In a NASH mouse model, a cholestasis mouse model, and a hepatic fibrosis mouse model, the compounds of the present invention (such as compound 1) have better anti-NASH, anticholestasis, and anti-fibrosis effects than those of the clinically researched PPARα/δ dual agonist GFT505 at a very low dose, and have very good safety, indicating that the compounds of the present invention have very good clinical application prospects.
(6) The hydantoin compounds of the present invention have ingenious designs, simple structures, cheap and easily available raw materials, and safe and environment-friendly synthesis processes, and are easy for large-scale production.

### BRIEF DESCRIPTION OF THE DRAWINGS

FIG. 1 is a graph showing the effect of compound 1 on mouse serum triglyceride (n = 6, **p <* 0.05 and ****p* < 0.001 in t-test compared to the control group; *^{#}p <* 0.05, *^{##}p* < 0.01, and *^{###}p <* 0.001 in t-test compared to the compound 1 1 mpk group);
FIG. 2 shows the effect of compound 1 on liver morphology and serum color in ANIT-induced cholestasis in mice;
FIG. 3 is a graph showing the effect of compound 1 on aspartate aminotransferase, alanine aminotransferase, alkaline phosphatase, total bilirubin, and total bile acid in serum of cholestatic mice (n = 5, *^{#}p* < 0.05, *^{##}p* < 0.01, and *^{###}p* < 0.001 in t-test compared to the control group; **p* < 0.05, ***p* < 0.01, and ****p* < 0.001 in one-way analysis of variance compared to the model group; ^{$}*p* < 0.05, ^{$$}*p* < 0.01, and ^{$$$}*p* < 0.001 in t-test compared to the model group);
FIG. 4 is a graph showing the effect of compound 1 on alkaline phosphatase in the liver of cholestatic mice (n = 5, *^{###}p* < 0.001 in t-test compared to the control group; **p* < 0.05 in one-way analysis of variance compared to the model group);
FIG. 5 shows HE staining pictures of liver sections of cholestatic mice with compound 1;
FIG. 6 is a graph showing the effect of compound 1 on aspartate aminotransferase and alanine aminotransferase in serum of NASH model mice (n = 6, *^{###}p* < 0.001 in t-test compared to the control group; ****p* < 0.001 in t-test compared to the model group);
FIG. 7 shows HE staining pictures of liver sections of NASH model mice with compound 1;
FIG. 8 shows sirius red staining pictures of liver sections of NASH model mice with compound 1;
FIG. 9 shows oil red staining pictures of liver sections of NASH model mice with compound 1;
FIG. 10 is a graph showing the effect of compound 1 on intrahepatic triglyceride contents in NASH model mice (n = 6, *^{###}p <* 0.001 in t-test compared to the control group; **p* < 0.05 in t-test compared to the model group);
FIG. 11 is a graph showing the effect of compound 1 on genes associated with liver inflammation in NASH model mice (n = 6, *^{###}p* < 0.001 in t-test compared to the control group; **p* < 0.05, ***p* < 0.01, and ****p* < 0.001 in t-test compared to the model group);
FIG. 12 is a graph showing the effect of compound 1 on genes associated with hepatic fibrosis in NASH model mice (n = 6, *^{#}p <* 0.05, *^{##}p <* 0.01, and *^{###}p <* 0.001 in t-test compared to the control group; **p* < 0.05, ***p* < 0.01, and ****p* < 0.001 in t-test compared to the model group);
FIG. 13 is a graph showing the effect of compound 1 on hepatic hydroxyproline contents in hepatic fibrosis model mice (n = 6, *^{###}p <* 0.001 in t-test compared to the control group; ****p* < 0.001 in one-way analysis of variance compared to the model group; ^{$$}*p* < 0.01 in t-test compared to the model group);
FIG. 14 shows HE staining pictures of liver sections of hepatic fibrosis model mice with compound 1;
FIG. 15 shows sirius red staining pictures of liver sections of hepatic fibrosis model mice with compound 1; and
FIG. 16 is a diagram showing an eutectic structure of compound 1 with the PPAR6 protein.

### DETAILED DESCRIPTION

The content of the present invention will be specifically described below through examples. In the present invention, the following examples are intended to better illustrate the present invention and not to limit the scope of the present invention. Various changes and modifications may be made to the present invention without departing from the spirit and scope of the present invention.

### Example 1

### 2-(4-((2, 5-Dioxo-3-(4-(trifluoromethyl)phenyl)imidazolidin-1-yl)methyl)-2,6-dimethylphenoxy)-2-methylpropionic acid (compound 1)

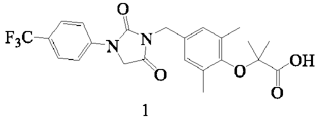

### Method (1)

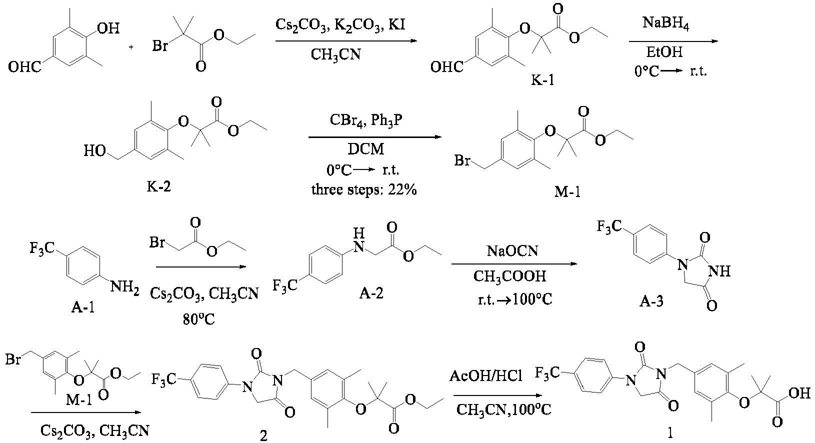

### Synthesis of intermediate K-1

4-Hydroxy-3,5-dimethylbenzaldehyde (21 g, 140 mmol) was dissolved in acetonitrile (200 mL), and ethyl 2-bromoisobutyrate (100.5 g, 520 mmol), cesium carbonate (45.6 g, 140 mmol), potassium carbonate (38.6 g, 280 mmol), and potassium iodide (2.3 g, 14 mmol) were added. The reaction system was heated to 80 °C and reacted for 36 h. After the reaction was completed, the mixture was filtered under vacuum. The solvent was distilled off under reduced pressure, water (200 mL) was added for dilution, and the resulting solution was extracted with ethyl acetate (EA) (200 mL × 3). The organic phase was washed with 1 N sodium hydroxide solution (200 mL × 3) and saturated brine (200 mL × 1) and dried over anhydrous sodium sulfate. The solvent was distilled off under reduced pressure, and the residue was purified by silica gel column chromatography (petroleum ether/ethyl acetate = 200:1) to give intermediate K-1 (yellow liquid, 16.3 g, 44.1% yield).

### Synthesis of intermediate K-2

Intermediate K-1 (3.66 g, 13.85 mmol) was dissolved in ethanol (20 mL), and sodium borohydride (280 mg, 7.5 mmol) was added slowly in an ice bath. The mixture was stirred at room temperature for 4 h. After the reaction was completed, water (20 mL) was added for quenching. The solvent was distilled off under reduced pressure, water (30 mL) was added for dilution, and the resulting solution was extracted with EA (20 mL × 3). The organic phase was washed with saturated sodium chloride solution (30 mL × 1) and dried over anhydrous sodium sulfate. The solvent was distilled off under reduced pressure to give crude intermediate K-2, which was directly used in the next step without further purification.

### Synthesis of intermediate M-1

All of crude intermediate K-2 obtained from the previous reaction was dissolved in dichloromethane (DCM) (20 mL), tetrabromomethane (13.6 g, 41 mmol) was added, and triphenylphosphine (9.9 g, 37.8 mmol) was added slowly in an ice bath. The mixture was stirred at room temperature for 8 h. The solvent was distilled off under reduced pressure, and the residue was purified by silica gel column chromatography (petroleum ether/ethyl acetate = 20: 1) to give intermediate M-1 (yellow liquid, 3.54 g, 78.0% yield).

### Synthesis of intermediate A-2

*p*-Trifluoromethylaniline A-1 (1.6 g, 10 mmol) was dissolved in acetonitrile (10 mL), and ethyl 2-bromoacetate (1.2 mL, 11 mmol) and cesium carbonate (3.3 g, 10 mmol) were added. The reaction system was heated to 80 °C and reacted for 12 h. After the reaction was completed, the solvent was distilled off under reduced pressure, water (50 mL) was added, and the resulting solution was extracted with EA (50 mL × 3). The organic phase was washed with saturated sodium chloride solution (50 mL × 1). The solvent was distilled off under reduced pressure, and the residue was purified by silica gel column chromatography (petroleum ether/ethyl acetate = 40: 1) to give intermediate A-2 (yellow solid, 1.24 g, 50.2% yield).

### Synthesis of intermediate A-3

Intermediate A-2 (2.4 g, 10 mmol) was dissolved in acetic acid (10 mL) under argon atmosphere, and a suspension of sodium cyanate (3.9 g, 60 mmol) in acetic acid (20 mL) was added. The mixture was stirred at room temperature for 12 h. The reaction system was heated to 100 °C and stirred for 12 h. After the reaction was completed, the solvent was distilled off under reduced pressure, water (50 mL) was added, and the resulting solution was extracted with EA (50 mL × 3). The organic phase was washed with saturated sodium chloride solution (50 mL × 1). The solvent was distilled off under reduced pressure, and the residue was purified by silica gel column chromatography (petroleum ether/ethyl acetate = 2:1) to give compound A-3 (white solid, 1.1 g, 45.1% yield).

### Synthesis of compound 2

Intermediate A-3 (244 mg, 1 mmol) was dissolved in acetonitrile (3 mL), and M-1 (492 mg, 1.5 mmol) and cesium carbonate (815 mg, 2.5 mmol) were added. The mixture was stirred at room temperature for 12 h. After the reaction was completed, the solvent was distilled off under reduced pressure, and the residue was purified by silica gel column chromatography (petroleum ether/ethyl acetate = 5:1) to give compound 2 (colorless liquid, 469.8 mg, 95.4% yield).

### Synthesis of compound 1

Compound 2 (441 mg, 0.89 mmol) was dissolved in acetonitrile (3 mL), and a mixed solution of concentrated hydrochloric acid (at a concentration of 12 M) and acetic acid (10 mL, 1:1) was added. The reaction system was heated to 100 °C and stirred for 4 h. After the reaction was completed, the solvent was distilled off under reduced pressure, water (20 mL) was added, and the resulting solution was extracted with EA (25 mL × 3). The organic phase was washed with saturated sodium chloride solution (20 mL × 1). The solvent was distilled off under reduced pressure, and the residue was purified by silica gel column chromatography (dichloromethane/methanol = 100:1) to give compound 1 (white solid, 98.7 mg, 23.9% yield): ¹H NMR (300 MHz, DMSO-*d₆*) δ 12.80 (s, 1H), 7.87 (d, *J* = 8.8 Hz, 2H), 7.77 (d, *J* = 8.8 Hz, 2H), 6.98 (s, 2H), 4.63 (s, 2H), 4.54 (s, 2H), 2.15 (s, 6H), 1.34 (s, 6H). HRMS (ESI) calcd. for C₂₃H₂₃F₃N₂O₅ [M+NH₄]⁺: 482.1903, found: 482.1898.

### Method (2)

### Intermediate A-3 could also be synthesized according to the following route:

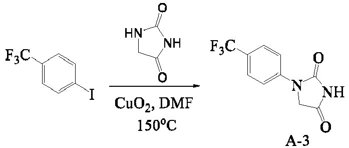

*p*-Iodotrifluoromethylbenzene (2.7 g, 10 mmol), cuprous oxide (1.4 g, 10 mmol), and hydantoin (1.5 g, 15 mmol) were added to a three-necked flask, and the reaction system was purged with argon. Anhydrous DMF (10 mL) was then added, and the reaction system was heated to 150 °C and reacted for 12 h. After the reaction was completed, the reaction solution was filtered through celite, water (50 mL) was added, and the resulting solution was extracted with EA (50 mL × 3) and washed with saturated sodium chloride (50 mL × 1). The solvent was distilled off under reduced pressure, and the residue was purified by silica gel column chromatography (petroleum ether/ethyl acetate = 4:1) to give compound A-3 (white solid, 949 mg, 38.9% yield).

### Method (3)

Compound 2 could also be synthesized according to the following route:

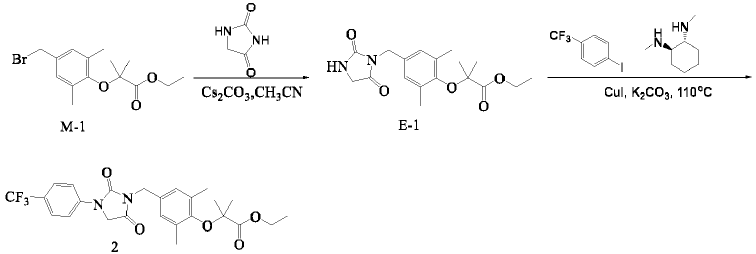

Intermediate M-1 (3.28 g, 10 mmol) was dissolved in acetonitrile (10 mL), and hydantoin (1.5 g, 15 mmol) and cesium carbonate (4.89 g, 15 mmol) were added. The mixture was stirred at room temperature for 12 h. After the reaction was completed, the solvent was distilled off under reduced pressure, and the residue was purified by silica gel column chromatography (petroleum ether/ethyl acetate = 5:1) to give intermediate E-1 (white solid, 1.8 g, 51.7% yield). Intermediate E-1 (1.17 g, 5 mmol), cuprous iodide (189 mg, 1 mmol), potassium carbonate (1.37 g, 10 mmol), and (1R,2R)-(-)-N,N'-dimethyl-1,2-cyclohexanediamine (284 mg, 2 mmol) were added to a three-necked flask, and the reaction system was purged with argon. A solution of *p-*iodotrifluoromethylbenzene (1.6 g, 6 mmol) in toluene (15 mL) was added, and the reaction system was heated to 110 °C. After the reaction was completed, the solvent was distilled off under reduced pressure, and the residue was purified by silica gel column chromatography (petroleum ether/ethyl acetate = 10:1) to give compound 2 (colorless liquid, 1.23 g, 41.7% yield).

### Example 2

### Ethyl 2-(4-((2,5-dioxo-3-(4-(trifluoromethyl)phenyl)imidazolidin-1-yl)methyl)-2,6-dimethylphenoxy)-2-methylpropionate (compound 2)

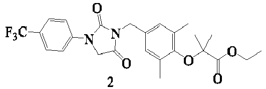

Referring to the method in Example 1, compound 2 (colorless liquid, 469.8 mg, 95.4% yield) was prepared without hydrolysis: ¹H NMR (300 MHz, CDCl₃) δ 7.73 (d, *J* = 8.8 Hz, 2H), 7.65 (d, *J* = 8.9 Hz, 2H), 7.07 (s, 2H), 4.65 (s, 2H), 4.36 (s, 2H), 4.29 (q, *J* = 7.1 Hz, 2H), 2.20 (s, 6H), 1.46 (s, 6H), 1.36 (t, *J* = 7.1 Hz, 3H). MS (ESI): *m*/*z* 515.2[M+Na]⁺.

### Example 3

### 2-(4-((2, 5-Dioxo-3-(4-(trifluoromethoxy)phenyl)imidazolidin-1-yl)methyl)-2,6-dimethylphenoxy)-2-methylpropionic acid (compound 3)

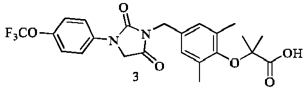

Referring to the method in Example 1, *p*-trifluoromethylaniline was replaced by p-trifluoromethoxyaniline to give compound 3 (white solid, 62.0 mg, 66.9% yield): ¹H NMR (300 MHz, DMSO-*d₆*) δ 12.79 (s, 1H), 7.76 (d, *J* = 9.1 Hz, 2H), 7.43 (d, *J* = 8.6 Hz, 2H), 6.97 (s, 2H), 4.60 (s, 2H), 4.53 (s, 2H), 2.15 (s, 6H), 1.35 (s, 6H). HRMS (ESI) calcd. for C₂₃H₂₃F₃N₂O₆ [M+NH₄]⁺: 498.1852, found: 498.1847.

### Example 4

### Ethyl 2-(4-((2,5-dioxo-3-(4-(trifluoromethoxy)phenyl)imidazolidin-1-yl)methyl)-2,6-dimethylphenoxy)-2-methylpropionate (compound 4)

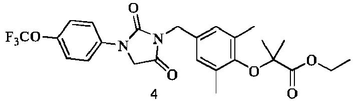

Referring to the method in Example 1, *p*-trifluoromethylaniline was replaced by p-trifluoromethoxyaniline to give compound 4 (colorless liquid, 98 mg, 44.4% yield) without hydrolysis: ¹H NMR (300 MHz, CDCl₃) δ 7.73 (d, *J* = 8.8 Hz, 2H), 7.65 (d, *J* = 8.9 Hz, 2H), 7.07 (s, 2H), 4.65 (s, 2H), 4.36 (s, 2H), 4.29 (q, *J* = 7.1 Hz, 2H), 2.20 (s, 6H), 1.46 (s, 6H), 1.36 (t, *J* = 7.1 Hz, 3H). MS (ESI): *m*/*z* 531.2[M+Na]⁺.

### Example 5

### 2-(4-((4,4-Dimethyl-2,5-dioxo-3-(4-(trifluoromethyl)phenyl)imidazolin-1-yl)methyl)-2,6-dimethylphenoxy)-2-methylpropionic acid (compound 5)

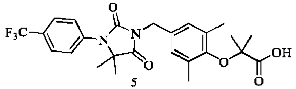

Referring to the method in Example 1, hydantoin in the synthetic route of method (3) was replaced by 5,5-dimethylhydantoin to give compound 5 (white solid, 21 mg, 55.5% yield): ¹H NMR (300 MHz, DMSO-*d₆*) δ 12.79 (s, 1H), 7.86 (d, *J* = 8.4 Hz, 2H), 7.68 (d, *J* = 8.3 Hz, 2H), 6.92 (s, 2H), 4.57 (s, 2H), 2.17 (s, 6H), 1.44 (s, 6H), 1.35 (s, 6H). HRMS (ESI) calcd. for C₂₅H₂₇F₃N₂O₅ [M+NH₄]⁺: 510.2216, found: 510.2211.

### Example 6

### Ethyl 2-(4-((4,4-dimethyl-2,5-dioxo-3-(4-(trifluoromethyl)phenyl)imidazolin-1-yl)methyl)-2,6-dimethylphenoxy)-2-methylpropionate (compound 6)

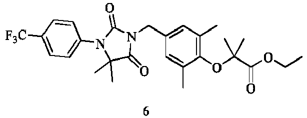

Referring to the method in Example 1, hydantoin in the synthetic route of method (3) was replaced by 5,5-dimethylhydantoin to give compound 6 (yellow liquid, 40 mg, 19.1% yield) without hydrolysis: ¹H NMR (300 MHz, CDCl₃) δ 7.71 (d, *J* = 8.4 Hz, 2H), 7.46 (d, *J* = 8.4 Hz, 2H), 7.04 (s, 2H), 4.65 (s, 2H), 4.30 (q, *J* = 7.1 Hz, 2H), 2.20 (s, 6H), 1.50 (s, 6H), 1.48 (s, 6H), 1.36 (t, *J* = 7.1 Hz, 3H). MS (ESI): *m*/*z* 543.2[M+Na]⁺.

### Example 7

### 2-(4-((4,4-Dimethyl-2,5-dioxo-3-(4-(trifluoromethyl)phenyl)imidazolin-1-yl)methyl)-2,6-dimethylphenoxy)-2-methylpropionic acid (compound 7)

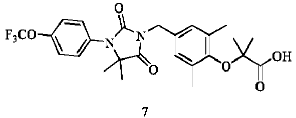

Referring to the method in Example 1, 4-iodotrifluoromethylbenzene in the synthetic route of method (3) was replaced by 4-(trifluoromethoxy)iodobenzene, and hydantoin was replaced by 5,5-dimethylhydantoin to give compound 7 (white solid, 40 mg, 54.5% yield): ¹H NMR (300 MHz, DMSO-*d₆*) δ 12.61 (s, 1H), 7.55 (d, *J* = 9.0 Hz, 2H), 7.49 (d, *J* = 8.9 Hz, 2H), 6.91 (s, 2H), 4.55 (s, 2H), 2.16 (s, 6H), 1.39 (s, 6H), 1.35 (s, 6H). HRMS (ESI) calcd. for C₂₅H₂₇F₃N₂O₆ [M+NH₄]⁺: 526.2165, found: 526.2159.

### Example 8

### Ethyl 2-(4-((4,4-dimethyl-2,5-dioxo-3-(4-(trifluoromethoxy)phenyl)imidazolin-1-yl)methyl)-2,6-dimethylphenoxy)-2-methylpropionate (compound 8)

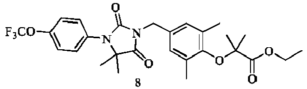

Referring to the method in Example 1, 4-iodotrifluoromethylbenzene in the synthetic route of method (3) was replaced by 4-(trifluoromethoxy)iodobenzene, and hydantoin was replaced by 5,5-dimethylhydantoin to give compound 8 (colorless liquid, 80 mg, 29.7% yield) without hydrolysis: ¹H NMR (300 MHz, CDCl₃) δ 7.31 (s, 4H), 7.04 (s, 2H), 4.63 (s, 2H), 4.30 (q, 7.1 Hz, 2H), 2.20 (s, 6H), 1.48 (s, 6H), 1.46 (s, 6H), 1.36 (t, *J* = 7.1 Hz, 3H). MS (ESI): *m*/*z* 559.2[M+Na]⁺.

### Example 9

### 2-(4-((2, 5-Dioxo-3-(4-(trifluoromethyl)phenyl)imidazolidin-1-yl)methyl)-2-methylphenoxy)acetic acid (compound 9)

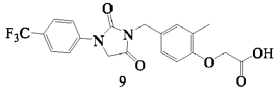

Referring to the method in Example 1, 4-hydroxy-3,5-dimethylbenzaldehyde was replaced by 4-hydroxy-3-methylbenzaldehyde, and ethyl 2-bromoisobutyrate was replaced by ethyl 2-bromoacetate to give compound 9 (white solid, 85.2 mg, 66.0% yield): ¹H NMR (300 MHz, DMSO-*d₆*) δ 12.96 (s, 1H), 7.85 (d, *J* = 8.7 Hz, 2H), 7.76 (d, *J* = 8.7 Hz, 2H), 7.12 (d, *J* = 11.3 Hz, 2H), 6.78 (d, *J* = 8.3 Hz, 1H), 4.67 (s, 2H), 4.60 (s, 2H), 4.55 (s, 2H), 2.17 (s, 3H). HRMS (ESI) calcd. for C₂₀H₁₇F₃N₂O₅ [M+Na]⁺: 445.0987, found: 445.0992.

### Example 10

### Ethyl 2-(4-(2,5-dioxo-3-(4-(trifluoromethyl)phenyl)imidazolidin-1-yl)methyl)-2-methylphenoxy)acetate (compound 10)

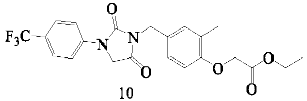

Referring to the method in Example 1, 4-hydroxy-3,5-dimethylbenzaldehyde was replaced by 4-hydroxy-3-methylbenzaldehyde, and ethyl 2-bromoisobutyrate was replaced by ethyl 2-bromoacetate to give compound 10 (white solid, 137.7 mg, 57.0% yield) without hydrolysis: ¹H NMR (300 MHz, CDCl₃) δ 7.71 (d, *J* = 8.8 Hz, 2H), 7.65 (d, *J* = 8.9 Hz, 2H), 7.27 (d, *J* = 6.9 Hz, 2H), 6.66 (d, *J* = 8.1 Hz, 1H), 4.69 (s, 2H), 4.63 (s, 2H), 4.33 (s, 2H), 4.27 (q, *J* = 7.1 Hz, 2H), 2.29 (s, 3H), 1.31 (t, *J* = 7.2 Hz, 3H). MS (ESI): *m*/*z* 573.2[M+Na]⁺.

### Example 11

### 2-(4-((2,5-Dioxo-3-(4-(trifluoromethyl)phenyl)imidazolidin-1-yl)methyl)-2-(trifluoromethyl)phenoxy)-2-methylpropionic acid (compound 11)

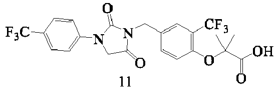

Referring to the method in Example 1, 4-hydroxy-3,5-dimethylbenzaldehyde was replaced by 4-hydroxy-3-trifluoromethylbenzaldehyde to give compound 11 (white solid, 89 mg, 55.6% yield): ¹H NMR (300 MHz, DMSO-*d₆*) δ 13.37 (s, 1H), 7.85 (d, *J* = 8.7 Hz, 2H), 7.76 (d, *J* = 8.7 Hz, 2H), 7.63 (s, 1H), 7.55 (d, *J* = 9.2 Hz, 1H), 6.89 (d, *J* = 8.6 Hz, 1H), 4.65 (s, 2H), 4.59 (s, 2H), 1.53 (s, 6H). HRMS (ESI) calcd. for C₂₂H₁₈F₆N₂O₅ [M+Na]⁺: 527.1018, found: 527.1012.

### Example 12

### Ethyl 2-(4-((2,5-dioxo-3-(4-(trifluoromethyl)phenyl)imidazolidin-1-yl)methyl)-2-(trifluoromethyl)phenoxy)-2-methylpropionate (compound 12)

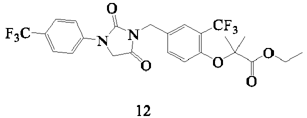

Referring to the method in Example 1, 4-hydroxy-3,5-dimethylbenzaldehyde was replaced by 4-hydroxy-3-trifluoromethylbenzaldehyde to give compound 12 (yellow liquid, 169 mg, 63.4% yield) without hydrolysis: ¹H NMR (300 MHz, CDCl₃) δ 7.77 - 7.62 (m, 5H), 7.53 (d, *J* = 8.3 Hz, 1H), 6.78 (d, *J* = 8.9 Hz, 1H), 4.73 (s, 2H), 4.36 (s, 2H), 4.26 (q, *J* = 6.3 Hz, 2H), 1.63 (s, 6H), 1.28 (t, *J* = 6.3, 3H). MS (ESI): *m*/*z* 555. 1[M+Na]⁺.

### Example 13

### 2-(2-Chloro-4-((2,5-dioxo-3-(4-(trifluoromethyl)phenyl)imidazolidin-1-yl)methyl)-6-methylphenoxy)-2-methylpropionic acid (compound 13)

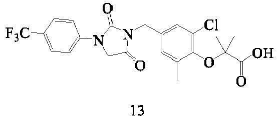

Referring to the method in Example 1, 4-hydroxy-3,5-dimethylbenzaldehyde was replaced by 4-hydroxy-3-methyl-5-chlorobenzaldehyde to give compound 13 (white solid, 50 mg, 30.7% yield): ¹H NMR (300 MHz, DMSO-*d₆*) δ 12.84 (s, 1H), 7.86 (d, *J* = 8.7 Hz, 2H), 7.77 (d, *J* = 8.8 Hz, 2H), 7.27 (s, 1H), 7.15 (s, 1H), 4.62 (s, 2H), 4.59 (s, 2H), 2.20 (s, 3H), 1.41 (s, 6H). HRMS (ESI) calcd. for C₂₂H₂₀ClF₃N₂O₅ [M+Na]⁺: 507.0911, found: 507.0905.

### Example 14

### Ethyl 2-(2-chloro-4-((2,5-dioxo-3-(4-(trifluoromethyl)phenyl)imidazolidin-1-yl)methyl)-6-methylphenoxy)-2-methylpropionate (compound 14)

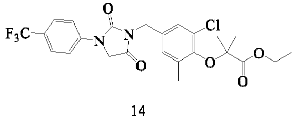

Referring to the method in Example 1, 4-hydroxy-3,5-dimethylbenzaldehyde was replaced by 4-hydroxy-3-methyl-5-chlorobenzaldehyde to give compound 14 (yellow liquid, 174 mg, 67.8% yield) without hydrolysis: ¹H NMR (300 MHz, CDCl₃) δ 7.73 (d, *J* = 8.9 Hz, 2H), 7.66 (d, *J* = 8.8 Hz, 2H), 7.31 (s, 1H), 7.16 (s, 1H), 4.66 (s, 2H), 4.38 (s, 2H), 4.29 (q, *J* = 7.1 Hz, 2H), 2.24 (s, 3H), 1.53 (s, 6H), 1.36 (t, *J* = 7.1 Hz, 3H). MS (ESI): *m*/*z* 535.1[M+Na]⁺.

### Example 15

### 2-(4-((2,5-Dioxo-3-(2-(trifluoromethyl)phenyl)imidazolidin-1-yl)methyl)-2,6-dimethylphenoxy)-2-methylpropionic acid (compound 15)

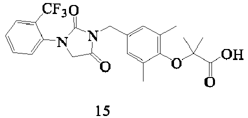

Referring to the method in Example 1, 4-iodotrifluoromethylbenzene in the synthetic route of method (2) was replaced by 2-iodotrifluoromethylbenzene to give compound 15 (white solid, 37.2 mg, 80.1% yield): ¹H NMR (300 MHz, DMSO-*d₆*) δ 12.79 (s, 1H), 7.90 - 7.76 (m, 3H), 7.71 - 7.64 (m, 1H), 6.92 (s, 2H), 4.54 (s, 2H), 4.42 (s, 2H), 2.16 (s, 6H), 1.35 (s, 6H). HRMS (ESI) calcd. for C₂₃H₂₃F₃N₂O₅ [M+Na]⁺: 487.1457, found: 487.1453.

### Example 16

### Ethyl 2-(4-((2,5-dioxo-3-(2-(trifluoromethyl)phenyl)imidazolidin-1-yl)methyl)-2,6-dimethylphenoxy)-2-methylpropionate (compound 16)

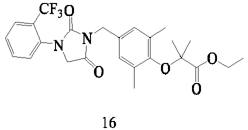

Referring to the method in Example 1, 4-iodotrifluoromethylbenzene in the synthetic route of method (2) was replaced by 2-iodotrifluoromethylbenzene to give compound 16 (colorless liquid, 78.7 mg, 39.6% yield) without hydrolysis: ¹H NMR (300 MHz, CDCl₃) δ 7.79 (d, *J* = 7.5 Hz, 1H), 7.68 (t, *J* = 7.2 Hz, 1H), 7.56 (t, *J* = 7.7 Hz, 1H), 7.42 (d, *J* = 7.7 Hz, 1H), 7.05 (s, 2H), 4.65 (s, 2H), 4.30 (q, *J* =7.1 Hz, 2H), 4.26 (s, 2H), 2.20 (s, 6H), 1.48 (s, 6H), 1.36 (t, *J* = 7.1 Hz, 3H). MS (ESI): *m*/*z* 515.2[M+Na]⁺.

### Example 17

### 2-(2,6-Dichloro-4-((2,5-dioxo-3-(4-(trifluoromethyl)phenyl)imidazolidin-1-yl)methyl)phenoxy)-2-methylpropionic acid (compound 17)

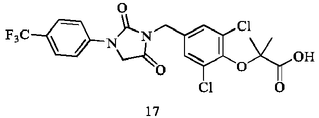

Referring to the method in Example 1, 4-hydroxy-3,5-dimethylbenzaldehyde was replaced by 4-hydroxy-3,5-dichlorobenzaldehyde to give compound 17 (white solid, 100.1 mg, 62.2% yield): ¹H NMR (300 MHz, DMSO-*d₆*) δ 12.85 (s, 1H), 7.87 (d, *J* = 8.7 Hz, 2H), 7.78 (d, *J* = 8.8 Hz, 2H), 7.48 (s, 2H), 4.65 (s, 2H), 4.61 (s, 2H), 1.46 (s, 6H). HRMS (ESI) calcd. for C₂₁H₁₇Cl₂F₃N₂O₅ [M+Na]⁺: 527.0364, found: 527.0358.

### Example 18

### Ethyl 2-(2,6-dichloro-4-((2,5-dioxo-3-(4-(trifluoromethyl)phenyl)imidazolidin-1-yl)methyl)phenoxy)-2-methylpropionate (compound 18)

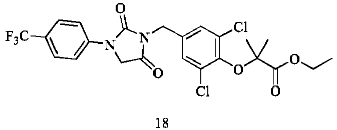

Referring to the method in Example 1, 4-hydroxy-3,5-dimethylbenzaldehyde was replaced by 4-hydroxy-3,5-dichlorobenzaldehyde to give compound 18 (yellow liquid, 171 mg, 64.0% yield) without hydrolysis: ¹H NMR (300 MHz, CDCl₃) δ 7.73 (d, *J* = 9.0 Hz, 2H), 7.67 (d, *J* = 9.0 Hz, 2H), 7.41 (s, 2H), 4.67 (s, 2H), 4.40 (s, 2H), 4.21 - 4.07 (m, 2H), 1.58 (s, 6H), 1.28 (t, *J* = 7.2 Hz, 3H). MS (ESI): *m*/*z* 555.1[M+Na]⁺.

### Example 19

### 2-(2,6-Dibromo-4-((2,5-dioxo-3-(4-(trifluoromethyl)phenyl)imidazolidin-1-yl)methyl)phenoxy)-2-methylpropionic acid (compound 19)

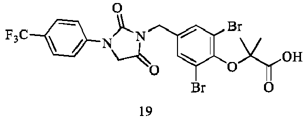

Referring to the method in Example 1, 4-hydroxy-3,5-dimethylbenzaldehyde was replaced by 4-hydroxy-3,5-dibromobenzaldehyde to give compound 19 (white solid, 120.2 mg, 70.3% yield): ¹H NMR (300 MHz, DMSO-*d₆*) δ 12.86 (s, 1H), 7.87 (d, *J* = 8.7 Hz, 2H), 7.78 (d, *J* = 8.8 Hz, 2H), 7.66 (s, 2H), 4.65 (s, 2H), 4.61 (s, 2H), 1.50 (s, 6H). HRMS (ESI) calcd. for C₂₁H₁₇Br₂F₃N₂O₅ [M+Na]⁺: 614.9354, found: 614.9348.

### Example 20

### Ethyl 2-(2,6-dibromo-4-((2,5-dioxo-3-(4-(trifluoromethyl)phenyl)imidazolidin-1-yl)methyl)phenoxy)-2-methylpropionate (compound 20)

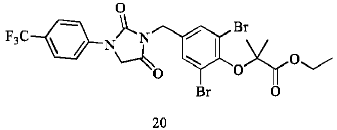

Referring to the method in Example 1, 4-hydroxy-3,5-dimethylbenzaldehyde was replaced by 4-hydroxy-3,5-dibromobenzaldehyde to give compound 20 (colorless liquid, 180 mg, 57.9% yield) without hydrolysis: ¹H NMR (300 MHz, CDCl₃) δ 7.73 (d, *J* = 8.9 Hz, 2H), 7.67 (d, *J* = 8.9 Hz, 2H), 7.62 (s, 2H), 4.67 (s, 2H), 4.40 (s, 2H), 4.30 (q, *J* = 7.1 Hz, 2H), 1.59 (s, 6H), 1.37 (t, *J* = 7.1 Hz, 3H). MS (ESI): *m*/*z* 642.2[M+Na]⁺.

### Example 21

### 2-(4-((2,5-Dioxo-3-(4-(trifluoromethyl)phenyl)imidazolin-1-yl)methyl)-2,6-difluorophenoxy)-2-methylpropionic acid (compound 21)

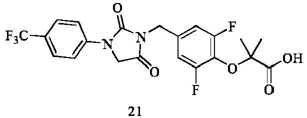

Referring to the method in Example 1, 4-hydroxy-3,5-dimethylbenzaldehyde was replaced by 4-hydroxy-3,5-difluorobenzaldehyde to give compound 21 (white solid, 22.4 mg, 56.7% yield): ¹H NMR (300 MHz, DMSO-*d₆*) δ 12.94 (s, 1H), 7.87 (d, *J* = 8.7 Hz, 2H), 7.77 (d, *J* = 8.8 Hz, 2H), 7.15 (d, *J* = 8.9 Hz, 2H), 4.65 (s, 2H), 4.62 (s, 2H), 1.44 (s, 6H). HRMS (ESI) calcd. for C₂₁H₁₇F₅N₂O₅ [M+Na]⁺: 495.0955, found: 495.0946.

### Example 22

### Ethyl 2-(4-((2,5-dioxo-3-(4-(trifluoromethyl)phenyl)imidazolin-1-yl)methyl)-2,6-difluorophenoxy)-2-methylpropionate (compound 22)

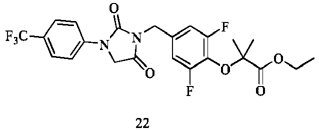

Referring to the method in Example 1, 4-hydroxy-3,5-dimethylbenzaldehyde was replaced by 4-hydroxy-3,5-difluorobenzaldehyde to give compound 22 (colorless liquid, 41.9 mg, 16.7% yield) without hydrolysis: ¹H NMR (300 MHz, CDCl₃) δ 7.73 (d, *J* = 8.6 Hz, 2H), 7.67 (d, *J* = 8.7 Hz, 2H), 7.03 (d, *J* = 8.0 Hz, 2H), 4.69 (s, 2H), 4.39 (s, 2H), 4.26 (q, *J* = 7.1 Hz, 2H), 1.56 (s, 6H), 1.30 (t, *J* = 7.1 Hz, 3H). MS (ESI): *m*/*z* 523.1[M+Na]⁺.

### Example 23

### 2-(4-((2,5-Dioxo-3-(4-methylphenyl)imidazolin-1-yl)methyl)-2,6-dimethylphenoxy)-2-methylpropionic acid (compound 23)

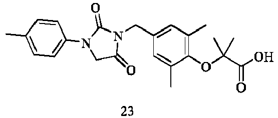

Referring to the method in Example 1, 4-iodotrifluoromethylbenzene in the synthetic route of method (2) was replaced by 4-iodotoluene to give compound 23 (white solid, 61.7 mg, 83.7% yield): ¹H NMR (300 MHz, DMSO-*d₆*) δ 12.82 (s, 1H), 7.52 (d, *J* = 8.5 Hz, 2H), 7.20 (d, *J* = 8.4 Hz, 2H), 6.97 (s, 2H), 4.55 (s, 2H), 4.52 (s, 2H), 2.28 (s, 3H), 2.15 (s, 6H), 1.35 (s, 6H). HRMS (ESI) calcd. for C₂₃H₂₆N₂O₅ [M+Na]⁺: 433.1739, found: 433.1727.

### Example 24

### Ethyl 2-(4-((2,5-dioxo-3-(4-methyltolyl)imidazolin-1-yl)methyl)-2,6-dimethylphenoxy)-2-methylpropionate (compound 24)

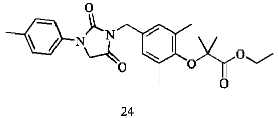

Referring to the method in Example 1, 4-iodotrifluoromethylbenzene in the synthetic route of method (2) was replaced by 4-iodotoluene to give compound 24 (colorless liquid, 78.7 mg, 71.9% yield) without hydrolysis: ¹H NMR (300 MHz, CDCl₃) δ 7.45 (d, *J* = 8.5 Hz, 2H), 7.19 (d, *J* = 8.3 Hz, 2H), 7.07 (s, 2H), 4.63 (s, 2H), 4.30 (s, 2H), 4.29 (q, *J* = 7.1 Hz, 2H), 2.34 (s, 3H), 2.19 (s, 6H), 1.46 (s, 6H), 1.36 (t, *J* = 7.1 Hz, 3H). MS (ESI): *m*/*z* 461.2[M+Na]⁺.

### Example 25

### 2-(4-((3-(4-Fluorophenyl)-2,5-dioxoimidazolin-1-yl)methyl)-2,6-dimethylphenoxy)-2-methylpropionic acid (compound 25)

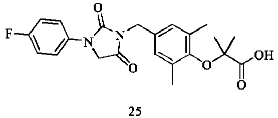

Referring to the method in Example 1, 4-iodotrifluoromethylbenzene in the synthetic route of method (2) was replaced by p-fluoroiodobenzene to give compound 25 (white solid, 87.2 mg, 90.6% yield): ¹H NMR (300 MHz, DMSO-*d₆*) δ 12.81 (s, 1H), 7.66 (dd, J = 8.9, 4.7 Hz, 2H), 7.38 - 7.17 (m, 2H), 6.96 (s, 2H), 4.57 (s, 2H), 4.52 (s, 2H), 2.15 (s, 6H), 1.34 (s, 6H). HRMS (ESI) calcd. for C₂₂H₂₃FN₂O₅ [M+Na]⁺: 437.1489, found: 437.1482.

### Example 26

### Ethyl 2-(4-((3-(4-fluorophenyl)-2,5-dioxoimidazolin-1-yl)methyl)-2,6-dimethylphenoxy)-2-methylpropionate (compound 26)

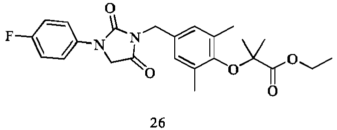

Referring to the method in Example 1, 4-iodotrifluoromethylbenzene in the synthetic route of method (2) was replaced by *p*-fluoroiodobenzene to give compound 26 (colorless liquid, 102.8 mg, 77.4% yield) without hydrolysis: ¹H NMR (300 MHz, CDCl₃) δ 7.59 - 7.50 (m, 2H), 7.11 (d, *J* = 8.2 Hz, 2H), 7.07 (s, 2H), 4.63 (s, 2H), 4.31 (s, 2H), 4.30 (q, *J* = 7.1 Hz, 2H), 2.19 (s, 6H), 1.47 (s, 6H), 1.36 (t, *J* = 7.1 Hz, 3H). MS (ESI): *m*/*z* 465.2[M+Na]⁺.

### Example 27

### 2-(4-((3-(4-Chlorophenyl)-2,5-dioxoimidazolin-1-yl)methyl)-2,6-dimethylphenoxy)-2-methylpropionic acid (compound 27)

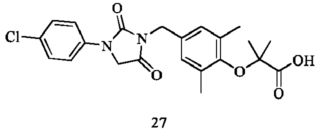

Referring to the method in Example 1, 4-iodotrifluoromethylbenzene in the synthetic route of method (2) was replaced by *p*-chloroiodobenzene to give compound 27 (white solid, 63.1 mg, 66.7% yield): ¹H NMR (300 MHz, DMSO-*d₆*) δ 12.83 (s, 1H), 7.67 (d, *J* = 8.9 Hz, 2H), 7.46 (d, *J* = 8.9 Hz, 2H), 6.97 (s, 2H), 4.57 (s, 2H), 4.52 (s, 2H), 2.15 (s, 6H), 1.35 (s, 6H). HRMS (ESI) calcd. for C₂₂H₂₃ClN₂O₅ [M+Na]⁺: 453.1193, found: 453.1191.

### Example 28

### Ethyl 2-(4-((3-(4-chlorophenyl)-2,5-dioxoimidazolin-1-yl)methyl)-2,6-dimethylphenoxy)-2-methylpropionate (compound 28)

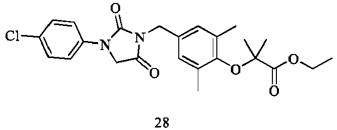

Referring to the method in Example 1, 4-iodotrifluoromethylbenzene in the synthetic route of method (2) was replaced by *p*-chloroiodobenzene to give compound 28 (colorless liquid, 102.5 mg, 62.0% yield) without hydrolysis: ¹H NMR (300 MHz, CDCl₃) δ 7.54 (d, *J* = 9.0 Hz, 2H), 7.36 (d, *J* = 9.0 Hz, 2H), 7.07 (s, 2H), 4.63 (s, 2H), 4.31 (s, 2H), 4.28 (q, *J* = 7.1 Hz, 2H), 2.20 (s, 6H), 1.47 (s, 6H), 1.36 (t, *J* = 7.1 Hz, 3H). MS (ESI): *m*/*z* 481.1[M+Na]⁺.

### Example 29

### 2-(2,6-Dimethyl-4-((3-(3-methyl-4-(trifluoromethyl)phenyl)-2,5-dioxoimidazolin-1-yl)methyl)phenoxy)-2-methylpropionic acid (compound 29)

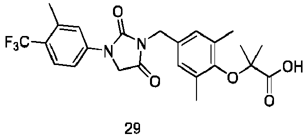

Referring to the method in Example 1, 4-iodotrifluoromethylbenzene in the synthetic route of method (3) was replaced by 4-bromo-2-methyltrifluorotoluene to give compound 29 (white solid, 103.3 mg, 68.2% yield): ¹H NMR (300 MHz, DMSO-*d₆*) δ 12.80 (s, 1H), 7.94 - 7.53 (m, 3H), 6.97 (s, 2H), 4.60 (s, 2H), 4.53 (s, 2H), 2.45 (s, 3H), 2.14 (s, 6H), 1.34 (s, 6H). HRMS (ESI) calcd. for C₂₄H₂₅F₃N₂O₅ [M+Na]⁺: 501.1613, found: 501.1612.

### Example 30

### Ethyl 2-(2,6-dimethyl-4-((3-(3-methyl-4-(trifluoromethyl)phenyl)-2,5-dioxoimidazolin-1-yl)methyl)phenoxy)-2-methylpropionate (compound 30)

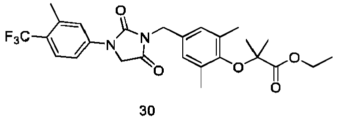

Referring to the method in Example 1, 4-iodotrifluoromethylbenzene in the synthetic route of method (3) was replaced by 4-bromo-2-methyltrifluorotoluene to give compound 30 (colorless liquid, 160.8 mg, 40.9% yield) without hydrolysis: ¹H NMR (300 MHz, CDCl₃) δ 7.62 (d, *J* = 8.4 Hz, 2H), 7.44 (d, *J* = 8.5 Hz, 1H), 7.07 (s, 2H), 4.64 (s, 2H), 4.33 (s, 2H), 4.29 (q, *J* = 7.1 Hz, 2H), 2.52 (s, 3H), 2.20 (s, 6H), 1.46 (s, 6H), 1.35 (t, *J* = 7.1 Hz, 3H). MS (ESI): *m*/*z* 529.2[M+Na]⁺.

### Example 31

### 2-(2,6-Dimethyl-4-((3-(3-fluoro-4-(trifluoromethyl)phenyl)-2,5-dioxoimidazolin-1-yl)methyl)phenoxy)-2-methylpropionic acid (compound 31)

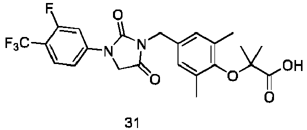

Referring to the method in Example 1, 4-iodotrifluoromethylbenzene in the synthetic route of method (3) was replaced by 4-bromo-2-fluorotrifluorotoluene to give compound 31 (white solid, 170.3 mg, 69.4% yield): ¹H NMR (300 MHz, DMSO-*d₆*) δ 12.84 (s, 1H), 7.86 - 7.78 (m, 2H), 7.68 (d, *J* = 8.6 Hz, 1H), 6.99 (s, 2H), 4.63 (s, 2H), 4.55 (s, 2H), 2.16 (s, 6H), 1.36 (s, 6H). HRMS (ESI) calcd. for C₂₃H₂₂F₄N₂O₅ [M+Na]⁺: 505.1363, found: 505.1363.

### Example 32

### Ethyl 2-(2,6-dimethyl-4-((3-(3-fluoro-4-(trifluoromethyl)phenyl)-2,5-dioxoimidazolin-1-yl)methyl)phenoxy)-2-methylpropionate (compound 32)

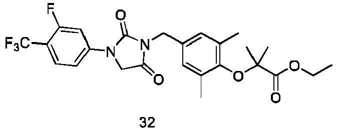

Referring to the method in Example 1, 4-iodotrifluoromethylbenzene in the synthetic route of method (3) was replaced by 4-bromo-2-fluorotrifluorotoluene to give compound 32 (colorless liquid, 260.5 mg, 65.6% yield) without hydrolysis: ¹H NMR (300 MHz, CDCl₃) δ 7.74 (d, *J* = 12.5 Hz, 1H), 7.61 (t, *J* = 8.3 Hz, 1H), 7.30 (d, *J* = 9.0 Hz, 1H), 7.07 (s, 2H), 4.64 (s, 2H), 4.33 (s, 2H), 4.30 (q, *J* = 7.1 Hz, 2H), 2.20 (s, 6H), 1.46 (s, 6H), 1.36 (t, *J* = 7.1 Hz, 3H). MS (ESI): *m*/*z* 533.2[M+Na]⁺.

### Example 33

### 2-(2,6-Dimethyl-4-((3-(3-chloro-4-(trifluoromethyl)phenyl)-2,5-dioxoimidazolin-1-yl)methyl)phenoxy)-2-methylpropionic acid (compound 33)

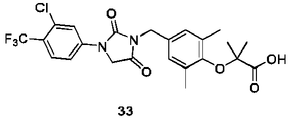

Referring to the method in Example 1, 4-iodotrifluoromethylbenzene in the synthetic route of method (3) was replaced by 2-chloro-4-bromotrifluorotoluene to give compound 33 (white solid, 260.1 mg, 85.8% yield): ¹H NMR (300 MHz, DMSO-*d₆*) δ 12.82 (s, 1H), 8.05 - 7.97 (m, 1H), 7.88 (d, *J* = 8.8 Hz, 1H), 7.84 - 7.75 (m, 1H), 6.97 (s, 2H), 4.63 (s, 2H), 4.53 (s, 2H), 2.14 (s, 6H), 1.34 (s, 6H). HRMS (ESI) calcd. for C₂₃H₂₂ClF₃N₂O₅ [M+Na]⁺: 521.1067, found: 521.1064.

### Example 34

### Ethyl 2-(2,6-dimethyl-4-((3-(3-chloro-4-(trifluoromethyl)phenyl)-2,5-dioxoimidazolin-1-yl)methyl)phenoxy)-2-methylpropionate (compound 34)

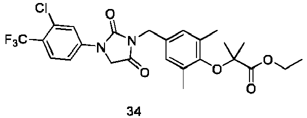

Referring to the method in Example 1, 4-iodotrifluoromethylbenzene in the synthetic route of method (3) was replaced by 2-chloro-4-bromotrifluorotoluene to give compound 34 (colorless liquid, 321.5 mg, 77.7% yield) without hydrolysis: ¹H NMR (300 MHz, CDCl₃) δ 7.90 (s, 1H), 7.69 (d, *J* = 8.8 Hz, 1H), 7.53 (d, *J* = 8.6 Hz, 1H), 7.06 (s, 2H), 4.64 (s, 2H), 4.33 (s, 2H), 4.28 (q, *J* = 7.1 Hz, 2H), 2.20 (s, 6H), 1.46 (s, 6H), 1.36 (t, *J* = 7.1 Hz, 3H). MS (ESI): *m*/*z* 549. 1[M+Na]⁺.

### Example 35

### 2-(4-((2, 5-Dioxo-3-phenylimidazolin-1-yl)methyl)-2, 6-dimethylphenoxy)-2-methylpropionic acid (compound 35)

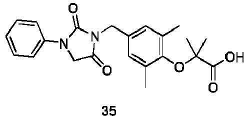

Referring to the method in Example 1, 4-iodotrifluoromethylbenzene in the synthetic route of method (3) was replaced by iodobenzene to give compound 35 (white solid, 157.2 mg, 75.2% yield): ¹H NMR (300 MHz, DMSO-*d₆*) δ 12.82 (s, 1H), 7.65 (d, *J* = 8.1 Hz, 2H), 7.46 - 7.33 (m, 2H), 7.18 - 7.10 (m, 1H), 6.97 (s, 2H), 4.58 (s, 2H), 4.53 (s, 2H), 2.15 (s, 6H), 1.34 (s, 6H). HRMS (ESI) calcd. for C₂₂H₂₄N₂O₅ [M+Na]⁺: 419.1583, found: 419.1573.

### Example 36

### Ethyl 2-(4-((2,5-dioxo-3-phenylimidazolin-1-yl)methyl)-2,6-dimethylphenoxy)-2-methylpropionate (compound 36)

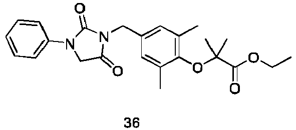

Referring to the method in Example 1, 4-iodotrifluoromethylbenzene in the synthetic route of method (3) was replaced by iodobenzene to give compound 36 (white solid, 226.9 mg, 63.7% yield) without hydrolysis: ¹H NMR (300 MHz, CDCl₃) δ 7.59 (d, *J* = 7.9 Hz, 2H), 7.40 (t, *J* = 8.0 Hz, 2H), 7.46 - 7.35 (m, 2H), 7.08 (s, 2H), 4.64 (s, 2H), 4.33 (s, 2H), 4.29 (q, *J* = 7.9 Hz, 2H), 2.19 (s, 6H), 1.46 (s, 6H), 1.35 (t, *J* = 7.1 Hz, 3H). MS (ESI): *m*/*z* 447.2[M+Na]⁺.

### Example 37

### 2-(4-((3-(4-Cyanobenzene)-2,5-dioxoimidazolin-1-yl)methyl)-2,6-dimethylphenoxy)-2-methylpropionic acid (compound 37)

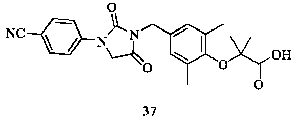

Referring to the method in Example 1, 4-iodotrifluoromethylbenzene in the synthetic route of method (3) was replaced by 4-iodocyanobenzene to give compound 37 (white solid, 177.1 mg, 83.1% yield): ¹H NMR (300 MHz, DMSO-*d₆*) δ 12.82 (s, 1H), 7.88 (d, *J* = 9.0 Hz, 2H), 7.83 (d, *J* = 9.1 Hz, 2H), 6.97 (s, 2H), 4.61 (s, 2H), 4.54 (s, 2H), 2.15 (s, 6H), 1.34 (s, 6H). HRMS (ESI) calcd. for C₂₃H₂₃N₃O₅ [M+Na]⁺: 444.1535, found: 444.1531.

### Example 38

### Ethyl 2-(4-((3-(4-cyanobenzene)-2,5-dioxoimidazolin-1-yl)methyl)-2,6-dimethylphenoxy)-2-methylpropionate (compound 38)

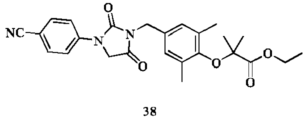

Referring to the method in Example 1, 4-iodotrifluoromethylbenzene in the synthetic route of method (3) was replaced by 4-iodocyanobenzene to give compound 38 (yellow foamy solid, 228 mg, 67.7% yield) without hydrolysis: ¹H NMR (300 MHz, CDCl₃) δ 7.74 (d, *J* = 9.1 Hz, 2H), 7.69 (d, *J* = 9.0 Hz, 2H), 7.07 (s, 2H), 4.65 (s, 2H), 4.35 (s, 2H), 4.29 (q, *J* = 7.1 Hz, 2H), 2.20 (s, 6H), 1.46 (s, 6H), 1.36 (t, *J* = 7.1 Hz, 3H). MS (ESI): *m*/*z* 472.2[M+Na]⁺.

### Example 39

### 2-(4-((3-(4-Methoxyphenyl)-2, 5-dioxoimidazolin-1-yl)methyl)-2, 6-dimethylphenoxy)-2-methylpropionic acid (compound 39)

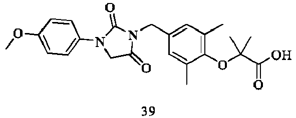

Referring to the method in Example 1, 4-iodotrifluoromethylbenzene in the synthetic route of method (3) was replaced by 4-bromoanisole to give compound 39 (white solid, 143.2 mg, 72.6% yield): ¹H NMR (300 MHz, DMSO-*d₆*) δ 12.81 (s, 1H), 7.54 (d, *J* = 9.0 Hz, 2H), 7.01 - 6.93 (m, 4H), 4.54 (s, 2H), 4.51 (s, 2H), 3.75 (s, 3H), 2.15 (s, 6H), 1.35 (s, 6H). HRMS (ESI) calcd. for C₂₃H₂₆N₂O₆ [M+Na]⁺: 449.1689, found: 449.1685.

### Example 40

### 2-(4-((3-(4-Methoxyphenyl)-2, 5-dioxoimidazolin-1-yl)methyl)-2, 6-dimethylphenoxy)-2-methylpropionic acid (compound 40)

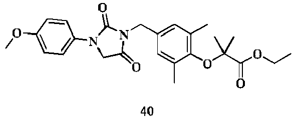

Referring to the method in Example 1, 4-iodotrifluoromethylbenzene in the synthetic route of method (3) was replaced by 4-bromoanisole to give compound 40 (white solid, 211 mg, 61.7% yield) without hydrolysis: ¹H NMR (300 MHz, CDCl₃) δ 7.47 (d, *J* = 9.1 Hz, 2H), 7.07 (s, 2H), 6.93 (d, *J* = 9.1 Hz, 2H), 4.63 (s, 2H), 4.30 (s, 2H), 4.28 (q, *J* = 9.6, 4.6 Hz, 2H), 3.82 (s, 3H), 2.19 (s, 6H), 1.47 (s, 6H), 1.36 (t, *J* = 7.1 Hz, 3H). MS (ESI): *m*/*z* 477.2[M+Na]⁺.

### Example 41

### 2-(4-((3-(4-Biphenyl-2,5-dioxoimidazolin-1-yl)methyl)-2,6-dimethylphenoxy)-2-methylpropionic acid (compound 41)

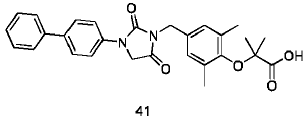

Referring to the method in Example 1, 4-iodotrifluoromethylbenzene in the synthetic route of method (3) was replaced by 4-bromobiphenyl to give compound 41 (white solid, 163.1 mg, 78.7% yield): ¹H NMR (300 MHz, DMSO-*d₆*) δ 12.82 (s, 1H), 7.79 - 7.73 (m, 4H), 7.68 (d, *J* = 7.7 Hz, 2H), 7.53 - 7.41 (m, 2H), 7.39 - 7.19 (m, 1H), 6.99 (s, 2H), 4.63 (s, 2H), 4.55 (s, 2H), 2.16 (s, 6H), 1.35 (s, 6H). HRMS (ESI) calcd. for C₂₈H₂₈N₂O₅ [M+Na]⁺: 495.1896, found: 495.1883.

### Example 42

### 2-(4-((3-(4-Biphenyl-2,5-dioxoimidazolin-1-yl)methyl)-2,6-dimethylphenoxy)-2-methylpropionate (compound 42)

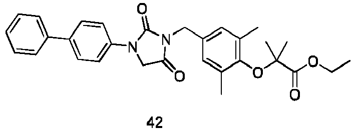

Referring to the method in Example 1, 4-iodotrifluoromethylbenzene in the synthetic route of method (3) was replaced by 4-bromobiphenyl to give compound 42 (white solid, 208.5 mg, 53.9% yield) without hydrolysis: ¹H NMR (300 MHz, CDCl₃) δ 7.67 (d, *J* = 8.8 Hz, 2H), 7.64 (d, *J* = 6.2 Hz, 2H), 7.59 (d, *J* = 8.0 Hz, 2H), 7.46 (t, *J* = 7.5 Hz, 2H), 7.36 (t, *J* = 7.3 Hz, 1H), 7.09 (s, 2H), 4.66 (s, 2H), 4.37 (s, 2H), 4.30 (q, *J* = 7.1 Hz, 2H), 2.21 (s, 6H), 1.47 (s, 6H), 1.36 (t, *J* = 7.1 Hz, 3H). MS (ESI): *m*/*z* 523.2[M+Na]⁺.

### Example 43

### 2-(4-((3-(4-Methylthiophenyl)-2,5-dioxoimidazolin-1-yl)methyl)-2,6-dimethylphenoxy)-2-methylpropionic acid (compound 43)

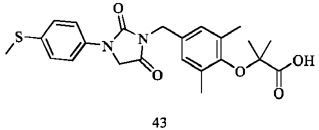

Referring to the method in Example 1, 4-iodotrifluoromethylbenzene in the synthetic route of method (3) was replaced by 4-iodobenzylthiomethane to give compound 43 (yellow solid, 200.3 mg, 81.1% yield): ¹H NMR (300 MHz, DMSO-*d₆*) δ 12.83 (s, 1H), 7.60 (d, *J* = 8.8 Hz, 2H), 7.31 (d, *J* = 8.8 Hz, 2H), 6.97 (s, 2H), 4.56 (s, 2H), 4.52 (s, 2H), 2.47 (s, 3H), 2.15 (s, 6H), 1.36 (s, 6H). HRMS (ESI) calcd. for C₂₃H₂₆N₂O₅S [M+Na]⁺: 465.1460, found: 465.1455.

### Example 44

### Ethyl 2-(4-((3-(4-Methylthiophenyl)-2,5-dioxoimidazolin-1-yl)methyl)-2,6-dimethylphenoxy)-2-methylpropionate (compound 44)

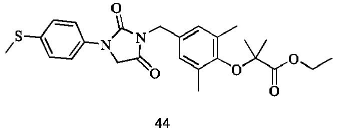

Referring to the method in Example 1, 4-iodotrifluoromethylbenzene in the synthetic route of method (3) was replaced by 4-iodobenzylthiomethane to give compound 44 (yellow solid, 266.5 mg, 74.9% yield) without hydrolysis: ¹H NMR (300 MHz, CDCl₃) δ 7.52 (d, *J* = 8.7 Hz, 2H), 7.30 (d, *J* = 10.1 Hz, 2H), 7.07 (s, 2H), 4.63 (s, 2H), 4.30 (s, 2H), 4.29 (q, *J* = 7.1 Hz, 2H), 2.49 (s, 3H), 2.19 (s, 6H), 1.46 (s, 6H), 1.36 (t, *J* = 7.1 Hz, 3H). MS (ESI): *m*/*z* 493.2[M+Na]⁺.

### Example 45

### 2-(4-((2,5-Dioxo-3-(3-(trifluoromethyl)phenyl)imidazolin-1-yl)methyl)-2,6-dimethylphenoxy)-2-methylpropionic acid (compound 45)

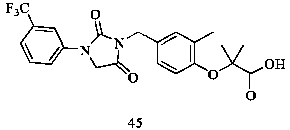

Referring to the method in Example 1, 4-iodotrifluoromethylbenzene in the synthetic route of method (3) was replaced by 3-iodotrifluorotoluene to give compound 45 (white solid, 169.1 mg, 67.1% yield): ¹H NMR (300 MHz, DMSO-*d₆*) δ 12.83 (s, 1H), 8.14 (s, 1H), 7.83 (d, *J* = 8.7 Hz, 1H), 7.69 - 7.61 (m, 1H), 7.49 (d, *J* = 7.8 Hz, 1H), 6.98 (s, 2H), 4.65 (s, 2H), 4.54 (s, 2H), 2.15 (s, 6H), 1.35 (s, 6H). HRMS (ESI) calcd. for C₂₃H₂₃F₃N₂O₅ [M+Na]⁺: 487.1457, found: 487.1451.

### Example 46

### Ethyl 2-(4-((2, 5-dioxo-3-(3-(trifluoromethyl)phenyl)imidazolin-1-yl)methyl)-2,6-dimethylphenoxy)-2-methylpropionate (compound 46)

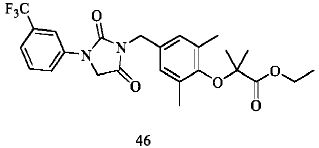

Referring to the method in Example 1, 4-iodotrifluoromethylbenzene in the synthetic route of method (3) was replaced by 3-iodotrifluorotoluene to give compound 46 (yellow liquid, 268.1 mg, 54.5% yield) without hydrolysis: ¹H NMR (300 MHz, CDCl₃) δ 7.91 (s, 1H), 7.77 (d, *J* = 8.0 Hz, 1H), 7.53 (t, *J* = 8.0 Hz, 1H), 7.42 (d, *J* = 7.6 Hz, 1H), 7.07 (s, 2H), 4.65 (s, 2H), 4.36 (s, 2H), 4.29 (q, *J* = 7.1 Hz, 2H), 2.20 (s, 6H), 1.47 (s, 6H), 1.36 (t, *J* = 7.1 Hz, 3H). MS (ESI): *m*/*z* 515.2[M+Na]⁺.

### Example 47

### 2-(2-Chloro-4-((2,5-dioxo-3-(4-(trifluoromethyl)phenyl)imidazolin-1-yl)methyl)-6-fluorophenoxy)-2-methylpropionic acid (compound 47)

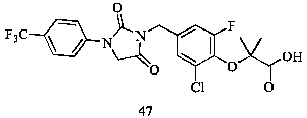

Referring to the method in Example 1, 4-hydroxy-3,5-dimethylbenzaldehyde was replaced by 4-hydroxy-3-fluoro-5-chlorobenzaldehyde to give compound 47 (white solid, 73.3 mg, 64.8% yield): ¹H NMR (300 MHz, DMSO-*d₆*) δ 12.92 (s, 1H), 7.87 (d, *J* = 8.8 Hz, 2H), 7.78 (d, *J* = 8.8 Hz, 2H), 7.35 (s, 1H), 7.27 (d, *J* = 11.6 Hz, 1H), 4.65 (s, 2H), 4.61 (s, 2H), 1.46 (s, 6H). HRMS (ESI) calcd. for C₂₁H₁₇ClF₄N₂O₅ [M+Na]⁺: 511.0660, found: 511.0654.

### Example 48

### Ethyl 2-(2-chloro-4-((2,5-dioxo-3-(4-(trifluoromethyl)phenyl)imidazolin-1-yl)methyl)-6-fluorophenoxy)-2-methylpropionate (compound 48)

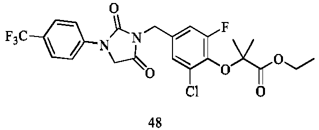

Referring to the method in Example 1, 4-hydroxy-3,5-dimethylbenzaldehyde was replaced by 4-hydroxy-3-fluoro-5-chlorobenzaldehyde to give compound 48 (yellow liquid, 120.6 mg, 46.7% yield) without hydrolysis: ¹H NMR (300 MHz, CDCl₃) δ 7.73 (d, *J* = 9.0 Hz, 2H), 7.67 (d, *J* = 9.1 Hz, 2H), 7.29 (s, 1H), 7.13 (dd, *J* = 10.8, 2.0 Hz, 1H), 4.68 (s, 2H), 4.39 (s, 2H), 4.27 (q, *J* = 7.1 Hz, 2H), 1.57 (s, 6H), 1.33 (t, *J* = 7.1 Hz, 3H). MS (ESI): *m*/*z* 539.1[M+Na]⁺.

### Example 49

### 2-(4-((2,5-Dioxo-3-(4-(trifluoromethyl)phenyl)imidazolin-1-yl)methyl)-2-fluorophenoxy)-2-methylpropionic acid (compound 49)

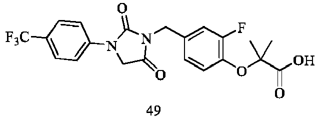

Referring to the method in Example 1, 4-hydroxy-3,5-dimethylbenzaldehyde was replaced by 3-fluoro-4-hydroxybenzaldehyde to give compound 49 (white solid, 47.2 mg, 59.0% yield): ¹H NMR (300 MHz, DMSO-*d₆*) δ 13.14 (s, 1H), 7.86 (d, *J* = 8.8 Hz, 2H), 7.77 (d, *J* = 8.8 Hz, 2H), 7.23 (d, *J* = 12.0 Hz, 1H), 7.09 (d, *J* = 8.6 Hz, 1H), 6.95 (t, *J* = 8.5 Hz, 1H), 4.62 (s, 4H), 1.50 (s, 6H). HRMS (ESI) calcd. for C₂₁H₁₈F₄N₂O₅ [M+Na]⁺: 477.1050, found: 477.1044.

### Example 50

### Ethyl 2-(4-((2,5-dioxo-3-(4-(trifluoromethyl)phenyl)imidazolin-1-yl)methyl)-2-fluorophenoxy)-2-methylpropionate (compound 50)

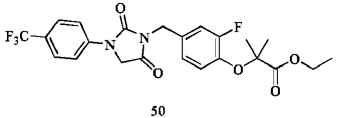

Referring to the method in Example 1, 4-hydroxy-3,5-dimethylbenzaldehyde was replaced by 3-fluoro-4-hydroxybenzaldehyde to give compound 50 (yellow liquid, 85.3 mg, 35.3% yield) without hydrolysis: ¹H NMR (300 MHz, CDCl₃) δ 7.72 (d, *J* = 8.9 Hz, 2H), 7.66 (d, *J* = 9.0 Hz, 2H), 7.22 (dd, *J* = 11.3, 2.0 Hz, 1H), 7.13 (d, *J* = 8.4 Hz, 1H), 6.94 (t, *J* = 8.3 Hz, 1H), 4.70 (s, 2H), 4.36 (s, 2H), 4.26 (q, *J* = 7.1 Hz, 2H), 1.59 (s, 6H), 1.30 (t, *J* = 7.1 Hz, 3H). MS (ESI): m/z 505.1[M+Na]⁺.

### Example 51

### 2-(4-((2,5-Dioxo-3-(4-(trifluoromethyl)phenyl)imidazolin-1-yl)methyl)-2-chlorophenoxy)-2-methylpropionic acid (compound 51)

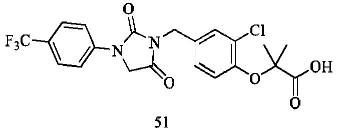

Referring to the method in Example 1, 4-hydroxy-3,5-dimethylbenzaldehyde was replaced by 3-chloro-4-hydroxybenzaldehyde to give compound 51 (white solid, 21.3 mg, 55.7% yield): ¹H NMR (300 MHz, DMSO-*d₆*) δ 13.21 (s, 1H), 7.86 (d, *J* = 8.7 Hz, 2H), 7.77 (d, *J* = 8.8 Hz, 2H), 7.45 (d, *J* = 2.0 Hz, 1H), 7.24 (dd, *J* = 8.5, 2.0 Hz, 1H), 6.88 (d, *J* = 8.5 Hz, 1H), 4.61 (s, 2H), 4.60 (s, 2H), 1.53 (s, 6H). HRMS (ESI) calcd. for C₂₁H₁₈ClF₃N₂O₅ [M+Na]⁺: 493.0754, found: 493.0749.

### Example 52

### Ethyl 2-(4-((2,5-dioxo-3-(4-(trifluoromethyl)phenyl)imidazolin-1-yl)methyl)-2-chlorophenoxy)-2-methylpropionate (compound 52)

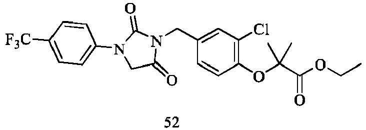

Referring to the method in Example 1, 4-hydroxy-3,5-dimethylbenzaldehyde was replaced by 3-chloro-4-hydroxybenzaldehyde to give compound 52 (yellow liquid, 40.0 mg, 16.0% yield) without hydrolysis: ¹H NMR (300 MHz, CDCl₃) δ 7.72 (d, *J* = 8.9 Hz, 2H), 7.66 (d, *J* = 8.9 Hz, 2H), 7.51 (d, *J* = 2.1 Hz, 1H), 7.29 - 7.21 (m, 1H), 6.85 (d, *J* = 8.4 Hz, 1H), 4.68 (s, 2H), 4.36 (s, 2H), 4.26 (q, *J* = 7.1 Hz, 2H), 1.60 (s, 6H), 1.29 (t, *J* = 7.1 Hz, 3H). MS (ESI): *m*/*z* 521.1[M+Na]⁺.

### Example 53

### 2-(4-((2,5-Dioxo-3-(4-(trifluoromethyl)phenyl)imidazolin-1-yl)methyl)-2,6-dimethylphenoxy)acetic acid (compound 53)

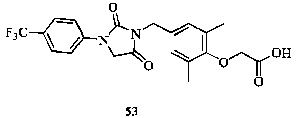

Referring to the method in Example 1, ethyl 2-bromoisobutyrate was replaced by ethyl 2-bromoacetate to give compound 53 (white solid, 73.4 mg, 78.5% yield): ¹H NMR (300 MHz, DMSO-*d₆*) δ 12.86 (s, 1H), 7.86 (d, *J* = 8.8 Hz, 2H), 7.77 (d, *J* = 8.7 Hz, 2H), 7.01 (s, 2H), 4.62 (s, 2H), 4.54 (s, 2H), 4.34 (s, 2H), 2.21 (s, 6H). HRMS (ESI) calcd. for C₂₁H₁₉F₃N₂O₅ [M+Na]⁺: 459.1144, found: 459.1135.

### Example 54

### Ethyl 2-(4-((2,5-dioxo-3-(4-(trifluoromethyl)phenyl)imidazolin-1-yl)methyl)-2,6-dimethylphenoxy)acetate (compound 54)

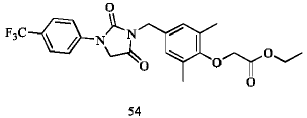

Referring to the method in Example 1, ethyl 2-bromoisobutyrate was replaced by ethyl 2-bromoacetate to give compound 54 (white solid, 99.4 mg, 42.7% yield) without hydrolysis: ¹H NMR (300 MHz, CDCl₃) δ 7.73 (d, *J* = 8.8 Hz, 2H), 7.66 (d, *J* = 8.9 Hz, 2H), 7.12 (s, 2H), 4.67 (s, 2H), 4.38 (s, 2H), 4.36 (s, 2H), 4.32 (q, *J* = 7.1 Hz, 2H), 2.30 (s, 6H), 1.34 (t, *J* = 7.1 Hz, 3H). MS (ESI): *m*/*z* 487.1[M+Na]⁺.

### Example 55

### 2-(4-((2,5-Dioxo-3-(4-(trifluoromethyl)phenyl)imidazolin-1-yl)methyl)-2-(trifluoromethoxy)phenoxy)-2-methylpropionic acid (compound 55)

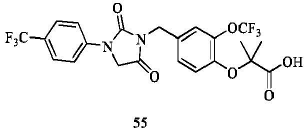

Referring to the method in Example 1, 4-hydroxy-3,5-dimethylbenzaldehyde was replaced by 3-trifluoromethoxy-4-hydroxybenzaldehyde to give compound 55 (white solid, 143.4 mg, 90.5% yield): ¹H NMR (300 MHz, DMSO-*d₆*) δ 13.22 (s, 1H), 7.85 (d, *J* = 8.7 Hz, 2H), 7.77 (d, *J* = 8.7 Hz, 2H), 7.38 (s, 1H), 7.29 (d, *J* = 8.5 Hz, 1H), 6.91 (d, *J* = 8.5 Hz, 1H), 4.63 (s, 2H), 4.61 (s, 2H), 1.52 (s, 6H). HRMS (ESI) calcd. for C₂₂H₁₈F₆N₂O₆ [M+Na]⁺: 543.0967, found: 543.0961.

### Example 56

### Ethyl 2-(4-((2,5-dioxo-3-(4-(trifluoromethyl)phenyl)imidazolin-1-yl)methyl)-2-(trifluoromethoxy)phenoxy)-2-methylpropionate (compound 56)

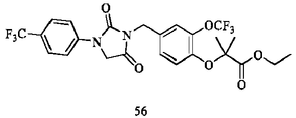

Referring to the method in Example 1, 4-hydroxy-3,5-dimethylbenzaldehyde was replaced by 3-trifluoromethoxy-4-hydroxybenzaldehyde to give compound 56 (yellow liquid, 167.0 mg, 62.2% yield) without hydrolysis: ¹H NMR (300 MHz, CDCl₃) δ 7.72 (d, *J* = 8.9 Hz, 2H), 7.66 (d, *J* = 9.0 Hz, 2H), 7.40 (s, 1H), 7.34 - 7.29 (m, 1H), 6.85 (d, *J* = 8.5 Hz, 1H), 4.71 (s, 2H), 4.36 (s, 2H), 4.24 (q, *J* = 7.1 Hz, 2H), 1.61 (s, 6H), 1.27 (t, *J* = 7.1 Hz, 3H). MS (ESI): *m*/*z* 571.1[M+Na]⁺.

### Example 57

### 2-(4-((2,5-Dioxo-3-(4-(trifluoromethyl)phenyl)imidazolin-1-yl)methyl)-2-(methyl)phenoxy)-2-methylpropionic acid (compound 57)

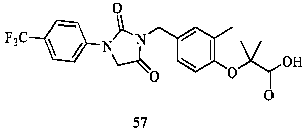

Referring to the method in Example 1, 4-hydroxy-3,5-dimethylbenzaldehyde was replaced by 3-methyl-4-hydroxybenzaldehyde to give compound 57 (white solid, 139.0 mg, 80.3% yield): ¹H NMR (300 MHz, DMSO-*d₆*) δ 13.00 (s, 1H), 7.85 (d, *J* = 8.8 Hz, 2H), 7.76 (d, *J* = 8.7 Hz, 2H), 7.18 - 7.12 (m, 1H), 7.12 - 7.02 (m, 1H), 6.64 (d, *J* = 8.4 Hz, 1H), 4.61 (s, 2H), 4.55 (s, 2H), 2.14 (s, 3H), 1.50 (s, 6H). HRMS (ESI) calcd. for C₂₂H₂₁F₃N₂O₅ [M+Na]⁺: 473.1300, found: 473.1297.

### Example 58

### Ethyl 2-(4-((2,5-dioxo-3-(4-(trifluoromethyl)phenyl)imidazolin-1-yl)methyl)-2-(methyl)phenoxy)-2-methylpropionate (compound 58)

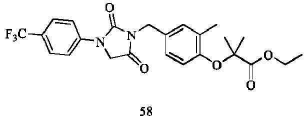

Referring to the method in Example 1, 4-hydroxy-3,5-dimethylbenzaldehyde was replaced by 3-methyl-4-hydroxybenzaldehyde to give compound 58 (yellow liquid, 184.0 mg, 77.0% yield) without hydrolysis: ¹H NMR (300 MHz, CDCl₃) δ 7.72 (d, *J* = 8.9 Hz, 2H), 7.65 (d, *J* = 8.9 Hz, 2H), 7.27 (s, 1H), 7.18 (d, *J* = 8.1 Hz, 1H), 6.61 (d, *J* = 8.3 Hz, 1H), 4.67 (s, 2H), 4.33 (s, 2H), 4.25 (q, *J* = 7.1 Hz, 2H), 2.23 (s, 3H), 1.58 (s, 6H), 1.27 (t, *J* = 7.1 Hz, 3H). MS (ESI): *m*/*z* 501.1[M+Na]⁺.

### Example 59

### 2-(4-((2,5-Dioxo-3-(4-(trifluoromethyl)phenyl)imidazolin-1-yl)methyl)phenoxy)-2-methylpropionic acid (compound 59)

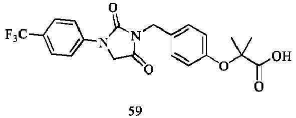

Referring to the method in Example 1, 4-hydroxy-3,5-dimethylbenzaldehyde was replaced by p-hydroxybenzaldehyde to give compound 59 (white solid, 273.4 mg, 89.5% yield): ¹H NMR (300 MHz, DMSO-*d₆*) δ 13.02 (s, 1H), 7.85 (d, *J* = 8.8 Hz, 2H), 7.76 (d, *J* = 8.8 Hz, 2H), 7.26 (d, *J* = 8.5 Hz, 2H), 6.79 (d, *J* = 8.5 Hz, 2H), 4.61 (s, 2H), 4.59 (s, 2H), 1.50 (s, 6H). HRMS (ESI) calcd. for C₂₁H₁₉F₃N₂O₅ [M+Na]⁺: 459.1144, found: 459.1137.

### Example 60

### Ethyl 2-(4-((2, 5-dioxo-3-(4-(trifluoromethyl)phenyl)imidazolin-1-yl)methyl)phenoxy)-2-methylpropionate (compound 60)

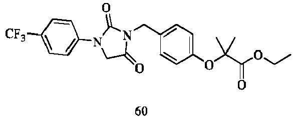

Referring to the method in Example 1, 4-hydroxy-3,5-dimethylbenzaldehyde was replaced by p-hydroxybenzaldehyde to give compound 60 (white solid, 237 mg, 97.2% yield) without hydrolysis: ¹H NMR (300 MHz, CDCl₃) δ 7.72 (d, *J* = 8.8 Hz, 2H), 7.65 (d, *J* = 8.9 Hz, 2H), 7.37 (d, *J* = 8.6 Hz, 2H), 6.81 (d, *J* = 8.6 Hz, 2H), 4.71 (s, 2H), 4.34 (s, 2H), 4.24 (q, 7.1 Hz, 2H), 1.60 (s, 6H), 1.27 (t, *J* = 7.1 Hz, 3H). MS (ESI): *m*/*z* 487.2 [M+Na]⁺.

### Example 61

### 2-(4-((3-(4-Bromophenyl)-2,5-dioxoimidazolin-1-yl)methyl)-2,6-dimethylphenoxy)-2-methylpropionic acid (compound 61)

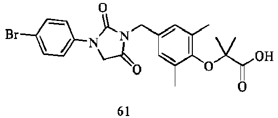

Referring to the method in Example 1, *p*-trifluoromethylaniline was replaced by p-bromoaniline to give compound 61 (white solid, 225.0 mg, 78.9% yield): ¹H NMR (300 MHz, DMSO-*d₆*) δ 12.85 (s, 1H), 7.65 - 7.55 (m, 4H), 6.96 (s, 2H), 4.56 (s, 2H), 4.51 (s, 2H), 2.14 (s, 6H), 1.33 (s, 6H). HRMS (ESI) calcd. for C₂₂H₂₃BrN₂O₅ [M+Na]⁺: 497.0688, found: 497.0675.

### Example 62

### Ethyl 2-(4-((3-(4-bromophenyl)-2,5-dioxoimidazolin-1-yl)methyl)-2,6-dimethylphenoxy)-2-methylpropionate (compound 62)

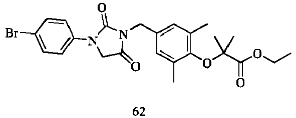

Referring to the method in Example 1, *p*-trifluoromethyl aniline was replaced by p-bromoaniline to give compound 62 (white solid, 356.0 mg, 48.0% yield) without hydrolysis: ¹H NMR (300 MHz, CDCl₃) δ 7.52 - 7.45 (m, 4H), 7.05 (s, 2H), 4.61 (s, 2H), 4.31 - 4.24 (m, 4H), 2.17 (s, 6H), 1.44 (s, 6H), 1.34 (t, J = 7.1 Hz, 3H). MS (ESI): *m*/*z* 527.1[M+Na]⁺.

### Example 63

### 2-(4-((2,5-Dioxo-3-(4-(trifluoromethyl)phenyl)imidazolin-1-yl)methyl)-2,6-dimethylphenoxy)propionic acid (compound 63)

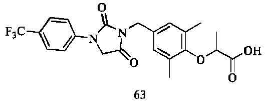

Referring to the method in Example 1, ethyl 2-bromoisobutyrate was replaced by ethyl *p*-2-bromopropionate to give compound 63 (white solid, 82.3 mg, 58.8% yield): ¹H NMR (300 MHz, DMSO-*d₆*) δ 12.86 (s, 1H), 7.86 (d, *J* = 8.6 Hz, 2H), 7.77 (d, *J* = 8.7 Hz, 2H), 6.99 (s, 2H), 4.62 (s, 2H), 4.54 (s, 2H), 4.38 (q, *J* = 6.7 Hz, 1H), 2.20 (s, 6H), 1.40 (d, *J=* 6.7 Hz, 3H). HRMS (ESI) calcd. for C₂₂H₂₁F₃N₂O₅ [M+Na]⁺: 473.1300, found: 473.1300.

### Example 64

### Ethyl 2-(4-((2,5-dioxo-3-(4-(trifluoromethyl)phenyl)imidazolin-1-yl)methyl)-2,6-dimethylphenoxy)propionate (compound 64)

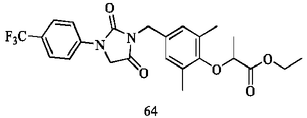

Referring to the method in Example 1, ethyl 2-bromoisobutyrate was replaced by ethyl *p*-2-bromopropionate to give compound 64 (colorless liquid, 160 mg, 66.9% yield) without hydrolysis: ¹H NMR (300 MHz, CDCl₃) δ 7.73 (d, *J* = 8.7 Hz, 2H), 7.66 (d, *J* = 8.8 Hz, 2H), 7.11 (s, 2H), 4.66 (s, 2H), 4.47 (q, *J* = 6.8 Hz, 1H), 4.36 (s, 2H), 4.24 (q, *J* = 7.0 Hz, 2H), 2.29 (s, 6H), 1.53 (d, *J* = 6.7 Hz, 3H), 1.29 (t, *J* = 7.1 Hz, 3H). MS (ESI): *m*/*z* 501.2 [M+Na]⁺.

### Example 65

### 2-(4-((3-(4-Isopropylphenyl)-2,5-dioxoimidazolin-1-yl)methyl)-2,6-dimethylphenoxy)-2-methylpropionic acid (compound 65)

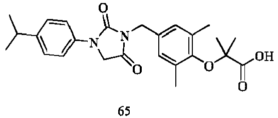

Referring to the method in Example 1, 4-iodotrifluoromethylbenzene in the synthetic route of method (3) was replaced by 4-bromocumene to give compound 65 (white solid, 112.4 mg, 64.4% yield): ¹H NMR (300 MHz, DMSO-*d₆*) δ 12.82 (s, 1H), 7.54 (d, *J* = 8.5 Hz, 2H), 7.26 (d, *J* = 8.5 Hz, 2H), 6.96 (s, 2H), 4.55 (s, 2H), 4.51 (s, 2H), 3.03 - 2.69 (m, 1H), 2.14 (s, 6H), 1.34 (s, 6H), 1.19 (d, *J* = 6.9 Hz, 6H). HRMS (ESI) calcd. for C₂₅H₃₀N₂O₅ [M+Na]⁺: 461.2052, found: 461.2046.

### Example 66

### Ethyl 2-(4-((3-(4-isopropylphenyl)-2,5-dioxoimidazolin-1-yl)methyl)-2,6-dimethylphenoxy)-2-methylpropionate (compound 66)

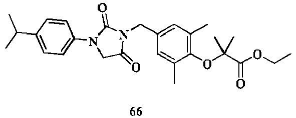

Referring to the method in Example 1, 4-iodotrifluoromethylbenzene in the synthetic route of method (3) was replaced by 4-bromocumene to give compound 66 (yellow liquid, 185.3 mg, 39.7% yield) without hydrolysis: ¹H NMR (300 MHz, CDCl₃) δ 7.49 (d, *J* = 8.6 Hz, 2H), 7.26 (d, *J* = 8.6 Hz, 2H), 7.07 (s, 2H), 4.64 (s, 2H), 4.32 (t, *J* = 7.1 Hz, 2H), 4.30 (q, *J* = 7.1 Hz, 2H), 2.91 (dt, *J* = 13.8, 6.9 Hz, 1H), 2.19 (s, 6H), 1.46 (s, 6H), 1.36 (t, *J* = 7.1 Hz, 3H), 1.25 (d, *J* = 6.9 Hz, 6H). MS (ESI): *m*/*z* 489.2 [M+Na]⁺.

### Example 67

### 2-(4-((2,5-Dioxo-3-(4-(trifluoromethyl)phenyl)imidazolin-1-yl)methyl)-2,6-dimethylphenoxy)butyric acid (compound 67)

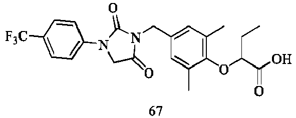

Referring to the method in Example 1, ethyl 2-bromoisobutyrate was replaced by ethyl 2-bromobutyrate to give compound 67 (white solid, 132.0 mg, 72.1% yield): *¹*H NMR (300 MHz, DMSO-*d₆*) δ 12.87 (s, 1H), 7.86 (d, *J* = 8.6 Hz, 2H), 7.77 (d, *J* = 8.7 Hz, 2H), 6.99 (s, 2H), 4.63 (s, 2H), 4.54 (s, 2H), 4.30 (t, *J* = 5.9 Hz, 1H), 2.22 (s, 6H), 1.86 (dt, 2H), 0.96 (t, *J* = 7.3 Hz, 3H). HRMS (ESI) calcd. for C₂₃H₂₃F₃N₂O₅ [M+Na]⁺: 487.1457, found: 487.1451.

### Example 68

### Ethyl 2-(4-((2,5-dioxo-3-(4-(trifluoromethyl)phenyl)imidazolin-1-yl)methyl)-2,6-dimethylphenoxy)butyrate (compound 68)

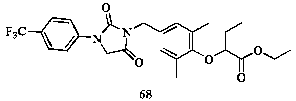

Referring to the method in Example 1, ethyl 2-bromoisobutyrate was replaced by ethyl 2-bromobutyrate to give compound 68 (colorless liquid, 194.6 mg, 78.9% yield) without hydrolysis: ¹H NMR (300 MHz, CDCl₃) δ 7.73 (d, *J* = 8.7 Hz, 2H), 7.65 (d, *J* = 8.9 Hz, 2H), 7.09 (s, 2H), 4.65 (s, 2H), 4.39 (t, *J* = 7.5 Hz, 1H), 4.35 (s, 2H), 4.20 (q, *J* = 6.1 Hz, 2H), 2.28 (s, 6H), 1.97 (dt, 2H), 1.26 (t, *J* = 6.6 Hz, 3H), 1.02 (t, *J* = 7.5 Hz, 3H). MS (ESI): *m*/*z* 515.2 [M+Na]⁺.

### Example 69

### 2-(4-((3-(4-(tert-Butyl)phenyl)-2,5-dioxoimidazolin-1-yl)methyl)-2,6-dimethylphenoxy)-2-methylpropionic acid (compound 69)

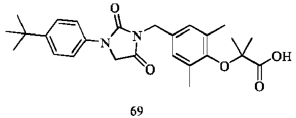

Referring to the method in Example 1, 4-iodotrifluoromethylbenzene in the synthetic route of method (3) was replaced by 4-*tert*-butylbromobenzene to give compound 69 (white solid, 81.7 mg, 53.8% yield): ¹H NMR (300 MHz, DMSO-*d₆*) δ 12.81 (s, 1H), 7.53 (d, *J* = 8.7 Hz, 2H), 7.39 (d, *J* = 8.7 Hz, 2H), 6.94 (s, 2H), 4.54 (s, 2H), 4.50 (s, 2H), 2.13 (s, 6H), 1.32 (s, 6H), 1.26 (s, 9H). HRMS (ESI) calcd. for C₂₆H₃₂N₂O₅ [M+Na]⁺: 475.2209, found: 475.2203.

### Example 70

### Ethyl 2-(4-((3-(4-(tert-butyl)phenyl)-2,5-dioxoimidazolin-1-yl)methyl)-2,6-dimethylphenoxy)-2-methylpropionate (compound 70)

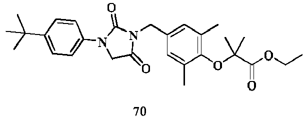

Referring to the method in Example 1, 4-iodotrifluoromethylbenzene in the synthetic route of method (3) was replaced by 4-*tert*-butylbromobenzene to give compound 70 (white solid, 147 mg, 61.2% yield) without hydrolysis: ¹H NMR (300 MHz, DMSO-*d₆*) δ 7.54 (d, *J* = 8.7 Hz, 2H), 7.40 (d, *J* = 8.8 Hz, 2H), 6.96 (s, 2H), 4.54 (d, *J* = 7.1 Hz, 2H), 4.16 (q, *J* = 7.1 Hz, 2H), 2.10 (s, 2H), 1.36 (s, 6H), 1.27 (s, 6H), 1.23 (s, 9H), 1.21 (t, *J* = 7.1 Hz, 3H). MS (ESI): *m*/*z* 503.2 [M+Na]⁺.

### Example 71

### 2-(2,6-Dimethyl-4-((3-(4-(methylsulfonyl)phenyl)-2,5-dioxoimidazolin-1-yl)methyl)phenoxy)-2-methylpropionic acid (compound 71)

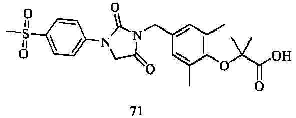

Referring to the method in Example 1, 4-iodotrifluoromethylbenzene in the synthetic route of method (3) was replaced by 4-bromophenyl methyl sulfone to give compound 71 (white solid, 37.2 mg, 26.2% yield): ¹H NMR (300 MHz, DMSO-*d₆*) δ 12.79 (s, 1H), 8.30 - 7.55 (m, 4H), 6.96 (s, 2H), 4.62 (s, 2H), 4.52 (s, 2H), 3.17 (s, 3H), 2.13 (s, 6H), 1.32 (s, 6H). HRMS (ESI) calcd. for C₂₃H₂₆N₂O₇S [M+Na]⁺: 497.1358, found: 497.1346.

### Example 72

### Ethyl 2-(2,6-dimethyl-4-((3-(4-(methylsulfonyl)phenyl)-2,5-dioxoimidazolin-1-yl)methyl)phenoxy)-2-methylpropionate (compound 72)

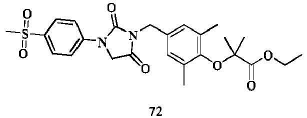

Referring to the method in Example 1, 4-iodotrifluoromethylbenzene in the synthetic route of method (3) was replaced by 4-bromophenyl methyl sulfone to give compound 72 (yellow solid, 131.2 mg, 27.8% yield) without hydrolysis: ¹H NMR (300 MHz, DMSO-*d₆*) δ 7.92 (q, *J* = 9.1 Hz, 4H), 6.99 (s, 2H), 4.64 (s, 2H), 4.55 (s, 2H), 4.17 (q, *J* = 7.1 Hz, 2H), 3.34 (s, 3H), 2.11 (s, 6H), 1.37 (s, 6H), 1.24 (t, *J* = 7.1 Hz, 3H). MS (ESI): *m*/*z* 525.2 [M+Na]⁺.

### Example 73

### 2-(2-Chloro-4-((2,5-dioxo-3-(4-(trifluoromethyl)phenyl)imidazolin-1-yl)methyl)-6-methoxyphenoxy)-2-methylpropionic acid (compound 73)

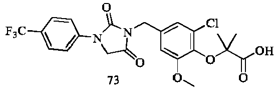

Referring to the method in Example 1, 4-hydroxy-3,5-dimethylbenzaldehyde was replaced by 4-hydroxy-3-chloro-5-methoxybenzaldehyde to give compound 73 (white solid, 100.2 mg, 66.7% yield): ¹H NMR (300 MHz, DMSO-*d₆*) δ 12.45 (s, 1H), 7.85 (d, *J* = 8.6 Hz, 2H), 7.75 (d, *J* = 8.7 Hz, 2H), 6.97 (d, *J* = 6.8 Hz, 2H), 4.60 (s, 4H), 3.69 (s, 3H), 1.34 (s, 6H). HRMS (ESI) calcd. for C₂₂H₂₀ClF₃N₂O₆ [M+Na]⁺: 523.0860, found: 523.0854.

### Example 74

### Ethyl 2-(2-chloro-4-((2,5-dioxo-3-(4-(trifluoromethyl)phenyl)imidazolin-1-yl)methyl)-6-methoxyphenoxy)-2-methylpropionate (compound 74)

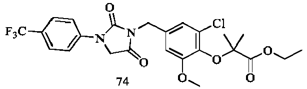

Referring to the method in Example 1, 4-hydroxy-3,5-dimethylbenzaldehyde was replaced by 4-hydroxy-3-chloro-5-methoxybenzaldehyde to give compound 74 (colorless liquid, 190.2 mg, 72.3% yield) without hydrolysis: ¹H NMR (300 MHz, DMSO-*d₆*) δ 7.86 (d, *J* = 8.7 Hz, 2H), 7.77 (d, *J* = 8.8 Hz, 2H), 6.99 (d, *J* = 8.9 Hz, 2H), 4.62 (s, 4H), 4.15 (q, *J* = 7.1 Hz, 2H), 3.70 (s, 3H), 1.37 (s, 6H), 1.23 (t, *J* = 7.2 Hz, 3H). MS (ESI): *m*/*z* 551.1 [M+Na]⁺.

### Example 75

### 2-(4-(3-(4-Ethylphenyl)-2, 5-dioxoimidazolidin-1-yl)methyl)-2, 6-dimethylphenoxy-2-methylpropionic acid (compound 75)

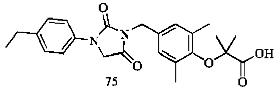

Referring to the method in Example 1, 4-iodotrifluoromethylbenzene in the synthetic route of method (3) was replaced by 1-ethyl-4-iodobenzene to give compound 75 (white solid, 79.2 mg, 70.5% yield): ¹H NMR (300 MHz, DMSO-*d₆*) δ 12.85 (s, 1H), 7.54 (d, *J* = 8.5 Hz, 2H), 7.23 (d, *J* = 8.5 Hz, 2H), 6.96 (s, 2H), 4.56 (s, 2H), 4.52 (s, 1H), 2.58 (q, *J,* 7.5 Hz, 2H), 2.14 (s, 6H), 1.34 (s, 6H), 1.16 (t, *J* = 7.5 Hz, 3H). HRMS (ESI) calcd. for C₂₄H₂₈N₂O₅ [M+Na]⁺: 447.1896, found: 447.1894.

### Example 76

### Ethyl 2-(4-(3-(4-ethylphenyl)-2,5-dioxoimidazolidin-1-yl)methyl)-2,6-dimethylphenoxy-2-methylpropionate (compound 76)

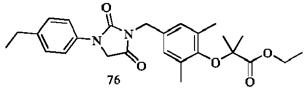

Referring to the method in Example 1, 4-iodotrifluoromethylbenzene in the synthetic route of method (3) was replaced by 1-ethyl-4-iodobenzene to give compound 76 (white solid, 120.0 mg, 26.4% yield) without hydrolysis: ¹H NMR (300 MHz, CDCl₃) δ 7.49 (d, *J* = 8.5 Hz, 2H), 7.23 (d, *J* = 8.5 Hz, 2H), 7.08 (s, 2H), 4.64 (s, 2H), 4.32 (s, 2H), 4.29 (q, *J* = 7.1 Hz, 2H), 2.65 (q, *J* = 7.5 Hz, 2H), 2.20 (s, 6H), 1.47 (s, 6H), 1.36 (t, *J* = 7.1 Hz, 3H), 1.26 (t, *J* = 7.7 Hz, 3H). MS (ESI): *m*/*z* 475.2 [M+Na]⁺.

### Example 77

### 2-(2-Bromo-4-((2, 5-dioxo-3 -(4-trifluoromethyl)phenyl)imidazolidin-1-yl)methylphenoxy)-2-methylpropionic acid (compound 77)

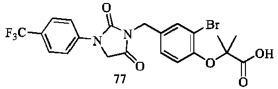

Referring to the method in Example 1, 4-hydroxy-3,5-dimethylbenzaldehyde was replaced by 4-hydroxy-3-bromobenzaldehyde to give compound 77 (white solid, 97.3 mg, 76.4% yield): ¹H NMR (300 MHz, DMSO-*d₆*) δ 13.28 (s, 1H), 7.86 (d, *J* = 8.7 Hz, 2H), 7.77 (d, *J* = 8.9 Hz, 2H), 7.60 (s, 1H), 7.28 (d, *J* = 7.9 Hz, 1H), 6.85 (d, *J* = 8.4 Hz, 1H), 4.61 (s, 2H), 4.60 (s, 2H), 1.53 (s, 6H). HRMS (ESI) calcd. for C₂₁H₁₈BrF₃N₂O₅ [M+Na]⁺: 537.0249, found: 537.0243.

### Example 78

### Ethyl 2-(2-bromo-4-((2,5-dioxo-3-(4-trifluoromethyl)phenyl)imidazolidin-1-yl)methylphenoxy)-2-methylpropionate (compound 78)

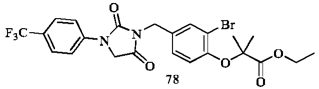

Referring to the method in Example 1, 4-hydroxy-3,5-dimethylbenzaldehyde was replaced by 4-hydroxy-3-bromobenzaldehyde to give compound 78 (white solid, 134.3 mg, 49.4% yield) without hydrolysis: ¹H NMR (300 MHz, DMSO-*d₆*) δ 7.86 (d, *J* = 8.8 Hz, 2H), 7.77 (d, *J* = 8.8 Hz, 2H), 7.62 (s, 1H), 7.28 (d, *J* = 6.4 Hz, 1H), 6.79 (d, *J* = 8.5 Hz, 1H), 4.64 - 4.55 (m, 4H),, 4.19 (dd, *J* = 14.1, 7.1 Hz, 2H), 1.55 (s, 6H), 1.19 (t, *J* = 7.1 Hz, 3H). MS (ESI): *m*/*z* 565.1 [M+Na]⁺.

### Example 79

### 2-(4-((4-Ethyl-4-methyl-2, 5-dioxo-3-(4-(trifluoromethyl)phenyl)imidazolin-1-yl)methyl)-2,6-dimethylphenoxy)-2-methylpropionic acid (compound 79)

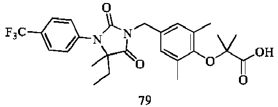

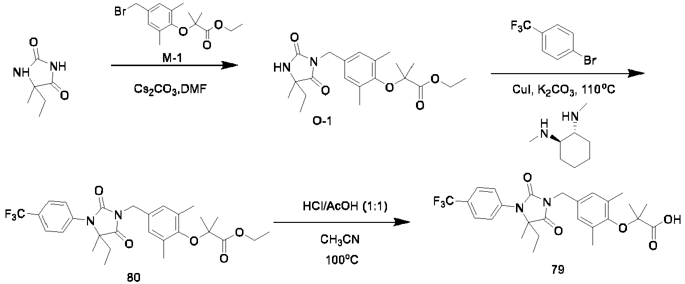

### Synthesis of intermediate O-1

5-Ethyl-5-methylimidazolidine-2,4-dione (142 mg, 1 mmol) was dissolved in DMF (5 mL), and M-1 (492 mg, 1.5 mmol) and cesium carbonate (652 mg, 2 mmol) were added. The mixture was stirred at room temperature for 12 h. After the reaction was completed, water (20 mL) was added, and the resulting solution was extracted with EA (25 mL × 3). The organic phase was washed with saturated sodium chloride solution (20 mL × 1). The solvent was distilled off under reduced pressure, and the residue was purified by silica gel column chromatography (petroleum ether/ethyl acetate = 2:1) to give intermediate O-1 (yellow liquid, 150 mg, 38.4% yield).

### Synthesis of compound 80

Intermediate 0-1 (150 mg, 0.4 mmol), cuprous iodide (15 mg, 0.08 mmol), potassium carbonate (110 mg, 0.8 mmol), and (1R,2R)-(-)-N,N'-dimethyl-1,2-cyclohexanediamine (23 mg, 0.16 mmol) were added to a Schlenk tube. Under argon atmosphere, a solution of *p-*trifluoromethylbromobenzene (107 mg, 0.48 mmol) in toluene (3 mL) was added, and the reaction system was heated to 110 °C. After the reaction was completed, the solvent was distilled off under reduced pressure, and the residue was purified by silica gel column chromatography (petroleum ether/ethyl acetate = 5:1) to give compound 80 (colorless liquid, 47 mg, 22.1% yield).

### Synthesis of compound 79

Compound 80 (47 mg, 0.09 mmol) was dissolved in acetonitrile (3 mL), and a mixed solution of concentrated hydrochloric acid (at a concentration of 12 M) and acetic acid (4 mL, 1:1) was added. The reaction system was heated to 100 °C and stirred for 4 h. After the reaction was completed, the solvent was distilled off under reduced pressure, water (10 mL) was added, and the resulting solution was extracted with EA (15 mL × 3). The organic phase was washed with saturated sodium chloride solution (10 mL × 1). The solvent was distilled off under reduced pressure, and the residue was purified by silica gel column chromatography (dichloromethane/methanol = 100:1) to give compound 79 (white solid, 21 mg, 55.4% yield): ¹H NMR (300 MHz, DMSO-*d₆*) δ 12.84 (s, 1H), 7.84 (d, *J* = 8.5 Hz, 2H), 7.64 (d, *J* = 8.3 Hz, 2H), 6.95 (s, 2H), 4.58 (s, 2H), 2.15 (s, 6H), 2.01 - 1.65 (m, 2H), 1.43 (s, 3H), 1.34 (s, 6H), 0.68 (t, *J* = 7.2 Hz, 3H). HRMS (ESI) calcd. for C₂₆H₂₉F₃N₂O₅ [M+NH₄]⁺ 524.2372, found 524.2370.

### Example 80

### Ethyl 2-(4-((4-ethyl-4-methyl-2,5-dioxo-3-(4-(trifluoromethyl)phenyl)imidazolin-1-yl)methyl)-2,6-dimethylphenoxy)-2-methylpropionate (compound 80)

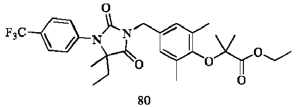

Referring to the method in Example 79, compound 80 (white solid, 47 mg, 22.1% yield) was prepared without hydrolysis: ¹H NMR (300 MHz, CDCl₃) δ 7.70 (d, *J* = 8.5 Hz, 2H), 7.46 (d, *J* = 8.3 Hz, 2H), 7.07 (s, 2H), 4.65 (s, 2H), 4.30 (q, *J* = 7.1 Hz, 2H), 2.19 (s, 6H), 2.06 - 1.93 (m, 2H), 1.79 - 1.66 (m, 3H), 1.46 (s, 6H), 1.36 (t, *J* = 7.1 Hz, 3H), 0.71 (t, *J* = 7.3 Hz, 3H). MS (ESI): *m*/*z* 557.2[M+Na]⁺.

### Example 81

### 2-(4-((2,4-Dioxo-3-(4-(trifluoromethyl)phenyl)imidazolidin-1-yl)methyl)-2,6-dimethylphenoxy)-2-methylpropionic acid (compound 81)

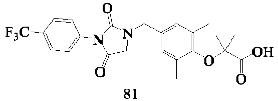

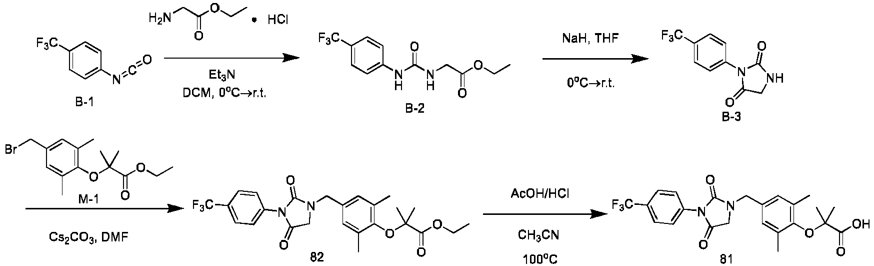

### Synthesis of intermediate B-2

p-Trifluoromethyl isocyanate (1.87 g, 10 mmol) was dissolved in dichloromethane (15 mL), triethylamine (1.66 mL, 12 mmol) was added, and glycine ethyl ester hydrochloride (1.67 g, 12 mmol) was added in an ice bath. The mixture was stirred at room temperature overnight. After the reaction was completed, the solvent was distilled off under reduced pressure, water (50 mL) was added, and the resulting solution was extracted with EA (50 mL × 3). The organic phase was washed with saturated sodium chloride solution (50 mL × 1). The solvent was distilled off under reduced pressure, and the residue was purified by silica gel column chromatography (petroleum ether/ethyl acetate = 40: 1) to give intermediate B-2 (white solid, 2.4 g, 82.7% yield).

### Synthesis of intermediate B-3

Intermediate B-2 (1.45 g, 5 mmol) was dissolved in tetrahydrofuran (15 mL), and sodium hydride (160 mg, 4 mmol) was added in an ice bath. The mixture was stirred at room temperature for 3 h. After the reaction was completed, water (20 mL) was added for quenching, and the solvent was distilled off under reduced pressure. Water (20 mL) was added, and the resulting solution was extracted with EA (25 mL × 3). The organic phase was washed with saturated sodium chloride solution (20 mL × 1). The solvent was distilled off under reduced pressure, and the residue was purified by silica gel column chromatography (petroleum ether/ethyl acetate = 2:1) to give intermediate B-3 (white solid, 486.1 mg, 39.8% yield).

### Synthesis of compound 82

Intermediate B-3 (296 mg, 1.2 mmol) was dissolved in DMF (3 mL), and M-1 (492 mg, 1.5 mmol) and cesium carbonate (782 mg, 2.4 mmol) were added. The mixture was stirred at room temperature for 12 h. After the reaction was completed, the solvent was distilled off under reduced pressure, and the residue was purified by silica gel column chromatography (petroleum ether/ethyl acetate = 5:1) to give compound 82 (colorless liquid, 45 mg, 7.5% yield).

### Synthesis of compound 81

Compound 82 (45 mg, 0.09 mmol) was dissolved in acetonitrile (2 mL), and a mixed solution of concentrated hydrochloric acid (at a concentration of 12 M) and glacial acetic acid (4 mL, 1:1) was added. The reaction system was heated to 100 °C and stirred for 4 h. After the reaction was completed, the solvent was distilled off under reduced pressure, water (10 mL) was added, and the resulting solution was extracted with EA (15 mL × 3). The organic phase was washed with saturated sodium chloride solution (10 mL × 1). The solvent was distilled off under reduced pressure, and the residue was purified by silica gel column chromatography (dichloromethane/methanol = 100:1) to give compound 81 (white solid, 10.3 mg, 25.7% yield): ¹H NMR (300 MHz, DMSO-*d₆*) δ 12.84 (s, 1H), 7.89 (d, *J* = 8.5 Hz, 2H), 7.71 (d, *J* = 8.3 Hz, 2H), 7.01 (s, 2H), 4.47 (s, 2H), 4.06 (s, 2H), 2.18 (s, 6H), 1.36 (s, 6H). HRMS (ESI) calcd. for C₂₃H₂₃F₃N₂O₅ [M+NH₄]⁺ 482.1903, found 482.1902.

### Example 82

### Ethyl 2-(4-((2,4-dioxo-3-(4-(trifluoromethyl)phenyl)imidazolidin-1-yl)methyl)-2,6-dimethylphenoxy)-2-methylpropionate (compound 82)

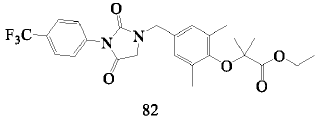

Referring to the method in Example 81, compound 82 (colorless liquid, 45 mg, 7.5% yield) was prepared without hydrolysis: ¹H NMR (300 MHz, CDCl₃) δ 7.75 (d, *J* = 8.5 Hz, 2H), 7.68 (d, *J* = 8.4 Hz, 2H), 6.93 (s, 2H), 4.54 (s, 2H), 4.31 (q, *J* = 7.2 Hz, 2H), 3.93 (s, 2H), 2.23 (s, 6H), 1.49 (s, 6H), 1.38 (t, *J* = 7.2 Hz, 3H). MS (ESI): *m*/*z* 515.2[M+Na]⁺.

### Example 83

### 2-(4-((4,4-Dimethyl-2, 5-dioxo-3-(4-(trifluoromethoxy)phenyl)imidazolidin-1-yl)methyl)-2,6-dimethylphenoxy)-2-methylpropionic acid (compound 83)

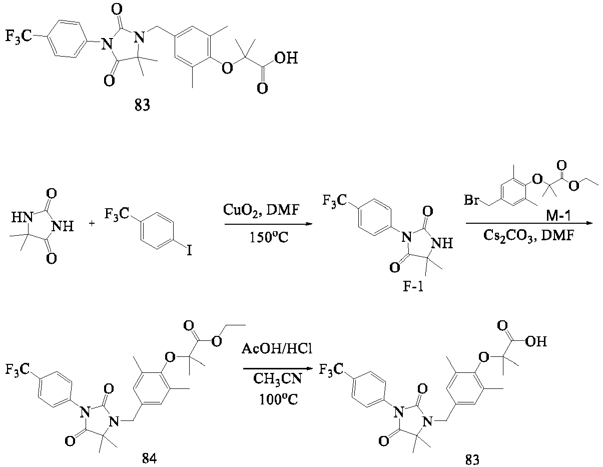

### Synthesis of intermediate F-1

p-Iodotrifluoromethylbenzene (271 mg, 1 mmol), cuprous oxide (141 mg, 1 mmol), and 5,5-dimethylhydantoin (192 mg, 1.5 mmol) were added to a three-necked flask, and the reaction system was purged with argon. Anhydrous DMF (3 mL) was then added, the reaction system was heated to 150 °C and reacted for 12 h. After the reaction was completed, the reaction solution was filtered through celite, water (10 mL) was added, and the resulting solution was extracted with EA (10 mL × 3) and washed with saturated sodium chloride (10 mL × 1). The solvent was distilled off under reduced pressure, and the residue was purified by silica gel column chromatography (petroleum ether/ethyl acetate = 5:1) to give compound F-1 (pale yellow solid, 160 mg, 58.2% yield).

### Synthesis of compound 84

Intermediate F-1 (50 mg, 0.2 mmol) was dissolved in DMF (2 mL), and M-1 (98 mg, 0.3 mmol) and cesium carbonate (163 mg, 0.5 mmol) were added. The mixture was stirred at room temperature for 12 h. After the reaction was completed, the solvent was distilled off under reduced pressure, and the residue was purified by silica gel column chromatography (petroleum ether/ethyl acetate = 5:1) to give compound 84 (colorless liquid, 80 mg, 76.2% yield).

### Synthesis of compound 83

Compound 84 (80 mg, 0.15 mmol) was dissolved in acetonitrile (2 mL), and a mixed solution of concentrated hydrochloric acid (at a concentration of 12 M) and glacial acetic acid (4 mL, 1:1) was added. The reaction system was heated to 100 °C and stirred for 4 h. After the reaction was completed, the solvent was distilled off under reduced pressure, water (10 mL) was added, and the resulting solution was extracted with EA (15 mL × 3). The organic phase was washed with saturated sodium chloride solution (10 mL × 1). The solvent was distilled off under reduced pressure, and the residue was purified by silica gel column chromatography (dichloromethane/methanol = 100:1) to give compound 83 (white solid, 42 mg, 55.5% yield): ¹H NMR (300 MHz, DMSO-*d₆*) δ 12.82 (s, 1H), 7.90 (d, *J* = 8.6 Hz, 2H), 7.75 (d, *J* = 8.4 Hz, 2H), 7.06 (s, 2H), 4.50 (s, 2H), 2.17 (s, 6H), 1.36 (s, 6H), 1.35 (s, 6H). HRMS (ESI) calcd. for C₂₅H₂₇F₃N₂O₅ [M+NH₄]⁺ 510.2216, found 510.2213.

### Example 84

### Ethyl 2-(4-((4,4-dimethyl-2, 5-dioxo-3 -(-(4-(trifluoromethoxy)phenyl)imidazolidin-1-yl)methyl)-2,6-dimethylphenoxy)-2-methylpropionate (compound 84)

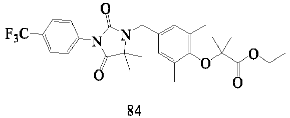

Referring to the method in Example 83, compound 84 (colorless liquid, 80 mg, 76.2% yield) was prepared without hydrolysis: ¹H NMR (300 MHz, CDCl₃) δ 7.75 (d, *J* = 9.0 Hz, 2H), 7.71 (d, *J* = 9.0 Hz, 2H), 6.99 (s, 2H), 4.54 (s, 2H), 4.31 (q, *J* = 7.1 Hz, 2H), 2.22 (s, 6H), 1.48 (s, 6H), 1.39 (s, 6H), 1.38 (t, *J* = 7.1 Hz, 3H). MS (ESI): *m*/*z* 543.2[M+Na]⁺.

### Example 85

### 2-(4-(2-(2, 5-Dioxo-3-(4-(trifluoromethyl)phenyl)imidazolidin-1-yl)ethyl)-2,6-dimethylphenoxy)-2-methylpropionic acid (compound 85)

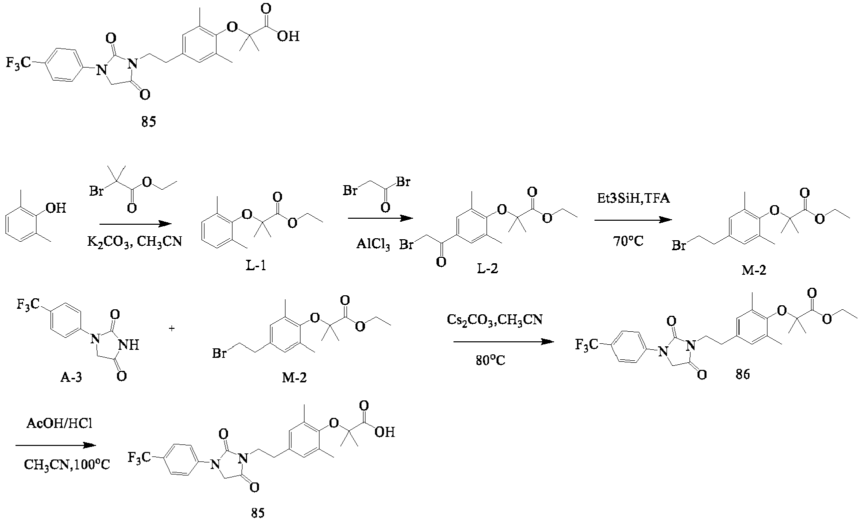

### Synthesis of intermediate L-1

2,6-Dimethylphenol (1.2 g, 10 mmol) was dissolved in acetonitrile (15 mL), and ethyl 2-bromoisobutyrate (3.4 mL, 30 mmol) and cesium carbonate (8.1 g, 25 mmol) were added. The reaction system was heated to 80 °C and stirred overnight. After the reaction was completed, the solvent was distilled off under reduced pressure, water (20 mL) was added, and the resulting solution was extracted with EA (50 mL × 3). The organic phase was washed with 1 N sodium hydroxide solution (20 mL × 3) and saturated sodium chloride solution (20 mL × 1), and dried over anhydrous Mg₂SO₄. The solvent was distilled off under reduced pressure, and the residue was intermediate L-1 (yellow liquid, 1.9 g, 80.5% yield).

### Synthesis of intermediate L-2

DCM (20 mL) and bromoacetyl bromide (2.1 mL, 24 mmol) were added to AlCl₃ (3.2 g, 24 mmol) in an ice bath under argon atmosphere. The mixture was stirred at room temperature for 1 h. Intermediate L-1 (1.9 g, 8 mmol) was added to the reaction solution described above in an ice bath, and the resulting solution was stirred at room temperature for another 12 h. After the reaction was completed, the solvent was distilled off under reduced pressure, water (20 mL) was added, and the resulting solution was extracted with EA (20 mL × 3). The organic phase was washed with saturated sodium chloride solution (20 mL × 1). The solvent was distilled off under reduced pressure, and the residue was intermediate L-2 (brown-black liquid, 1.8 g, 66.7% yield).

### Synthesis of intermediate M-2

Intermediate L-2 (1.8 g, 5 mmol) was dissolved in trifluoroacetic acid (15 mL), and triethylsilane (1.0 mL, 7.5 mmol) was added. The reaction system was heated to 70 °C and stirred for 12 h. After the reaction was completed, the mixture was diluted with water (20 mL) in an ice bath, and the resulting solution was stirred at room temperature for 10 min. The solvent was distilled off under reduced pressure, and the mixture was extracted with EA (20 mL × 3). The organic phase was washed with saturated sodium bicarbonate solution (20 mL) and saturated sodium chloride solution (20 mL × 1). The solvent was distilled off under reduced pressure, and the residue was intermediate M-2 (colorless liquid, 1.4 g, 82.3% yield).

### Synthesis of compound 86

Intermediate A-3 (110 mg, 0.45 mmol) was dissolved in acetonitrile (5 mL), and M-2 (184.7 mg, 0.54 mmol) and cesium carbonate (293.4 mg, 0.9 mmol) were added. The reaction system was heated to 80 °C and stirred for 12 h. After the reaction was completed, the solvent was distilled off under reduced pressure, and the residue was purified by silica gel column chromatography (petroleum ether/ethyl acetate = 5:1) to give compound 86 (colorless liquid, 66.9 mg, 29.3% yield).

### Synthesis of compound 85

Compound 86 (66.9 mg, 0.13 mmol) was dissolved in acetonitrile (2 mL), and a mixed solution of concentrated hydrochloric acid (at a concentration of 12 M) and acetic acid (4 mL, 1:1) was added. The reaction system was heated to 100 °C and stirred for 4 h. After the reaction was completed, the solvent was distilled off under reduced pressure, water (10 mL) was added, and the resulting solution was extracted with EA (15 mL × 3). The organic phase was washed with saturated sodium chloride solution (10 mL × 1). The solvent was distilled off under reduced pressure, and the residue was purified by silica gel column chromatography (dichloromethane/methanol = 100:1) to give compound 85 (white solid, 32 mg, 51.6% yield): ¹H NMR (300 MHz, DMSO-*d₆*) δ 12.76 (s, 1H), 7.83 (d, *J* = 8.7 Hz, 2H), 7.76 (d, *J* = 8.7 Hz, 2H), 6.86 (s, 2H), 4.53 (s, 2H), 3.64 (t, *J* = 7.4 Hz, 2H), 2.76 (t, *J* = 7.5 Hz, 2H), 2.13 (s, 6H), 1.31 (s, 6H). HRMS (ESI) calcd. for C₂₄H₂₅F₃N₂O₅ [M+Na]⁺: 501.1613, found: 501.1607.

### Example 86

### Ethyl 2-(4-(2-(2,5-dioxo-3-(4-(trifluoromethyl)phenyl)imidazolidin-1-yl)ethyl)-2,6-dimethylphenoxy)-2-methylpropionate (compound 86)

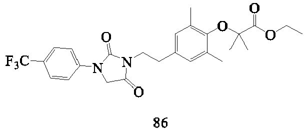

Referring to the method in Example 85, compound 86 (colorless liquid, 66.9 mg, 29.3% yield) was prepared without hydrolysis: ¹H NMR (300 MHz, CDCl₃) δ 7.71 (d, *J* = 9.1 Hz, 2H), 7.67 (d, *J* = 9.3 Hz, 2H), 6.87 (s, 2H), 4.33 (q, *J* = 7.1 Hz, 2H), 4.28 (s, 2H), 3.82 (t, *J* = 8.3 Hz, 2H), 2.88 (t, *J* = 8.3 Hz, 2H), 2.18 (s, 6H), 1.45 (s, 6H), 1.36 (t, *J* = 7.1 Hz, 3H). MS (ESI): *m*/*z* 529.2[M+Na]⁺.

### Example 87

### 2-(4-(2-(2,4-Dioxo-3-(4-(trifluoromethyl)phenyl)imidazolin-1-yl)ethyl)-2,6-dimethylphenoxy)-2-methylpropionic acid (compound 87)

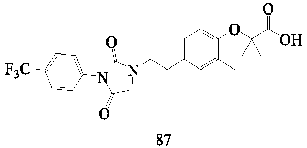

Referring to the method in Example 85, A-3 was replaced by B-3 to give compound 87 (white solid, 23 mg, 61.5% yield): ¹H NMR (300 MHz, DMSO-*d₆*) δ 12.69 (s, 1H), 7.87 (d, *J* = 8.4 Hz, 2H), 7.61 (d, *J* = 8.2 Hz, 2H), 6.91 (s, 2H), 4.10 (s, 2H), 3.56 (t, *J* = 7.3 Hz, 2H), 2.77 (t, *J* = 7.3 Hz, 2H), 2.14 (s, 6H), 1.32 (s, 6H). HRMS (ESI) calcd. for C₂₄H₂₅F₃N₂O₅ [M+Na]⁺: 501.1613, found: 501.1608.

### Example 88

### Ethyl 2-(4-(2-(2,4-dioxo-3-(4-(trifluoromethyl)phenyl)imidazolin-1-yl)ethyl)-2,6-dimethylphenoxy)-2-methylpropionate (compound 88)

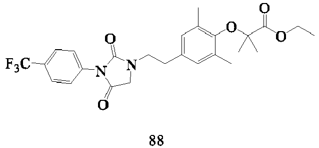

Referring to the method in Example 85, A-3 was replaced by B-3 to give compound 88 (colorless liquid, 39.3 mg, 26% yield) without hydrolysis: ¹H NMR (300 MHz, CDCl₃) δ 7.74 (d, *J* = 8.4 Hz, 2H), 7.61 (d, *J* = 8.5 Hz, 2H), 6.86 (s, 2H), 4.31 (q, *J* = 7.1 Hz, 2H), 3.85 (s, 2H), 3.71 (t, *J* = 7.2 Hz, 2H), 2.86 (t, *J* = 7.1 Hz, 2H), 2.20 (s, 6H), 1.47 (s, 6H), 1.38 (t, *J* = 7.1 Hz, 3H). MS (ESI): *m*/*z* 529.2[M+Na]⁺.

### Example 89

### 2-(4-(3-(2, 5-Dioxo-3-(4-(trifluoromethyl)phenyl)imidazolidin-1-yl)propyl)-2,6-dimethylphenoxy)-2-methylpropionic acid (compound 89)

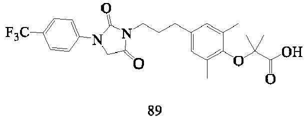

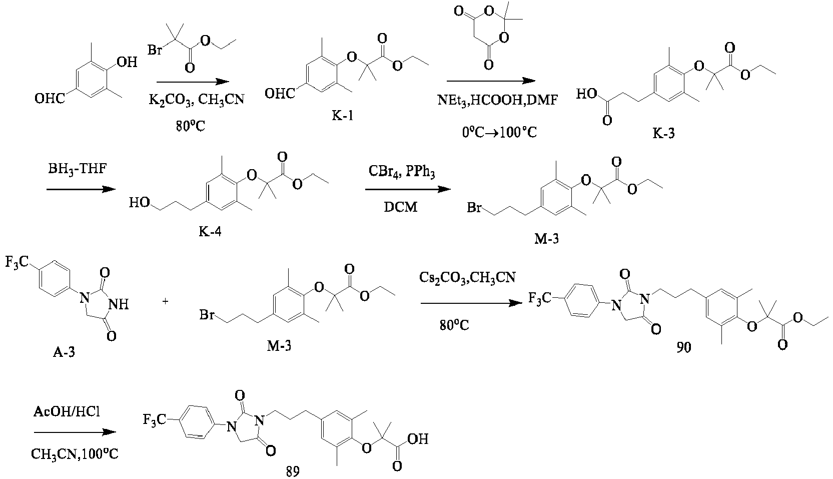

### Synthesis of intermediate K-3

Intermediate K-1 (0.5 g, 2.25 mmol) was dissolved in DMF (5 mL), and 2,2-dimethyl-1,3-dioxane-4,6-dione (0.5 g, 3.38 mmol) and triethylamine (0.4 mL, 2.7 mmol) were added. Formic acid (1.2 mL) was added in an ice bath, and the resulting solution was stirred at room temperature for 5 min. The reaction system was heated to 100 °C and stirred for 12 h. After the reaction was completed, water (20 mL) was added, and the resulting solution was extracted with EA (50 mL × 3). The organic phase was washed with saturated sodium chloride solution (20 mL × 1) and dried over anhydrous MgSO₄. The solvent was distilled off under reduced pressure, and the residue was intermediate K-3 (colorless liquid, 0.5 g, 71.8% yield).

### Synthesis of intermediate K-4

Intermediate K-3 (0.5 g, 1.5 mmol) was dissolved in tetrahydrofuran (5 mL), and boranetetrahydrofuran complex (1 mL, 1 mmol) was added in an ice bath. The resulting solution was stirred at room temperature for 12 h. After the reaction was completed, water (10 mL) was added, and the mixture was stirred for 30 min, and extracted with EA (20 mL × 3). The organic phase was washed with saturated sodium chloride solution (20 mL × 1). The solvent was distilled off under reduced pressure, and the residue was intermediate K-4 (colorless liquid, 0.2 g, 45.4% yield).

### Synthesis of intermediate M-3

Intermediate K-4 (0.2 g, 1 mmol) was dissolved in dichloromethane (5 mL), and tetrabromomethane (0.5 g, 1.5 mmol) and triphenylphosphine (0.5 g, 1.4 mmol) were added in an ice bath. The resulting solution was stirred at room temperature for 12 h. After the reaction was completed, the solvent was distilled off under reduced pressure, and the residue was purified by silica gel column chromatography (petroleum ether/ethyl acetate = 20:1) to give intermediate M-3 (colorless liquid, 0.25 g, 70.2% yield).

### Synthesis of compound 90

Intermediate A-3 (123 mg, 0.5 mmol) was dissolved in acetonitrile (5 mL), and M-3 (213.6 mg, 0.6 mmol) and cesium carbonate (326 mg, 1 mmol) were added. The reaction system was heated to 80 °C and stirred for 12 h. After the reaction was completed, the solvent was distilled off under reduced pressure, and the residue was purified by silica gel column chromatography (petroleum ether/ethyl acetate = 5:1) to give compound 90 (colorless liquid, 58.9 mg, 22.6% yield).

### Synthesis of compound 89

Compound 90 (58.9 mg, 0.12 mmol) was dissolved in acetonitrile (2 mL), and a mixed solution of concentrated hydrochloric acid (at a concentration of 12 M) and acetic acid (2 mL, 1:1) was added. The reaction system was heated to 100 °C and stirred for 4 h. After the reaction was completed, the solvent was distilled off under reduced pressure, water (10 mL) was added, and the resulting solution was extracted with EA (15 mL × 3). The organic phase was washed with saturated sodium chloride solution (10 mL × 1). The solvent was distilled off under reduced pressure, and the residue was purified by silica gel column chromatography (dichloromethane/methanol = 100:1) to give compound 89 (white solid, 43 mg, 72.8% yield): ¹H NMR (300 MHz, DMSO-*d₆*) δ 12.78 (s, 1H), 7.85 (d, *J* = 8.7 Hz, 2H), 7.76 (d, *J* = 8.8 Hz, 2H), 6.84 (s, 2H), 4.48 (s, 2H), 3.50 (t, *J* = 6.9 Hz, 2H), 2.55 - 2.51 (m, 2H), 2.12 (s, 6H), 1.96 - 1.79 (m, 2H), 1.32 (s, 6H). HRMS (ESI) calcd. for C₂₅H₂₇F₃N₂O₅ [M+Na]⁺: 515.1770, found: 515.1754.

### Example 90

### Ethyl 2-(4-(3-(2,5-dioxo-3-(4-(trifluoromethyl)phenyl)imidazolidin-1-yl)propyl)-2,6-dimethylphenoxy)-2-methylpropionate (compound 90)

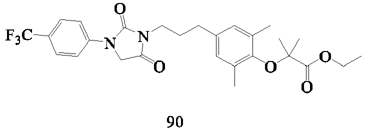

Referring to the method in Example 89, compound 90 (colorless liquid, 58.9 mg, 22.6% yield) was prepared without hydrolysis: ¹H NMR (300 MHz, CDCl₃) δ 7.70 (d, *J* = 9.2 Hz, 2H), 7.66 (d, *J* = 6.3 Hz, 2H), 6.81 (s, 2H), 4.28 (q, *J* = 7.1 Hz, 2H), 4.19 (s, 2H), 3.68 (t, *J* = 7.6 Hz, 2H), 2.60 (t, *J* = 7.6 Hz, 2H), 2.13 (s, 6H), 2.09 - 1.94 (m, 2H), 1.44 (s, 6H), 1.35 (t, *J* = 7.1 Hz, 3H). MS (ESI): *m*/*z* 543.2[M+Na]⁺.

### Example 91

### 2-(4-(3-(2,4-Dioxo-3-(4-(trifluoromethyl)phenyl)imidazolidin-1-yl)propyl)-2,6-dimethylphenoxy)-2-methylpropionic acid (compound 91)

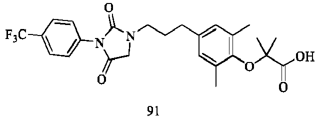

Referring to the method in Example 89, A-3 was replaced by B-3 to give compound 91 (white solid, 28 mg, 75% yield): ¹H NMR (300 MHz, DMSO-*d₆*) δ 12.60 (s, 1H), 7.87 (d, *J* = 8.4 Hz, 2H), 7.65 (d, *J* = 8.3 Hz, 2H), 6.87 (s, 2H), 4.13 (s, 2H), 3.40 (t, *J* = 7.0 Hz, 2H), 2.58 - 2.53 (m, 2H), 2.13 (s, 6H), 1.91 - 1.76 (m, 2H), 1.33 (s, 6H). HRMS (ESI) calcd. for C₂₅H₂₇F₃N₂O₅ [M+Na]⁺: 515.1770, found: 515.1764.

### Example 92

### Ethyl 2-(4-(3-(2,4-dioxo-3-(4-(trifluoromethyl)phenyl)imidazolidin-1-yl)propyl)-2,6-dimethylphenoxy)-2-methylpropionate (compound 92)

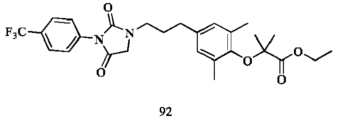

Referring to the method in Example 89, A-3 was replaced by B-3 to give compound 92 (yellow liquid, 39 mg, 21% yield) without hydrolysis: ¹H NMR (300 MHz, CDCl₃) δ 7.74 (d, *J* = 8.4 Hz, 2H), 7.63 (d, *J* = 8.4 Hz, 2H), 6.81 (s, 2H), 4.30 (q, *J* = 7.1 Hz, 2H), 4.00 (s, 2H), 3.54 (t, *J* = 7.2 Hz, 2H), 2.60 (t, *J* = 7.5 Hz, 2H), 2.18 (s, 6H), 2.01 - 1.87 (m, 2H), 1.47 (s, 6H), 1.37 (t, *J* = 7.1 Hz, 3H). MS (ESI): *m*/*z* 543.2[M+Na]⁺.

### Example 93

### 2-(4-((2, 5-Dioxo-3-(4-(trifluoromethyl)phenyl)imidazolidin-1-yl)methyl)-2,6-dimethylphenoxy)-2-methylpropionic acid (compound 93)

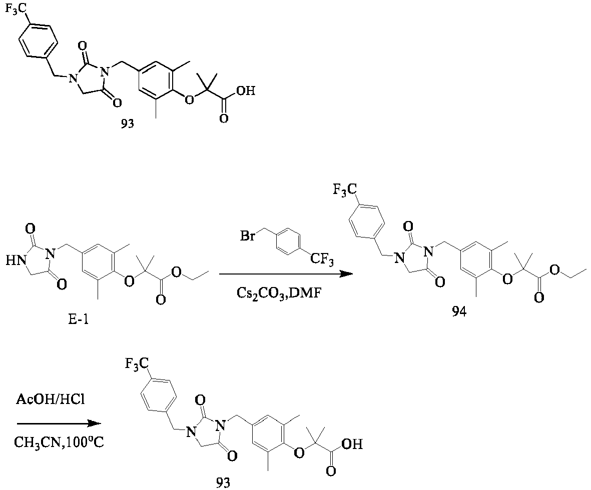

### Synthesis of compound 94

Intermediate E-1 (190 mg, 0.54 mmol) was dissolved in DMF (5 mL), and p-trifluoromethylbenzyl bromide (155 mg, 0.65 mmol) and cesium carbonate (352 mg, 1.08 mmol) were added. The reaction system was stirred at room temperature for 12 h. After the reaction was completed, the solvent was distilled off under reduced pressure, and the residue was purified by silica gel column chromatography (petroleum ether/ethyl acetate = 5:1) to give compound 94 (colorless liquid, 110 mg, 40.8% yield).

### Synthesis of compound 93

Compound 94 (110 mg, 0.22 mmol) was dissolved in acetonitrile (2 mL), and a mixed solution of concentrated hydrochloric acid (at a concentration of 12 M) and acetic acid (2 mL, 1:1) was added. The reaction system was heated to 100 °C and stirred for 4 h. After the reaction was completed, the solvent was distilled off under reduced pressure, water (10 mL) was added, and the resulting solution was extracted with EA (15 mL × 3). The organic phase was washed with saturated sodium chloride solution (10 mL × 1). The solvent was distilled off under reduced pressure, and the residue was purified by silica gel column chromatography (dichloromethane/methanol = 100:1) to give compound 93 (white solid, 67 mg, 63.8% yield): ¹H NMR (300 MHz, DMSO-*d₆*) δ 12.82 (s, 1H), 7.72 (d, *J* = 8.0 Hz, 2H), 7.53 (d, *J* = 8.1 Hz, 2H), 6.92 (s, 2H), 4.62 (s, 2H), 4.46 (s, 2H), 4.03 (s, 2H), 2.15 (s, 6H), 1.35 (s, 6H). HRMS (ESI) calcd. for C₂₄H₂₅F₃N₂O₅ [M+Na]⁺: 501.1613, found: 501.1606.

### Example 94

### Ethyl 2-(4-((2,5-dioxo-3-(4-(trifluoromethyl)phenyl)imidazolidin-1-yl)methyl)-2,6-dimethylphenoxy)-2-methylpropionate (compound 94)

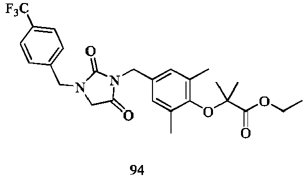

Referring to the method in Example 93, compound 94 (colorless liquid, 110 mg, 40.8% yield) was prepared without hydrolysis: ¹H NMR (300 MHz, CDCl₃) δ 7.64 (d, *J* = 8.0 Hz, 2H), 7.39 (d, *J* = 8.1 Hz, 2H), 7.03 (s, 2H), 4.64 (s, 2H), 4.58 (s, 2H), 4.30 (q, *J* = 7.1 Hz, 2H), 3.78 (s, 2H), 2.19 (s, 6H), 1.47 (s, 6H), 1.36 (t, *J* = 7.1 Hz, 3H). MS (ESI): *m*/*z* 529.2[M+Na]⁺.

### Example 95

### 2-(4-(3-(4-Ethoxyphenyl)-2, 5-dioxoimidazolidin-1-yl)methyl)-2, 6-dimethylphenoxy)-2-methylpropionic acid (compound 95)

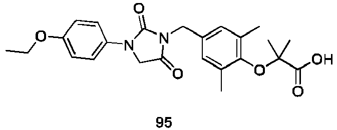

Referring to the method in Example 1, 4-iodotrifluoromethylbenzene in the synthetic route of method (3) was replaced byp-iodophenetole to give compound 95 (white solid, 60.9 mg, 34.6% yield): ¹H NMR (300 MHz, DMSO-*d₆*) δ 12.83 (s, 1H), 7.51 (d, J = 9.0 Hz, 2H), 6.95 (s, 2H), 6.93 (d, 2H), 4.52 (s, 2H), 4.49 (s, 2H), 3.99 (q, *J* = 6.9 Hz, 2H), 2.13 (s, 6H), 1.33 (s, 6H), 1.29 (q, *J* = 7.0 Hz, 3H). MS (ESI): *m*/*z* 463.2 [M+Na]⁺.

### Example 96

### Ethyl 2-(4-(3-(4-ethoxyphenyl)-2, 5-dioxoimidazolidin-1-yl)methyl)-2, 6-dimethylphenoxy)-2-methylpropionate (compound 96)

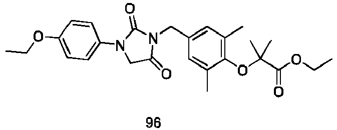

Referring to the method in Example 1, 4-iodotrifluoromethylbenzene in the synthetic route of method (3) was replaced by *p*-iodophenetole to give compound 96 (white solid, 195.6 mg, 41.8% yield) without hydrolysis: ¹H NMR (300 MHz, CDCl₃) δ 7.45 (d, *J* = 8.9 Hz, 2H), 7.06 (s, 2H), 6.91 (d, *J* = 9.0 Hz, 2H), 4.61 (s, 2H), 4.28 (s, 2H), 4.28 (q, *J* = 7.1 Hz, 2H), 4.02 (q, *J* = 6.8 Hz, 2H), 2.18 (s, 6H), 1.45 (s,6H), 1.40 (t, *J* = 7.0 Hz, 3H), 1.34 (t, *J* = 7.1 Hz, 3H). MS (ESI): *m*/*z* 491.2 [M+Na]⁺.

### Example 97

### 2-(4-(2,5-Dioxo-3-(4-trifluoromethylphenyl)imidazolidin-1-yl)methyl)-2-methoxyphenoxy)-2-methylpropionic acid (compound 97)

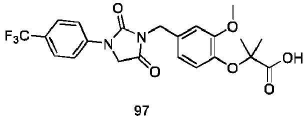

Referring to the method in Example 1, 3,5-dimethyl-p-hydroxybenzaldehyde was replaced by 3-methoxy-*p*-hydroxybenzaldehyde to give compound 97 (white solid, 85.9 mg, 59.7% yield): ¹H NMR (300 MHz, DMSO-*d₆*) δ 12.90 (s, 1H), 7.84 (d, *J* = 8.6 Hz, 2H), 7.75 (d, *J* = 8.8 Hz, 2H), 6.97 (s, 1H), 6.80 (d, *J* = 8.3 Hz, 1H), 6.75 (d, *J* = 8.2 Hz, 1H), 4.61 (s, 2H), 4.58 (s, 2H), 3.72 (s, 3H), 1.43 (s, 6H). MS (ESI): *m*/*z* 489.1 [M+Na]⁺.

### Example 98

### Ethyl 2-(4-(2,5-dioxo-3-(4-trifluoromethylphenyl)imidazolidin-1-yl)methyl)-2-methoxyphenoxy)-2-methylpropionate (compound 98)

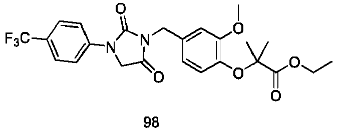

Referring to the method in Example 1, 3,5-dimethyl-*p*-hydroxybenzaldehyde was replaced by 3-methoxy-*p*-hydroxybenzaldehyde to give compound 98 (white solid, 158.5 mg, 35.3% yield) without hydrolysis: ¹H NMR (300 MHz, CDCl₃) δ 7.72 (d, *J* = 8.9 Hz, 2H), 7.66 (d, *J* = 8.9 Hz, 2H), 7.04 (d, *J* = 1.7 Hz, 1H), 6.95 (dd, *J* = 8.2, 1.8 Hz, 1H), 6.82 (d, *J* = 8.2 Hz, 1H), 4.70 (s, 2H), 4.35 (s, 2H), 4.25 (q, *J* = 7.1 Hz, 2H), 3.84 (s, 3H), 1.57 (s, 6H), 1.29 (t, *J* = 7.1 Hz, 3H). MS (ESI): *m*/*z* 517.2[M+Na]⁺.

### Example 99

### 2-(4-(3-(4-Formylphenyl)-2,5-dioxoimidazolidin-1-yl)methyl)-2,6-dimethylphenoxy)-2-methylpropionic acid (compound 99)

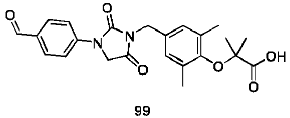

Referring to the method in Example 1, 4-iodotrifluoromethylbenzene in the synthetic route of method (3) was replaced by 4-bromobenzaldehyde to give compound 99 (white solid, 15.3 mg, 12.1% yield): ¹H NMR (300 MHz, DMSO-*d₆*) δ 12.83 (s, 1H), 9.92 (s, 1H), 7.94 (d, *J* = 8.8 Hz, 2H), 7.86 (d, *J* = 8.7 Hz, 2H), 6.97 (s, 2H), 4.63 (s, 2H), 4.53 (s, 2H), 2.13 (s, 6H), 1.33 (s, 6H). MS (ESI): *m*/*z* 425.1 [M+Na]⁺.

### Example 100

### Ethyl 2-(4-(3-(4-formylphenyl)-2, 5-dioxoimidazolidin-1-yl)methyl)-2, 6-dimethylphenoxy)-2-methylpropionate (compound 100)

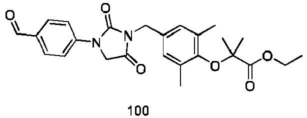

Referring to the method in Example 1, 4-iodotrifluoromethylbenzene in the synthetic route of method (3) was replaced by 4-bromobenzaldehyde to give compound 100 (white solid, 135.8 mg, 30.0% yield) without hydrolysis: ¹H NMR (300 MHz, CDCl₃) δ 9.97 (s, 1H), 7.92 (s, 2H), 7.79 (d, *J* = 8.7 Hz, 2H), 7.08 (s, 2H), 4.66 (s, 2H), 4.39 (s, 2H), 4.30 (q, *J* = 7.0 Hz, 2H), 2.20 (s, 6H), 1.47 (s, 6H), 1.36 (t, *J* = 7.1 Hz, 3H). MS (ESI): *m*/*z* 475.2[M+Na]⁺.

### Example 101

### 2-(3-(4-(Cyclopropylphenyl)-2,5-dioxoimidazolidin-1-yl)methyl)-2,6-dimethylphenoxy)-2-methylpropionic acid (compound 101)

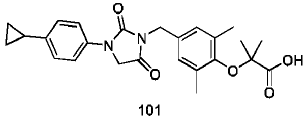

Referring to the method in Example 1, 4-iodotrifluoromethylbenzene in the synthetic route of method (3) was replaced by 4-bromocyclopropylbenzene to give compound 101 (white solid, 18.5 mg, 27.6% yield): ¹H NMR (300 MHz, DMSO-*d₆*) δ 12.83 (s, 1H), 7.56 (d, *J* = 8.6 Hz, 1H), 7.49 (d, *J* = 8.4 Hz, 1H), 7.39 (d, *J* = 8.6 Hz, 1H), 7.08 (d, *J* = 8.6 Hz, 1H), 6.95 (s, 2H), 4.53 (s, 2H), 4.50 (s, 2H), 2.13 (s, 6H), 1.87 - 1.78 (m, 1H), 1.33 (s, 6H), 1.00 - 0.86 (m, 2H), 0.66 - 0.56 (m, 2H). MS (ESI): *m*/*z* 437.2 [M+Na]⁺.

### Example 102

### Ethyl 2-(4-(3-(4-cyclopropylphenyl)-2, 5-dioxoimidazolidin-1-yl)methyl)-2,6-dimethylphenoxy)-2-methylpropionate (compound 102)

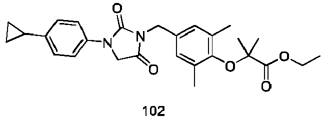

Referring to the method in Example 1, 4-iodotrifluoromethylbenzene in the synthetic route of method (3) was replaced by 4-bromocyclopropylbenzene to give compound 102 (white solid, 74.0 mg, 14.6% yield) without hydrolysis:¹H NMR (300 MHz, CDCl₃) δ 7.46 (d, *J* = 8.6 Hz, 2H), 7.12 (s, 2H), 7.08 (d, *J* = 4.1 Hz, 2H), 4.63 (s, 2H), 4.31 (s, 2H), 4.27 (q, *J* = 7.1 Hz, 2H), 2.29 - 2.21 (m, 1H), 2.19 (s, 6H), 1.47 (s, 6H), 1.37 (d, *J* = 7.2 Hz, 3H), 0.97 (dt, *J* = 13.7, 5.6 Hz, 2H), 0.68 (dt, *J* = 9.6, 4.7 Hz, 2H). MS (ESI): *m*/*z* 487.2[M+Na]⁺.

### Example 103

### 2-(4-(3-Fluorophenyl)-2, 5-dioxoimidazolidin-1-yl)methyl)-2, 6-dimethylphenoxy-2-methylpropionic acid (compound 103)

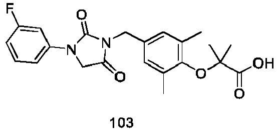

Referring to the method in Example 1, 4-iodotrifluoromethylbenzene in the synthetic route of method (3) was replaced by 3-fluoroiodobenzene to give compound 103 (white solid, 9.0 mg, 20.6% yield): ¹H NMR (300 MHz, DMSO-*d₆*) δ 12.82 (s, 1H), 7.57 (d, *J* = 11.1 Hz, 1H), 7.43 (s, 2H), 6.99 - 6.94 (m, 3H), 4.56 (s, 2H), 4.51 (s, 2H), 2.13 (s, 6H), 1.33 (s, 6H). MS (ESI): *m*/*z* 437.1 [M+Na]⁺.

### Example 104

### Ethyl 2-(4-(3-(3-fluorophenyl)-2,5-dioxoimidazolidin-1-yl)methyl)-2,6-dimethylphenoxy)-2-methylpropionate (compound 104)

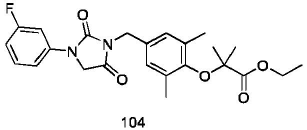

Referring to the method in Example 1, 4-iodotrifluoromethylbenzene in the synthetic route of method (3) was replaced by 3-fluoroiodobenzene to give compound 104 (white solid, 49.5 mg, 10.7% yield) without hydrolysis: ¹H NMR (300 MHz, CDCl₃) δ 7.54 (dt, *J* = 11.1, 2.1 Hz, 1H), 7.36 (dd, *J* = 14.8, 8.2 Hz, 1H), 7.24 (dd, *J* = 7.9, 1.1 Hz, 1H), 7.07 (s, 2H), 6.87 (td, *J* = 8.2, 2.2 Hz, 1H), 4.64 (s, 2H), 4.32 (s, 2H), 4.27 (q, *J* = 7.1 Hz, 2H), 2.20 (s, 6H), 1.47 (s, 6H), 1.36 (t, *J* = 7.1 Hz, 3H). MS (ESI): *m*/*z* 465.2[M+Na]⁺.

### Example 105

### 2-(3-Chloro-4-fluorophenyl)-2,5-dioxoimidazolidin-1-yl)methyl-2,6-dimethylphenoxy-2-methylpropionic acid (compound 105)

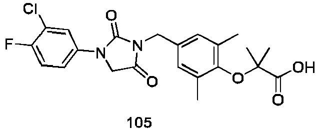

Referring to the method in Example 1, 4-iodotrifluoromethylbenzene in the synthetic route of method (3) was replaced by 2-chloro-1-fluoro-4-iodobenzene to give compound 105 (white solid, 25.4 mg, 20.5% yield): ¹H NMR (300 MHz, DMSO-*d₆*) δ 12.82 (s, 1H), 7.89 (d, *J* = 6.3 Hz, 1H), 7.61 (d, *J* = 9.0 Hz, 1H), 7.46 (t, *J* = 9.2 Hz, 1H), 6.95 (s, 2H), 4.56 (s, 2H), 4.50 (s, 2H), 2.13 (s, 6H), 1.32 (s, 6H). MS (ESI): *m*/*z* 471.1 [M+Na]⁺.

### Example 106

### Ethyl 2-(4-(3-chloro-4-fluorophenyl)-2,5-dioxoimidazolin-1-yl)methyl)-2,6-dimethylphenoxy-2-methylpropionate (compound 106)

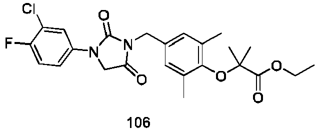

Referring to the method in Example 1, 4-iodotrifluoromethylbenzene in the synthetic route of method (3) was replaced by 2-chloro-1-fluoro-4-iodobenzene to give compound 106 (white solid, 134.6 mg, 30.8% yield) without hydrolysis: ¹H NMR (300 MHz, CDCl₃) δ 7.77 (d, *J* = 3.4 Hz, 1H), 7.39 (s, 1H), 7.21 - 7.13 (m, 1H), 7.07 (s, 2H), 4.63 (s, 2H), 4.32 - 4.24 (m, 4H), 2.20 (s, 6H), 1.47 (s, 6H), 1.36 (t, *J* = 7.1 Hz, 3H). MS (ESI): *m*/*z* 499.1[M+Na]⁺.

### Example 107

### 2-(4-((3-(3-Chlorophenyl)-2,5-dioxoimidazolidin-1-yl)methyl)-2,6-dimethylphenoxy-2-methylpropionic acid (compound 107)

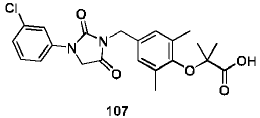

Referring to the method in Example 1, 4-iodotrifluoromethylbenzene in the synthetic route of method (3) was replaced by 1-chloro-3-iodobenzene to give compound 107 (white solid, 10 mg, 16.5% yield): ¹H NMR (300 MHz, DMSO-*d₆*) δ 12.84 (s, 1H), 7.79 (s, 1H), 7.56 (d, *J* = 7.8 Hz, 1H), 7.41 (t, *J* = 8.1 Hz, 1H), 7.18 (d, *J* = 7.9 Hz, 1H), 6.96 (s, 2H), 4.57 (s, 2H), 4.51 (s, 2H), 2.13 (s, 6H), 1.33 (s, 6H). MS (ESI): *m*/*z* 453.2 [M+Na]⁺.

### Example 108

### Ethyl 2-(4-(3-chlorophenyl)-2,5-dioxoimidazolidin-1-yl)methyl)-2,6-dimethylphenoxy)-2-methylpropionate (compound 108)

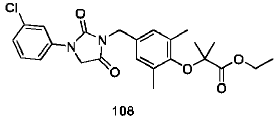

Referring to the method in Example 1, 4-iodotrifluoromethylbenzene in the synthetic route of method (3) was replaced by 1-chloro-3-iodobenzene to give compound 108 (yellow liquid, 60.7 mg, 12.7% yield) without hydrolysis: ¹H NMR (300 MHz, CDCl₃) δ 7.70 (s, 1H), 7.42 (d, *J* = 7.5 Hz, 1H), 7.32 (t, *J* = 7.9 Hz, 1H), 7.13 (d, *J* = 6.9 Hz, 1H), 7.06 (s, 2H), 4.63 (s, 2H), 4.30 (s, 2H), 4.26 (q, *J* = 7.0 Hz, 2H), 2.19 (s, 6H), 1.45 (s,6H), 1.35 (t, *J* = 7.0 Hz, 3H). MS (ESI): *m*/*z* 481.1[M+Na]⁺.

### Example 109

### 2-(4-(2,5-Dioxo-3-(3-trifluoromethoxyphenyl)imidazolidin-1-yl)methyl)-2,6-dimethylphenoxy)-2-methylpropionic acid (compound 109)

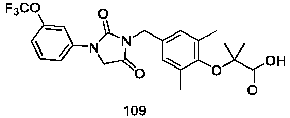

Referring to the method in Example 1, 4-iodotrifluoromethylbenzene in the synthetic route of method (3) was replaced by 3-trifluoromethoxybromobenzene to give compound 109 (white solid, 47.4 mg, 36.7% yield):¹H NMR (300 MHz, DMSO-*d₆*) δ 12.90 (s, 1H), 7.82 (s, 1H), 7.62 - 7.45 (m, 2H), 7.25 - 7.07 (m, 1H),, 6.95 (s, 2H), 4.58 (s, 2H), 4.51 (s, 2H), 2.13 (s, 6H), 1.32 (s, 6H). MS (ESI): *m*/*z* 503.2 [M+Na]⁺.

### Example 110

### Ethyl 2-(4-(3-chlorophenyl)-2,5-dioxoimidazolidin-1-yl)methyl)-2,6-dimethylphenoxy)-2-methylpropionate (compound 110)

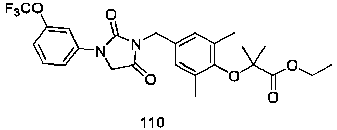

Referring to the method in Example 1, 4-iodotrifluoromethylbenzene in the synthetic route of method (3) was replaced by 3-trifluoromethoxybenzene to give compound 110 (yellow liquid, 176.1 mg, 38.4% yield) without hydrolysis: ¹H NMR (300 MHz, CDCl₃) δ 7.66 (s, 1H), 7.43 (d, *J* = 4.9 Hz, 2H), 7.08 (s, 2H), 7.03 (s, 1H), 4.65 (s, 2H), 4.33 (s, 2H), 4.29 (q, *J=* 7.1 Hz, 2H), 2.20 (s, 6H), 1.47 (s, 6H), 1.36 (t, *J=* 7.1 Hz, 3H). MS (ESI): *m*/*z* 531.2[M+Na]⁺.

### Example 111

### Tromethamine 2-(4-(2,5-Dioxo-3-(4-trifluoromethylphenyl)imidazolidin-1-yl)methyl)-2,6-dimethylphenoxy)-2-methylpropionate (compound 111)

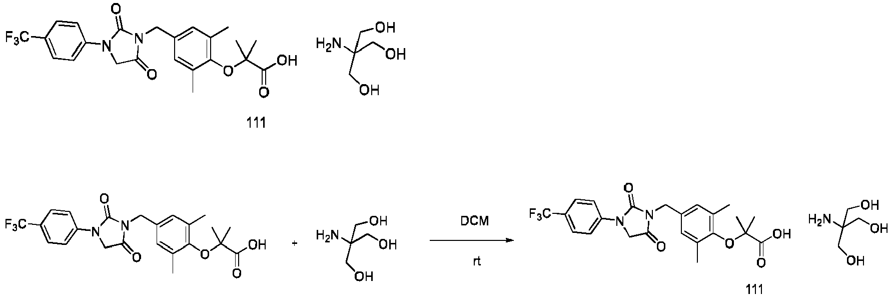

Compound 1 (232 mg, 0.5 mmol) was dissolved in DCM (4 mL), and tromethamine (60.5 mg, 0.5 mmol) was added. The mixture was stirred at room temperature for 12 h, and then a white solid was precipitated from the reaction solution. The mixture was filtered under vacuum, and acetone (0.5 mL) and n-hexane (2 mL) were added to the solid, followed by stirring at room temperature for 2 h. The mixture was filtered under vacuum to give compound 111 (white solid, 277.0 mg, 94.7% yield): ¹H NMR (300 MHz, DMSO-*d₆*) δ 7.86 (d, *J* = 8.4 Hz, 2H), 7.76 (d, *J* = 8.8 Hz, 2H), 6.94 (s, 2H), 6.03 (s, 3H), 4.62 (s, 2H), 4.52 (s, 2H), 3.38 (s, 6H), 2.16 (s, 6H), 1.27 (s, 6H).

### Example 112

### Esmolol 2-(4-(2,5-dioxo-3-(4-(trifluoromethyl)phenyl)imidazolidin-1-yl)methyl)-2,6-dimethylphenoxy)-2-methylpropionate (compound 112)

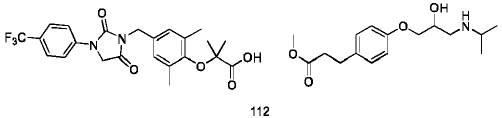

Referring to the method in Example 111, tromethamine in Example 111 was replaced by esmolol to give compound 112 (white solid, 27.7 mg, 47.1% yield): ¹H NMR (300 MHz, DMSO-*d₆*) δ 7.85 (d, *J* = 8.6 Hz, 2H), 7.75 (d, *J* = 8.7 Hz, 2H), 7.11 (d, *J* = 7.5 Hz, 2H), 6.94 (s, 2H), 6.83 (d, *J* = 7.7 Hz, 2H), 4.61 (s, 2H), 4.52 (s, 2H), 4.00 (s, 1H), 3.96 - 3.63 (m, 3H), 3.48 - 2.95 (m, 7H), 2.89 (d, *J* = 11.2 Hz, 1H), 2.75 (d, *J* = 8.0 Hz, 3H), 2.14 (s, 6H), 1.30 (s, 6H), 1.24 (s, 1H), 1.10 (d, *J* = 5.4 Hz, 6H).

### Example 113

### Cinacalcet 2-(4-(2,5-dioxo-3-(4-trifluoromethylphenyl)imidazolidin-1-yl)methyl)-2,6-dimethylphenoxy)-2-methylpropionate (compound 113)

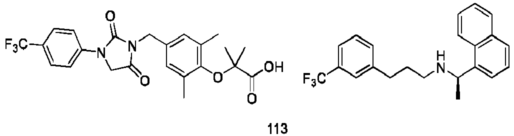

Referring to the method in Example 111, tromethamine in Example 111 was replaced by cinacalcet to give compound 113 (white solid, 49.0 mg, 55.8% yield): ¹H NMR (300 MHz, DMSO-*d₆*) δ 8.25 (s, 1H), 7.97 - 7.66 (m, 7H), 7.49 (s, 5H), 6.96 (s, 2H), 4.57 (d, *J=* 25.9 Hz, 5H), 3.56 - 3.09 (m, 7H), 2.69 (s, 2H), 2.43 - 2.33 (m, 2H), 2.14 (s, 6H), 1.75 (s, 1H), 1.53 - 1.17 (m, 9H).

### Example 114

### Trimetazidine 2-(4-(2,5-dioxo-3-(4-trifluoromethylphenyl)imidazolin-1-yl)methyl)-2,6-dimethylphenoxy)-2-methylpropionate (compound 114)

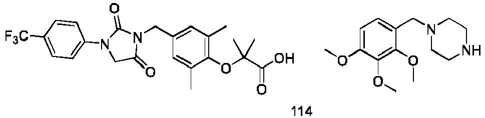

Referring to the method in Example 111, tromethamine in Example 111 was replaced by trimetazidine to give compound 114 (white solid, 55.8 mg, 76.4% yield): ¹H NMR (300 MHz, DMSO-*d₆*) δ 7.85 (d, *J* = 8.5 Hz, 2H), 7.75 (d, *J* = 8.8 Hz, 2H), 6.94 (s, 3H), 6.74 (d, *J* = 8.4 Hz, 1H), 4.61 (s, 2H), 4.52 (s, 2H), 3.76 (d, *J* = 1.6 Hz, 6H), 3.72 (s, 3H), 2.78 (s, 3H), 2.37 (s, 3H), 2.14 (s, 6H), 1.29 (s, 6H).

### Example 115

### Fasudil 2-(4-((2,5-dioxo-3-(4-(trifluoromethyl)phenyl)imidazolidinoxy-1-yl)methyl)-2,6-dimethylphenoxy)-2-methylpropionate (compound 115)

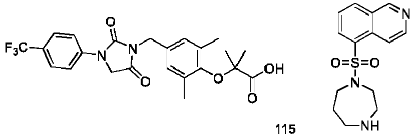

Referring to the method in Example 111, tromethamine in Example 111 was replaced by fasudil to give compound 115 (white solid, 51.8 mg, 63.3% yield): ¹H NMR (300 MHz, DMSO-*d₆*) δ 9.48 (s, 1H), 8.69 (d, *J* = 5.8 Hz, 1H), 8.44 (d, *J* = 7.8 Hz, 1H), 8.37 - 8.28 (m, 2H), 7.91 - 7.66 (m, 5H), 6.94 (s, 2H), 4.61 (s, 2H), 4.52 (s, 2H), 3.53 - 3.41 (m, 4H), 2.87 - 2.67 (m, 4H), 2.13 (s, 6H), 1.67 (d, 2H), 1.30 (s, 6H).

### Example 116

### Acebutolol 2-(2,5-dioxo-3-(4-(trifluoromethyl)phenyl)imidazolidin-1-yl)methyl)-2,6-dimethylphenoxy)-2-methylpropionate (compound 116)

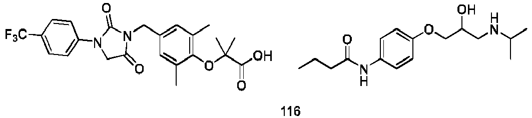

Referring to the method in Example 111, tromethamine in Example 111 was replaced by acebutolol to give compound 116 (white solid, 62.8 mg, 73.4% yield): ¹H NMR (300 MHz, DMSO-*d₆*) δ 9.86 (s, 1H), 7.91 - 7.65 (m, 7H), 7.09 (d, *J* = 8.9 Hz, 1H), 6.94 (s, 2H), 5.75 (s, 1H), 4.61 (s, 2H), 4.52 (s, 2H), 4.02 (d, *J* = 6.5 Hz, 2H), 2.96 - 2.80 (m, 2H), 2.73 (d, *J* = 7.1 Hz, 1H), 2.57 (s, 3H), 2.23 (t, *J* = 7.3 Hz, 2H), 2.14 (s, 6H), 1.58 (q, *J* = 14.7, 7.4 Hz, 2H), 1.29 (s, 6H), 1.05 (d, *J* = 6.2 Hz, 6H), 0.89 (t, *J* = 7.3 Hz, 3H).

### Example 117

### Bevantolol 2-(4-(2,5-dioxo-3-(4-(trifluoromethyl)phenyl)imidazolidin-1-yl)methyl)-2,6-dimethylphenoxy)-2-methylpropionate (compound 117)

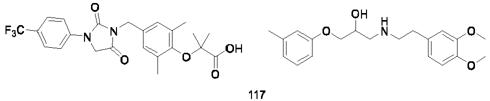

Referring to the method in Example 111, tromethamine in Example 111 was replaced by bevantolol to give compound 117 (white solid, 62.6 mg, 22.4% yield): ¹H NMR (300 MHz, DMSO-*d₆*) δ 7.85 (d, *J* = 8.6 Hz, 2H), 7.75 (d, *J* = 8.6 Hz, 2H), 7.14 (t, *J* = 7.8 Hz, 1H), 6.95 (s, 2H), 6.83 (d, *J* = 8.9 Hz, 2H), 6.71 (t, *J* = 6.6 Hz, 3H), 4.61 (s, 2H), 4.52 (s, 2H), 3.90 (d, *J* = 13.4 Hz, 3H), 3.72 (s, 2H), 3.70 (s, 3H), 3.04 - 2.74 (m, 6H), 2.26 (s, 3H), 2.14 (s, 6H), 1.32 (s, 6H).

### Example 118

### Metoprolol 2-(4-(2,5-dioxo-3-(4-(trifluoromethyl)phenyl)imidazolidin-1-yl)methyl)-2,6-dimethylphenoxy)-2-methylpropionate (compound 118)

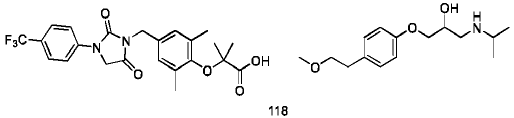

Referring to the method in Example 111, tromethamine in Example 111 was replaced by metoprolol to give compound 118 (white solid, 52.2 mg, 66.8% yield): ¹H NMR (300 MHz, DMSO-*d₆*) δ 7.85 (d, *J* = 8.7 Hz, 2H), 7.75 (d, *J* = 8.7 Hz, 2H), 7.12 (d, *J* = 8.4 Hz, 2H), 6.94 (s, 2H), 6.83 (d, *J* = 8.5 Hz, 2H), 4.61 (s, 2H), 4.52 (s, 2H), 3.96 (s, 1H), 3.89 (s, 2H), 3.46 (t, *J* = 6.9 Hz, 2H), 3.22 (s, 3H), 2.97 - 2.88 (m, 1H), 2.83 (d, *J* = 8.2 Hz, 1H), 2.74 - 2.63 (m, 3H), 2.15 (s, 6H), 1.29 (s, 6H), 1.07 (d, *J* = 6.0 Hz, 6H).

### Example 119

### Bisoprolol 2-(4-((2,5-dioxo-3-(4-(trifluoromethyl)phenyl))imidazolin-1-yl)methyl)-2,6-dimethylphenoxy)-2-methylpropionate (compound 119)

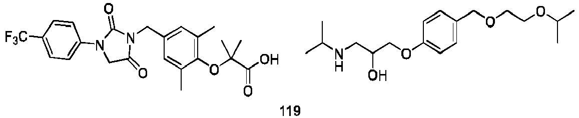

Referring to the method in Example 111, tromethamine in Example 111 was replaced by bisoprolol to give compound 119 (white solid, 72.4 mg, 85.8% yield): ¹H NMR (300 MHz, DMSO-*d₆*) δ 7.85 (d, *J* = 8.6 Hz, 2H), 7.75 (d, *J* = 8.6 Hz, 2H), 7.22 (d, *J* = 8.1 Hz, 2H), 6.94 (s, 2H), 6.90 (d, *J* = 8.4 Hz, 2H), 4.61 (s, 2H), 4.52 (s, 2H), 4.39 (s, 2H), 3.96 (s, 1H), 3.92 (s, 2H), 3.52 (d, *J* = 6.0 Hz, 1H), 3.47 (s, 4H), 2.93 (d, *J* = 6.2 Hz, 1H), 2.87 - 2.59 (m, 2H), 2.15 (s, 6H), 1.30 (s, 6H), 1.06 (d, *J* = 5.5 Hz, 12H).

### Example 120

### Carvedilol 2-(4-(2,5-dioxo-3-(4-(trifluoromethyl)phenyl)imidazolidin-1-yl)methyl)-2,6-dimethylphenoxy)-2-methylpropionate (compound 120)

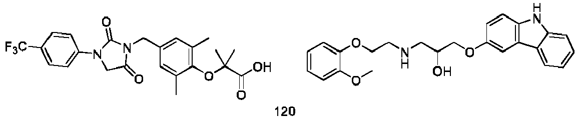

Referring to the method in Example 111, tromethamine in Example 111 was replaced by carvedilol to give compound 120 (white solid, 59.8 mg, 64.2% yield): ¹H NMR (300 MHz, DMSO-*d₆*) δ 11.23 (s, 1H), 8.21 (d, *J* = 7.8 Hz, 1H), 7.85 (d, *J* = 8.5 Hz, 2H), 7.76 (d, *J* = 8.6 Hz, 2H), 7.43 (d, *J* = 7.8 Hz, 1H), 7.30 (dd, *J* = 19.6, 7.6 Hz, 2H), 7.12 (d, *J* = 7.4 Hz, 1H), 7.06 (d, *J* = 8.0 Hz, 1H), 6.95 (d, *J* = 10.7 Hz, 2H), 6.93 (s, 2H), 6.91 - 6.80 (m, 2H), 6.67 (d, *J* = 7.8 Hz, 1H), 4.61 (s, 2H), 4.53 (s, 2H), 4.15 (s, 2H), 4.13 (s, 1H), 4.03 (t, *J* = 5.5 Hz, 2H), 3.72 (s, 3H), 3.02 - 2.92 (m, 3H), 2.85 (dd, *J* = 11.9, 5.9 Hz, 1H), 2.14 (s, 6H), 1.33 (s, 6H).

### Example 121

### Labetalol 2-(4-(2,5-dioxo-3-(4-(trifluoromethyl)phenyl)imidazolidin-1-yl)methyl)-2,6-dimethylphenoxy)-2-methylpropionate (compound 121)

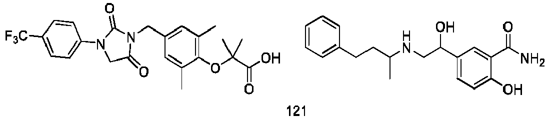

Referring to the method in Example 111, tromethamine in Example 111 was replaced by labetalol to give compound 121 (white solid, 69.1 mg, 81.6% yield): ¹H NMR (300 MHz, DMSO-*d₆*) δ 8.40 (s, 1H), 7.85 (d, *J* = 9.1 Hz, 4H), 7.75 (d, *J* = 8.4 Hz, 2H), 7.40 (d, *J* = 8.2 Hz, 1H), 7.24 (d, *J* = 6.5 Hz, 2H), 7.16 (d, *J* = 7.0 Hz, 4H), 6.95 (s, 2H), 6.84 (d, *J* = 8.4 Hz, 1H), 4.61 (s, 2H), 4.58 (s, 1H), 4.52 (s, 2H), 2.73 (s, 2H), 2.59 (s, 2H), 2.14 (s, 6H), 1.67 (d, *J* = 55.4 Hz, 2H), 1.31 (s, 6H), 1.08 (d, 3H).

### Example 122

### Diisopropylamine 2-(4-(2,5-dioxo-3-(4-(trifluoromethyl)phenyl)imidazolidin-1-yl)methyl)-2,6-dimethylphenoxy-2-methylpropionate (compound 122)

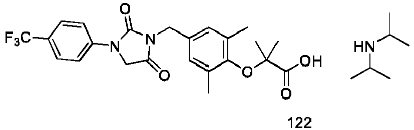

Referring to the method in Example 111, tromethamine in Example 111 was replaced by diisopropylamine to give compound 122 (white solid, 61.3 mg, 63.8% yield): ¹H NMR (300 MHz, DMSO-*d₆*) δ 7.86 (d, *J* = 8.8 Hz, 2H), 7.77 (d, *J* = 8.8 Hz, 2H), 6.94 (s, 2H), 4.63 (s, 2H), 4.53 (s, 2H), 3.11 (dt, *J* = 12.8, 6.5 Hz, 2H), 1.28 (s, 6H), 1.12 (s, 6H), 1.10 (s, 6H).

### Example 123

### Berberine 2-(4,5-dioxo-3-(4-(trifluoromethylphenyl)imidazolidin-1-yl)methyl)-2,6-dimethylphenoxy)-2-methylpropionate (compound 123)

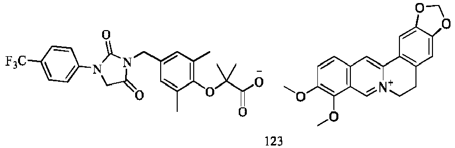

Referring to the method in Example 111, tromethamine in Example 111 was replaced by berberine to give compound 123 (white solid, 20.3 mg, 4.9% yield): ¹H NMR (300 MHz, DMSO-*d₆*) δ 9.90 (s, 1H), 8.95 (s, 1H), 8.21 (d, *J* = 9.3 Hz, 1H), 8.01 (d, *J* = 9.1 Hz, 1H), 7.86 (d, *J* = 8.8 Hz, 2H), 7.81 (s, 1H), 7.77 (d, *J* = 8.7 Hz, 2H), 7.10 (s, 1H), 6.90 (s, 2H), 6.18 (s, 2H), 4.94 (t, 21H), 4.63 (s, 2H), 4.51 (s, 2H), 4.10 (s, 3H), 4.08 (s,3H), 3.21 (t, 2H), 2.17 (s, 6H), 1.22 (s, 6H).

### Example 124

### Test of Agonistic Activities of Compounds for PPARα/PPARδ/PPARγ by Using GAL4 Hybrid Reporter Gene Method

Cos-7 cells (African green monkey kidney fibroblasts, commonly used tool cells) were cultured in a 10-cm cell culture dish with a DMEM complete medium containing 10% fetal bovine serum. When growing at a density of about 70%, the cells were ready for transfection. Firstly, a plasmid transfection working solution was prepared by the following procedures: 15 µg of pGL4.35-9×Gal4 UAS plasmid (purchased from Promega (Beijing) Biotech Co., Ltd.), 15 µg of pBIND-Gal4-PPARα (LBD) plasmid or pBIND-Gal4-PPARδ (LBD) plasmid or pBIND-Gal4-PPARy (LBD) plasmid (J. Chem. Inf. Model., 2020, 60, 1717), and 60 µL of a transfection reagent (HighGene, purchased from Wuhan ABclonal Biotechnology Co., Ltd.) were added to 2 mL of Opti-MEM, the mixture was left to stand at room temperature for 15 min, and then the plasmid transfection working solution was obtained. The working solution described above was then combined with 8 mL of the DMEM complete medium, and the mixture was added to the cell culture dish for cell transfection. After 4 h of transfection, the cells were digested, resuspended, and seeded into a 96-well plate at 25,000 cells/well. After 24 h of adherent culture, test compounds and a positive drug at appropriate test concentrations were prepared from the complete medium and added to the 96-well plate. In the test, the PPARα agonistic activity of GW7647 (purchased from MCE) at a final concentration of 10 nM was determined to be 100%, the PPARδ agonistic activity of GW501516 (purchased from MCE) at a final concentration of 10 nM was determined to be 100%, and the PPARγ agonistic activity of Rosiglitazone (purchased from Adamas) at a final concentration of 1 µM was determined to be 100%. After 16 h of drug action, the culture medium was removed, and 100 µL of a reporter gene lysis buffer (purchased from Shanghai Beyotime Biological Tech. Co., Ltd.) was added. The cells were shaken and lysed for 15 min, and then 10 µL of a lysate was pipetted and added into a white opaque 384-well plate. 10 µL of a reporter gene detection solution (purchased from Shanghai Beyotime Biological Tech. Co., Ltd.) was added. After the resulting solution was mixed and reacted, biological fluorescence was detected by using a multifunctional microplate reader, and corresponding half maximal effective concentration (EC₅₀) values were calculated according to the detection values. The experiment adopted the PPARα/δ agonist GFT505 in phase III clinical trials and the potent PPARα/δ agonist 5c reported in the literature (ACSMed. Chem. Lett., 2019, 10, 1068), and H11 (Journal of Medicinal Chemistry 2022, 65, 2571-2592) as positive control compounds. The experimental results are shown in Table 2.

**Table 2. Agonistic activities of compounds for PPARα/PPARδ/PPARγ**

| Compound No. | PPARα | PPARδ | PPARγ |
|---|---|---|---|
| | EC₅₀ (nM) | EC₅₀ (nM) | EC₅₀ (nM) |
| 1 | 0.7 | 0.4 | 1656.1 |
| 3 | 1.4 | 4.4 | 4147.8 |
| 5 | 251.6 | 60.0 | 1623.0 |
| 7 | 668.1 | 175.8 | 1240.3 |
| 9 | 994.0 | 976.0 | >5000 |
| 11 | 77.1 | 33.1 | 2484.3 |
| 13 | 1.2 | 10.8 | 1286.6 |
| 17 | 7.6 | 24.8 | 4867.5 |
| 19 | 2.2 | 23.6 | 2786.0 |
| 21 | 42.9 | 100.8 | >5000 |
| 23 | 26.5 | 25.7 | 628.8 |
| 25 | 100.6 | 72.3 | 3637.8 |
| 27 | 17.0 | 7.0 | 1416.4 |
| 29 | 1.4 | 1.0 | 736.4 |
| 31 | 2.1 | 0.8 | 1070.7 |
| 33 | 1.0 | 1.7 | 656.9 |
| 35 | 165.6 | 90.5 | 2105.0 |
| 37 | 98.1 | 84.4 | 4060.0 |
| 39 | 18.8 | 36.1 | 3372.5 |
| 41 | 0.9 | 6.2 | 629.6 |
| 43 | 2.7 | 8.2 | 1173.7 |
| 45 | 3.8 | 5.9 | 996.3 |
| 47 | 9.9 | 19.0 | >5000 |
| 51 | 89.9 | 40.1 | >5000 |
| 53 | 325.2 | 174.8 | >5000 |
| 55 | 82.1 | 24.0 | 2959.4 |
| 57 | 46.7 | 19.2 | 2720.1 |
| 59 | 276.9 | 358.9 | >5000 |
| 61 | 3.9 | 1.9 | 1204.5 |
| 63 | 25.1 | 4.0 | >5000 |
| 65 | 0.8 | 22.9 | 971.4 |
| 67 | 14.1 | 3.4 | 1920.3 |
| 69 | 1.5 | 67.3 | 1796.0 |
| 71 | 1094.0 | >5000 | >5000 |
| 73 | 2.4 | 31.0 | 2116.3 |
| 75 | 1.0 | 6.3 | 1646.0 |
| 77 | 74.1 | 25.3 | 3193.0 |
| 81 | 165.2 | 82.1 | >5000 |
| 83 | 79.3 | 6.5 | 1682.0 |
| 85 | 17.9 | 4.4 | 2722 |
| 87 | 352.6 | 352.4 | 3398.7 |
| 89 | 67.0 | 20.3 | 627.9 |
| 91 | 38.0 | 632.5 | 2691.9 |
| 93 | 866.7 | 3458.0 | 1487.8 |
| 95 | 10.9 | 66.9 | 1333.0 |
| 97 | 209.3 | 1398.0 | 2890.0 |
| 99 | 402.2 | >5000 | >5000 |
| 101 | 1.0 | 10.2 | 312.5 |
| 103 | 101.6 | 97.6 | 473.9 |
| 105 | 21.9 | 40.5 | 164.9 |
| 107 | 105.5 | 47.5 | 652.2 |
| 109 | 31.7 | 15.1 | 362.7 |
| GFT505 | 820 | 843 | 1844 |
| 5c | 65 | 24 | 2753 |
| H11 | 7.0 | 8.4 | 1316.1 |

The experimental results (Table 2) show that the compounds of the present invention had significant PPAR agonistic activities. For example, EC₅₀ values of compounds 1, 3, 29, 33, 41, 43, 45, 61, 75, 101, and the like for the PPARα and PPAR6 agonistic activities all reached low nanomolar levels. Especially, compound 1 (PPARα: EC₅₀ = 0.7 nM; PPARδ: EC₅₀ = 0.4 nM) had EC₅₀ values for the PPARα and PPARδ agonistic activities at picomolar levels and had very good selectivity (more than 2000-fold selectivity) for PPARγ. The above results suggest that the compounds of the present invention are potent and highly selective PPARα/PPARδ dual agonists.

### Example 125

### Evaluation of Metabolic Stability of Compound in Human Liver Microsomes

A solution of the compound in acetonitrile at a concentration of 500 µM was prepared and diluted in 0.1 M potassium phosphate solution to obtain a 1.5 µM drug working solution. The drug working solution was co-incubated with a human liver microsome working solution at a final concentration of 0.75 mg/mL and a NADPH solution (at a final concentration of 550 µM), and the incubation was terminated by adding an acetonitrile solution at 0 min, 15 min, 30 min, 45 min, and 60 min, respectively. The remaining amount of the compound remained in the system at each time point was detected by using LC/MS, the absolute value k of the slope was measured by plotting the natural logarithm of the percentage of the remaining amount of the compound over time, and the calculation was performed according to the formula: T_{1/2} (half-life) = ln2/k = 0.693/k. The experimental results are shown in Table 3.

**Table 3. Results of metabolic stability of compound in human liver microsomes**

| Compound No. | T_{1/2} (min) | Cₗᵢₙₜ (mL/min/kg) |
|---|---|---|
| 1 | >120 | 6.2 |
| GFT505 | 15 | 116.4 |

The experimental results (Table 3) show that compound 1 has very good metabolic stability in human liver microsomes, and the metabolic stability for human liver microsomes is far better than that of GFT505 under the same test conditions. Other compounds of the present invention also have good metabolic stability in human liver microsomes.

### Example 126

### Pharmacokinetic Evaluation of Compound 1 in Rats

Animals: 6 SPF-grade male SD rats were purchased from Beijing Vital River Laboratory Animal Technology Co., Ltd.

Grouping: rats were divided into 2 groups, with 3 rats in each group; one group was an oral administration group, and the other group was an intravenous injection group. The dose in the oral administration group was 10 mpk, and the dose in the intravenous injection group was 2 mpk.

Experimental procedures: after rats in the intravenous injection group were administered by tail vein injection, about 0.25 mL of blood was collected from the orbit at 0.083 h, 0.25 h, 0.5 h, 1 h, 2 h, 4 h, 6 h, 8 h, and 24 h, respectively, and then heparin sodium was rapidly added for anticoagulation after blood collection. The blood was placed on ice after collection. Rats in the oral administration group were fasted for 12 h before administration and fed 4 h after administration. After the rats were administered orally, about 0.25 mL of blood was collected from the orbit at 0.083 h, 0.25 h, 0.5 h, 1 h, 2 h, 4 h, 6 h, 8 h, and 24 h, respectively, and then heparin sodium was rapidly added for anticoagulation after blood collection. The blood was placed on ice after collection. All samples were centrifuged at 6000 r/min for 3 min in a low-temperature centrifuge, and plasma was obtained after separation. The content of the compound in the plasma was detected via LC-MS/MS-18, and relevant pharmacokinetic parameters were calculated according to the plasma concentration data of different time points. The experimental results are shown in Table 4.

**Table 4. Pharmacokinetic parameters of compound 1 after intravenous injection and oral administration in rats**

| Route of administration | Intravenous injection | Oral administration |
|---|---|---|
| Half-life T_{1/2} (h) | 4.82±1.05 | 6.72±1.39 |
| Peak time Tₘₐₓ (h) | 0.08±0.00 | 2.25±3.25 |
| Peak concentration Cmax (ng/mL) | 4306.67±1546.13 | 2816.67±1020.41 |
| Area under the curve AUC_{(0-∞)} (h*ng/mL) | 4252.83±1241.55 | 22264.47±3548.39 |
| Bioavailability F (%) | | 104.70±0.17 |

The experimental results (Table 4) show that the oral half-life of compound 1 was 6.72±1.39 h, and the bioavailability of the compound was 104.70±0.17%, indicating that compound 1 has good pharmacokinetic properties. Other compounds of the present invention also have relatively good *in-vivo* pharmacokinetic properties.

### Example 127

### High Selectivity of Compound 1 for PPARα/δ

The agonistic effects of compound 1 on common nuclear receptors were tested by using the GAL4 hybrid reporter gene method.

Reporter gene plasmids for different nuclear receptors were constructed by referring to the method in the literature (Journal of Medicinal Chemistry 2022, 65, 2571-2592). Transfection working solutions for different nuclear receptors were prepared and added into COS-7 cells. The preparation method for the transfection working solution was as follows: the constructed pBIND-Gal4-PPARα (LBD) (or pBIND-Gal4-PPARδ (LBD) plasmid or pBIND-Gal4-PPARγ (LBD) plasmid or pBIND-Gal4-RARα (LBD) plasmid or pBIND-Gal4-RARγ (LBD) plasmid or pBIND-Gal4-RARβ (LBD) plasmid or pBIND-Gal4-RORα (LBD) plasmid or pBIND-Gal4-RORy (LBD) plasmid or pBIND-Gal4-RORβ (LBD) plasmid or pBIND-Gal4-FXR (LBD) plasmid or pBIND-Gal4-RXRα (LBD) plasmid or pBIND-Gal4-RXRγ (LBD) plasmid or pBIND-Gal4-RXRβ (LBD) plasmid or pBIND-Gal4-VDR (LBD) plasmid or pBIND-Gal4-LXRα (LBD) plasmid or pBIND-Gal4-LXRβ (LBD) plasmid or pBIND-Gal4-THβ (LBD) plasmid or pBIND-Gal4-PXR (LBD) plasmid or pBIND-Gal4-CAR (LBD) plasmid), 15 µg of pGL4.35-9×Gal4 UAS plasmid (purchased from Promega (Beijing) Biotech Co., Ltd.), and 60 µL of a transfection reagent (HighGene, purchased from Wuhan ABclonal Biotechnology Co., Ltd.) were added to 2 mL of Opti-MEM, the mixture was left to stand at room temperature for 15 min, and then the working solution was obtained. The agonism of compound 1 for various types of nuclear receptors was tested at a concentration of 1 µM.

The experimental results (Table 5) show that compound 1 had no significant agonistic effect on non-PPAR nuclear receptors at a concentration of 1 µM, and had a relatively weak agonistic effect on PPARγ. Therefore, compound 1 is considered to have high selectivity for nuclear receptors PPARα/δ. Other compounds of the present invention also have similar effects.

**Table 5. Agonistic fold of compound 1 for common nuclear receptors**

| Nuclear receptor | Agonistic fold | Nuclear receptor | Agonistic fold |
|---|---|---|---|
| VDR | 1.182±0.095 | RARβ | 1.536±0.090 |
| FXR | 1.231±0.055 | RARγ | 0.717±0.059 |
| PXR | 1.039±0.178 | THRβ | 1.295±0.296 |
| RXRα | 1.216±0.116 | RORα | 1.265±0.189 |
| RXRβ | 1.141±0.072 | RORβ | 1.010±0.030 |
| RXRγ | 1.299±0.087 | RORγ | 1.293±0.127 |
| LXRα | 1.374±0.101 | PPARα | 14.703±1.158 |
| LXRβ | 1.285±0.043 | PPARδ | 15.869±1.182 |
| RARα | 0.984±0.182 | PPARγ | 3.312±0.182 |

### Example 128

### Compound 1 Capable of Effectively Activating Expression of PPARα/δ Downstream Target Genes in Mouse Liver and Skeletal Muscle

C57 mice were divided into three groups, i.e., a control group, a low dose group (0.03 mg/kg), and a high dose group (0.1 mg/kg), with 6 mice in each group. Each group of mice was intragastrically administered compound 1 or a solvent control with the same volume for three consecutive days according to the doses. Three days after administration, the mice were euthanized and dissected for sampling. Liver and skeletal muscle were snap-frozen in liquid nitrogen for subsequent experiments. After extracting RNA in the liver and skeletal muscle, the up-regulation fold of the expression of the PPARα/δ downstream target genes was detected. The results (Table 6) show that Pdk4, Acox1, Vlcad, and Angptl4 were significantly up-regulated in the liver, and Pdk4 and Angptl4 were significantly up-regulated in the skeletal muscle, all of which were dose-dependent. This suggests that compound 1 may have a dual agonistic effect on PPARα/δ in the mice. Some of other compounds of the present invention also have similar effects.

**Table 6. Up-regulation fold of expression of PPARα/δ downstream target genes in mouse liver and skeletal muscle by compound 1**

| Gene | Up-regulation fold at a dose of 0.03 mg/kg | Up-regulation fold at a dose of 0.1 mg/kg |
|---|---|---|
| Liver Pdk4 | 62.9±7.7 | 282.8±28.8 |
| Liver Acox1 | 3.1±0.4 | 5.0±0.4 |
| Liver Vlcad | 1.6±0.2 | 2.5±0.1 |
| Liver Angptl4 | 2.1±0.2 | 2.6±0.2 |
| Skeletal muscle Pdk4 | 1.1±0.1 | 2.8±0.3 |
| Skeletal muscle Angptl4 | 1.4±0.2 | 5.3±0.6 |

### Example 129

### Compound 1 Capable of Effectively Reducing Serum Triglyceride Levels in Mice

Animals: 48 male C57 mice, SPF grade, 8 weeks old, weighing about 20 g, purchased from Beijing Vital River. All animals maintained a 12-hour alternating circadian rhythm and were given *ad libitum* access to food and water.
Instruments: a scale for weighing animals; an automatic biochemical analyzer
Reagents: compound 1, the positive drug GFT505 (a PPARα/δ dual agonist, currently in the anti-NASH phase III clinical trials, the preparation method referred to CN100548960C), the positive drug fenofibrate (a PPARα agonist, a drug clinically used for the treatment of hypertriglyceridemia, purchased from Aladdin), and the positive drug pemafibrate (a PPARα agonist, a drug marketed in Japan for the treatment of hypertriglyceridemia, the preparation method referred to Bioorg. Med. Chem. Lett., 2007, 17).

### Experimental procedures

### 1. Animal grouping and administration

After 1 week of adaptive feeding, the mice were divided into 5 groups according to their body weight: a control group, a positive drug fenofibrate (30 mg/kg) group, a positive drug pemafibrate (1 mg/kg) group, a positive drug GFT505 (1 mg/kg) group, and a compound 1 (1 mg/kg) group. Mice in the control group were administered a solvent control CMC-Na every day, and mice in each of the administration groups were administered the corresponding drug every day. Intragastric administration was performed for 5 consecutive days, and the mice were given *ad libitum* access to food and water during administration. Each group of mice was weighed every day, and their body weight, hair, feces, and activities were carefully observed and recorded.

### 2. Sampling

Two hours after administration on day 5, blood was collected from the orbit, and the mice were euthanized.

### 3. Test of serum triglycerides

The whole blood was left to stand at room temperature for 2 h and centrifuged at 3000 rpm for 15 min, and serum was collected. The serum was placed onto the automatic biochemical analyzer (Google Biotechnology Co., Ltd.) to determine the triglyceride (TG) levels in the serum.

### 4. Experimental results

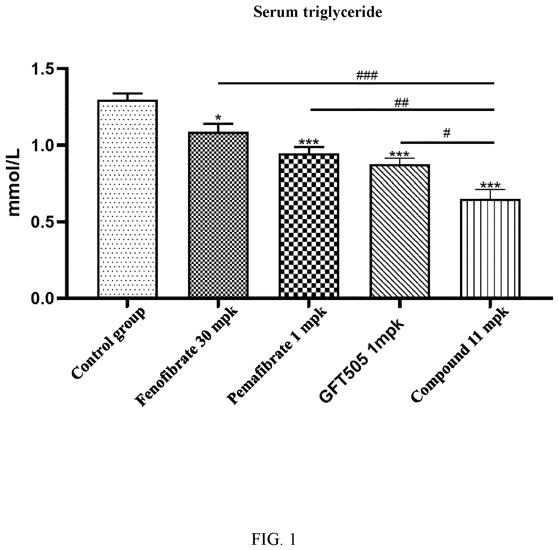

The results in FIG. 1 show that compound 1 could significantly reduce the triglyceride level in mouse serum. It is noted that compound 1, at a dose of 1 mg/kg, was significantly more effective in reducing triglycerides than GFT505 and pemafibrate at the equivalent dose, and was superior to fenofibrate, a commonly used clinical lipid-lowering drug. This suggests that compound 1 may be used for preventing and treating metabolic diseases such as hypertriglyceridemia. Other compounds of the present invention also have similar effects.

### Example 130

Improving Effect of Compound 1 on α-Naphthylisothiocyanate-Induced Cholestasis in Mice
Animals: 25 male C57 mice, SPF grade, 8 weeks old, weighing about 20 g, purchased from Beijing Vital River. All animals maintained a 12-hour alternating circadian rhythm and were given *ad libitum* access to food and water.
Instruments: a scale for weighing animals; an automatic biochemical analyzer; an inverted microscope; a microtome
Reagents: compound 1, the positive drug GFT505, and α-naphthylisothiocyanate (ANIT).

### Experimental procedures

### 1. Animal grouping and administration

After 1 week of adaptive feeding, the mice were divided into 5 groups according to their body weight: a control group, a model group, a positive drug GFT505 (30 mg/kg) group, a compound 1 low-dose (0.03 mg/kg) group, and a compound 1 high-dose (0.1 mg/kg) group. The mice were all fed food and water normally.

The molding and administration processes were as follows: the mice in each of the administration groups were intragastrically administered the corresponding doses and types of compounds 6 h before modeling, and the mice in the control group and the model group were administered solvent controls with the same volume. When modeling, except for the control group, the mice in each group were intragastrically administered ANIT at a dose of 80 mg/kg, and the mice in the control group were administered the solvent control with the same volume. The administration was then continued for two days, once per day. Each group of mice was weighed every day, and their body weight, hair, feces, and activities were carefully observed and recorded.

### 2. Sampling

Forty-eight hours after giving ANIT for molding, blood was collected from the orbit, the mice were euthanized, and the liver was collected. The right lobular tissue of the liver was fixed with 4% paraformaldehyde and used for sectioning followed by HE staining. The remaining liver tissues were snap-frozen in liquid nitrogen for subsequent assays of other indexes.

### 3. Serological assay

The whole blood was left to stand at room temperature for 2 h and centrifuged at 3000 rpm for 15 min, and serum was collected. Levels of aspartate aminotransferase (AST), alanine aminotransferase (ALT), alkaline phosphatase (ALP), total bilirubin (TBil), and total bile acid (TBA) in the serum were assayed.

### 4. Liver tissue sectioning

The fixed tissue was sent to Wuhan Servicebio Technology Co., Ltd. for preparing HE-stained sections.

### 5. Assay of alkaline phosphatase in liver

The test was performed using a tissue and blood alkaline phosphatase (AKP/ALP) activity assay kit (BC2145) purchased from Beijing Solarbio Science & Technology Co., Ltd. The liver tissue stored at -80 °C was taken and placed in liquid nitrogen, about 0.1 g of liver tissue was rapidly cut off, and 1 mL of an extracting solution was added. The mixture was ground thoroughly and centrifuged at 4 °C and 10000 rpm for 10 min, and the supernatant was collected for assay. The assay was performed by referring to the method in the product's instruction. Data were corrected according to protein concentrations of the samples, and the catalytic production of 1 µmol phenol per minute per milligram protein at 37 °C was defined as one enzymatic activity unit.

### 6. Experimental results

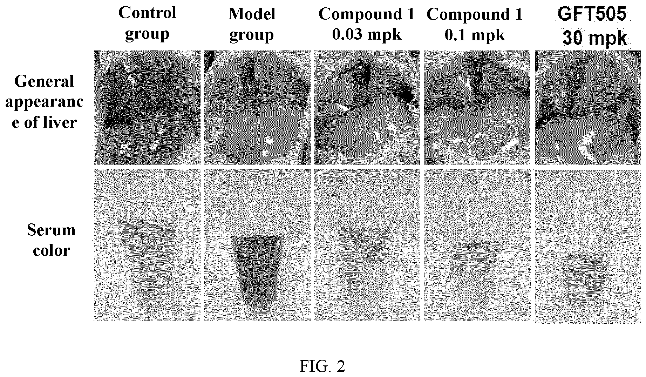

The results in FIG. 2 show that compound 1 could effectively improve intrahepatic cholestasis caused by ANIT. The liver in the model group was yellow overall, and had massive bleeding points and yellow crystalline substances, and the serum was brown yellow. After administration of compound 1, the liver of the mouse returned to red without visible bleeding points, and the serum was pale yellow. It is noted that at a dose of 0.03 mg/kg, the ability of compound 1 to improve the general appearance of the liver and the serum color was comparable to the effect of GFT505 at 30 mg/kg.

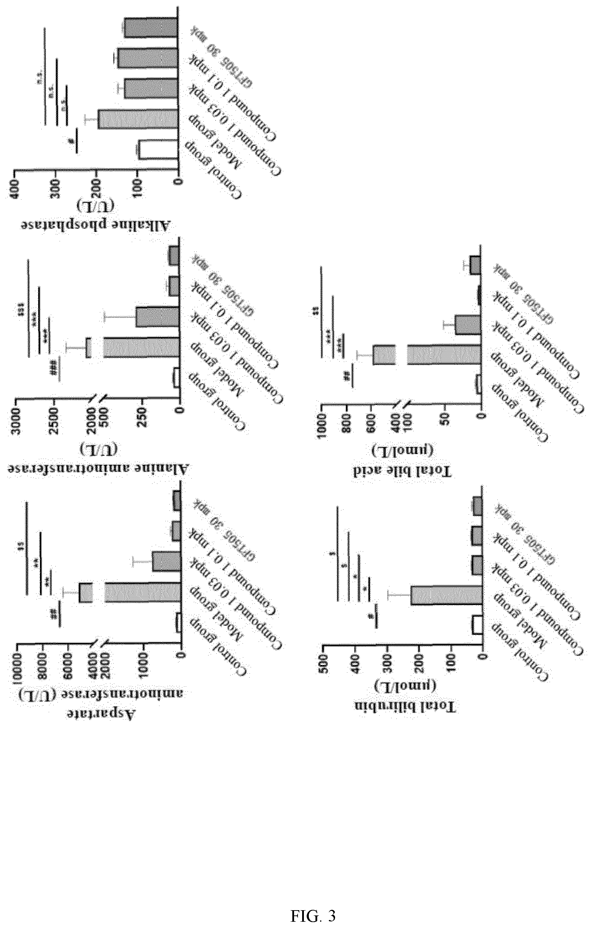

FIG. 3 shows the results of serological assays in mice and shows that compound 1 could dose-dependently and significantly down-regulate the serum ALT, AST, TBil, and TBA levels in the ANIT-induced cholestatic model mice. There was also a certain tendency to reduce the serum ALP. It is noted that at a dose of 0.1 mg/kg, the effects of compound 1 on reducing the levels of hepatic enzymes, total bilirubin, and total bile acid were comparable to that of GFT505 at 30 mg/kg, indicating that compound 1 can effectively alleviate cholestasis and has a potent hepatoprotective effect.

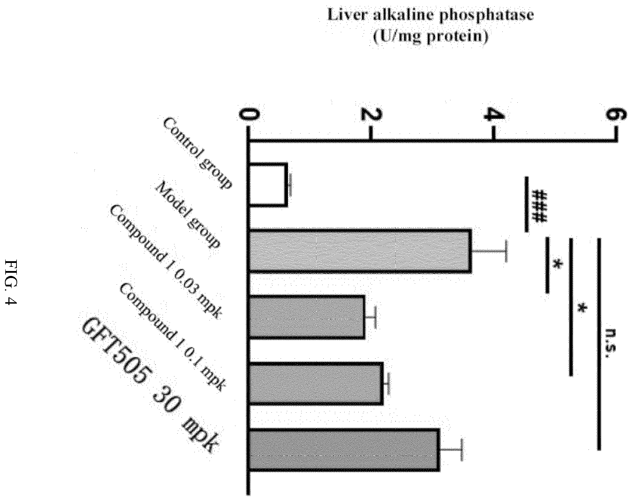

FIG. 4 shows an assay of the ALP level in the mouse liver. Since the fold increase in serum ALP was small in the ANIT model, the level of ALP in liver tissues was also assayed. As shown in FIG. 4, compound 1 could significantly down-regulate the level of intrahepatic ALP. It is noted that the effect of compound 1 on reducing the level of intrahepatic ALP at the two doses was slightly superior to that of GFT505 at 30 mg/kg.

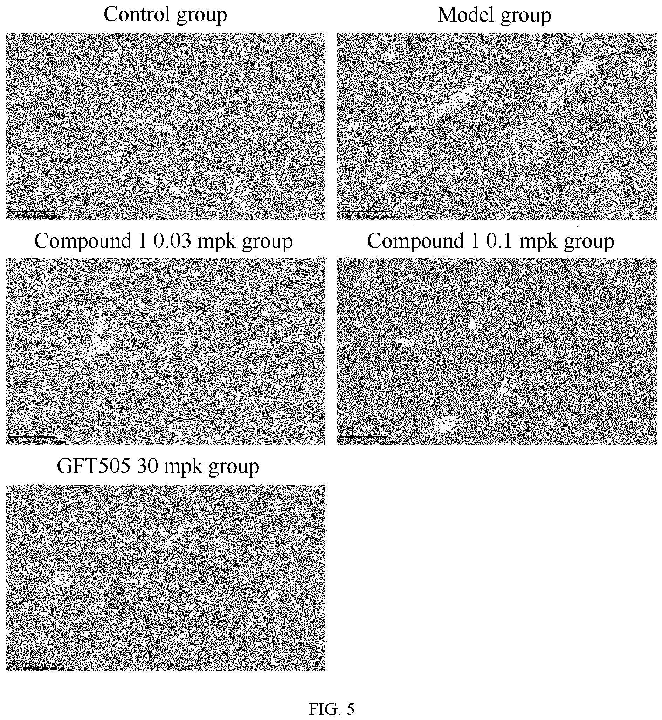

In addition, the anti-cholestasis effect of compound 1 was evaluated using a method in pathological research. The HE staining results (FIG. 5) show that compound 1 could dose-dependently reduce the number and area of necrotic areas in the liver, suggesting that compound 1 has excellent resistance to liver damage.

In conclusion, compound 1 has a potent therapeutic effect on a mouse cholestatic model, which suggests that compound 1 has a therapeutic effect on cholestatic liver diseases and can be used for preparing medicaments for the prevention and treatment of cholestatic liver diseases such as primary biliary cholangitis (PBC) and primary sclerosing cholangitis (PSC). Other compounds of the present invention also have similar effects.

### Example 131

### Improving Effect of Compound 1 on Nonalcoholic Steatohepatitis (NASH) Induced by High-Fat Choline-Deficient Methionine-Defined (HF-CDAA) Diet in Mice

Animals: 24 male C57 mice, SPF grade, 8 weeks old, weighing about 20 g, purchased from Beijing Vital River. All animals maintained a 12-hour alternating circadian rhythm and were given *ad libitum* access to food and water.
Instruments: a scale for weighing animals; a microtome; an automatic biochemical analyzer; an inverted microscope
Reagents: compound 1, the positive drug GFT505; control feeds purchased from Nantong Trophic Animal Feed High-tech Co. Ltd. (TP36225 MCS); molding feeds purchased from Nantong Trophic Animal Feed High-tech Co. Ltd. (TP36225 MCD).

### Experimental procedures:

### 1. Animal grouping and modeling

After 1 week of adaptive feeding, the mice were randomized into 4 groups according to the body weight: a control group, a model group, a positive drug GFT505 (10 mg/kg) group, and a compound 1 (0.1 mg/kg) group. The mice in the control group were fed control feeds (TP36225 MCS); the mice in other groups were given molding feeds (TP36225 MCD). All the mice were fed water normally, and the modeling lasted for 6 weeks.

### 2. Administration

After 4 weeks of molding, the mice in the positive drug GFT505 group were intragastrically administered GFT505 at 10 mg/kg every day; the mice in the compound 1 group were intragastrically administered compound 1 at 0.1 mg/kg every day; the mice in the control group and the model group were intragastrically administered control solvents with the same volume every day. The administration was performed for 2 weeks, during which time the mice in the control group were given the control feeds, and the mice in the remaining groups were given the molding feeds. All the mice were fed water normally. Each group of mice was weighed every day, and their body weight, hair, feces, and activities were carefully observed and recorded.

### 3. Sampling

After 2 weeks of administration, the mice were fasted for 6 h in advance but had free access to water. Blood was collected from the orbit, the mice were euthanized, and the liver was collected. The right lobular tissue of the liver was fixed with 4% paraformaldehyde and used for HE and oil red staining. The remaining liver tissues were divided into 2 parts and snap-frozen in liquid nitrogen for subsequent assays of other indexes.

### 4. Determination of biochemical indexes

The whole blood was left to stand at room temperature for 2 h and centrifuged at 3000 rpm for 15 min, and serum was collected. The levels of aspartate aminotransferase (AST) and alanine aminotransferase (ALT) in the serum were determined by using the automatic biochemical analyzer.

### 5. Liver tissue sectioning

The fixed tissue was sent to Wuhan Servicebio Technology Co., Ltd. for preparing HE-stained sections, sirius red-stained sections, and oil red-stained sections.

### 6. Extraction and q-PCR assay of RNA in liver tissue

The liver tissue stored at -80 °C was taken and placed in liquid nitrogen, and about 10 mg of liver tissue was rapidly cut off. About 500 µL of a pre-cooled RNA extraction reagent (Nanjing Vazyme Biotech Co., Ltd., R401-01) was added, and the tissue was homogenized in a homogenizer. The precipitated RNA was extracted by referring to the method in the product's instruction. The solid RNA was dissolved in an appropriate amount of DEPC-treated water. The RNA concentration was quantified using NanoDrop, a reverse transcription reagent purchased from Vazyme Biotech Co., Ltd. was added according to the instruction, and mRNA was reverse-transcribed into cDNA using a common PCR instrument. Finally, upstream and downstream primers of the target gene, q-PCR reagents (SYBR Green), and cDNA were added to a 96-well plate dedicated to q-PCR, and amplification and quantification were performed using the q-PCR instrument. A ΔΔCt value was selected to represent the difference in gene expression, and relevant software was adopted for data processing and statistical tests.

### 7. Extraction and assay of triglyceride in liver tissue

The liver tissue stored at -80 °C was taken and placed in liquid nitrogen, about 10 mg of liver tissue was rapidly cut off, and 300 µL of methanol was added. The tissue was homogenized and lysed. Then, 600 µL of chloroform was added, the mixture was shaken at room temperature overnight, and liposoluble substances were extracted from the tissue. The mixture was centrifuged at 6000 rpm for 10 min, and the supernatant was collected. The test was performed according to a triglyceride assay kit (290-63701) from WAKO, Japan, and the final results were corrected to milligrams of triglyceride per gram of liver tissue.

### 8. Experimental results

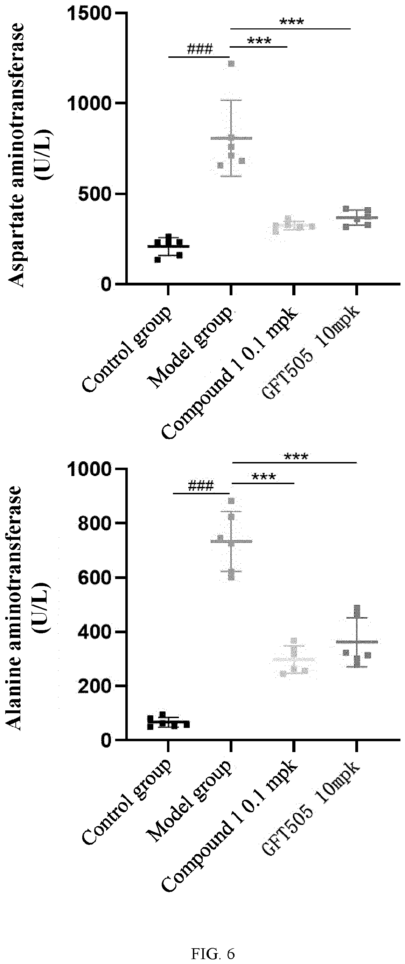

The results in FIG. 6 show that compound 1 down-regulated serum ALT and AST levels in NASH model mice at a dose of 0.1 mg/kg. It is noted that compound 1 at 0.1 mg/kg had a slightly better effect on reducing the levels of hepatic enzymes than that of GFT505 at 10 mg/kg, indicating that compound 1 has a very strong hepatoprotective effect in the NASH model.

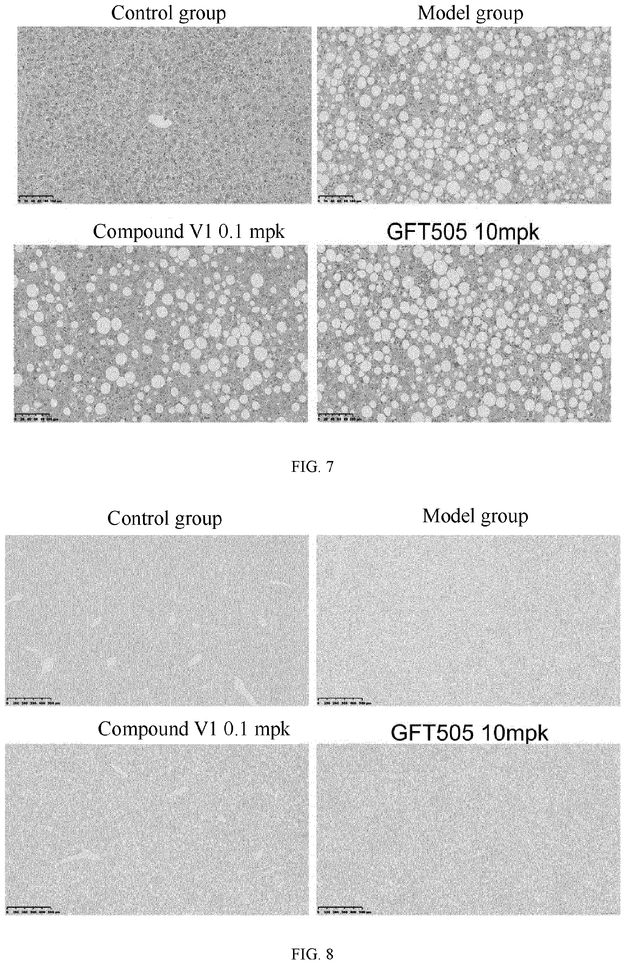

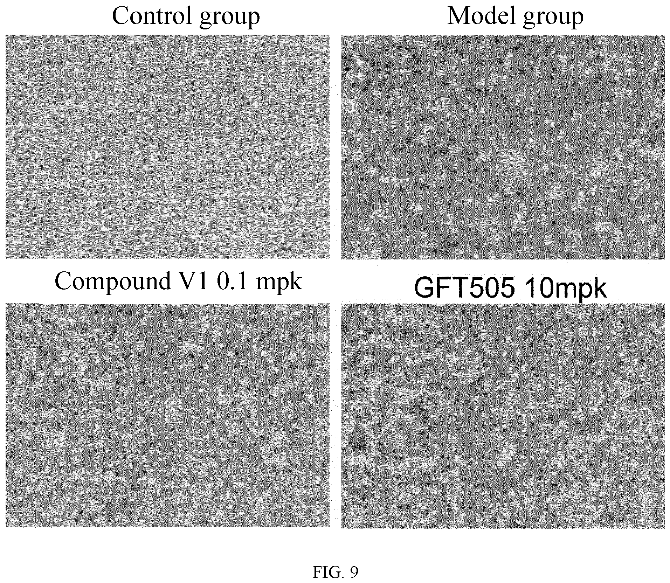

The anti-NASH effect of compound 1 was observed through a method in pathological research. The HE staining results (FIG. 7) show that compound 1 could reduce inflammatory infiltration appearing in mouse hepatic lobules. Sirius red staining results (FIG. 8) show that compound 1 could reduce collagen deposition in the mouse liver. The oil red staining results (FIG. 9) show that compound 1 could reduce the number and area of lipid droplets in the mouse liver. It is noted that the effect of compound 1 at a dose of 0.1 mg/kg on reducing lipid accumulation was superior to that of GFT505 at 10 mg/kg.

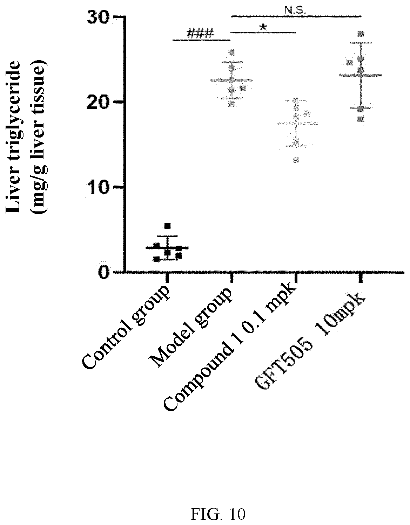

FIG. 10 shows an assay of the triglyceride content in the mouse liver. The results show that compound 1 could reduce triglyceride accumulation in the liver tissue induced by HF-CDAA modeling. It is noted that the effect of compound 1 at a dose of 0.1 mg/kg on reducing hepatic triglycerides was superior to that of GFT505 at 10 mg/kg.

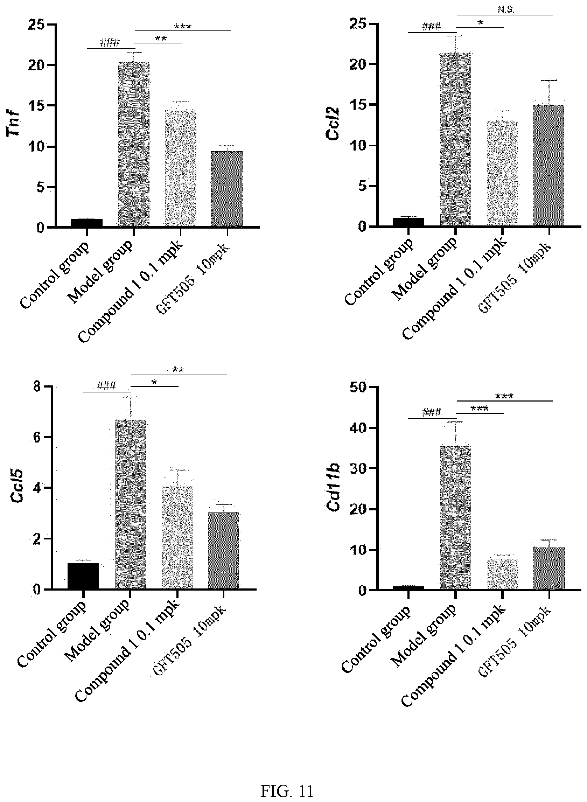

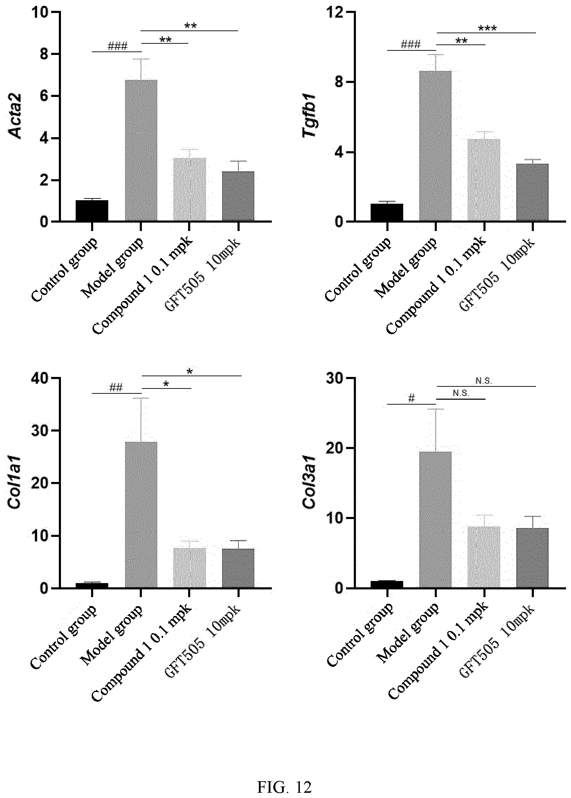

To further assay the effect of compound 1 on reducing liver inflammation and fibrosis in NASH model mice, mRNA expression levels of the relevant inflammatory factors and fibrosis-associated cytokines in the liver tissue were determined (see Table 7 for primer sequences of genes). The experimental results are shown in FIGs. 11 and 12.

**Table 7. Primer sequences of genes**

| Primer name | Primer sequence |
|---|---|
| Mus_*Tnf*_Forward Primer | CCCTCACACTCAGATCATCTTCT |
| Mus_*Tnf*_Reverse Primer | GCTACGACGTGGGCTACAG |
| Mus_*Ccl2*_Forward Primer | TTAAAAACCTGGATCGGAACCAA |
| Mus_*Ccl2*_Reverse Primer | GCATTAGCTTCAGATTTACGGGT |
| Mus_*Ccl5*_Forward Primer | GCTGCTTTGCCTACCTCTCC |
| Mus_Cc/*5*_Reverse Primer | TCGAGTGACAAACACGACTGC |
| Mus_*Cd11b*_Forward Primer | ATGGACGCTGATGGCAATACC |
| Mus_*Cd11b*_Reverse Primer | TCCCCATTCACGTCTCCCA |
| Mus_*Acta2_*Forward Primer | GTCCCAGACATCAGGGAGTAA |
| Mus_*Acta2*_Reverse Primer | TCGGATACTTCAGCGTCAGGA |
| Mus_*tgfb1*_Forward Primer | CTCCCGTGGCTTCTAGTGC |
| Mus_*tgfb1*_Reverse Primer | GCCTTAGTTTGGACAGGATCTG |
| Mus_*Col1a1_*Forward Primer | GCTCCTCTTAGGGGCCACT |
| Mus_*Col1a1_*Reverse Primer | CCACGTCTCACCATTGGGG |
| Mus_*Col3a1_*Forward Primer | CTGTAACATGGAAACTGGGGAAA |
| Mus_*Col3a1*_Reverse Primer | CCATAGCTGAACTGAAAACCACC |

The results in FIG. 11 show that compound 1 could inhibit the increase in the mRNA expression levels of *Tnf, Ccl2, Ccl5,* and *Cd11b* induced by HF-CDAA, indicating that compound 1 has a potent anti-inflammatory effect in the NASH. The results in FIG. 12 show that compound 1 could inhibit the increase in the mRNA expression levels of *Acta2, Tgfbl, Col1a1,* and *Col3a1* induced by HF-CDAA, indicating that compound 1 has an anti-fibrosis effect in the NASH.

The above results show that compound 1 could significantly improve the pathological state of NASH mice at a dose of 0.1 mg/kg, reduce the levels of hepatic enzymes, inhibit the development and progression of liver inflammation and fibrosis, and had an equivalent therapeutic effect to that of GFT505 at a dose of 10 mg/kg. The effect of the compound at 0.1 mg/kg on reducing lipid accumulation in the liver was superior to that of GFT505 at 10 mg/kg. It is suggested that compound 1 has therapeutic effects on fatty liver diseases such as NASH, and can be used for preparing medicaments for the prevention and treatment of chronic liver diseases such as nonalcoholic fatty liver disease (NAFLD), nonalcoholic steatohepatitis (NASH), metabolic fatty liver disease (MAFLD), alcoholic fatty liver disease (ALD), and the like. Other compounds of the present invention also have similar effects.

### Example 132

### Improving Effect of Compound 1 on Tetrachloromethane-Induced Hepatic Fibrosis in Mice

Animals: 30 male C57 mice, SPF grade, 8 weeks old, weighing about 20 g, purchased from Beijing Vital River. All animals maintained a 12-hour alternating circadian rhythm and were given *ad libitum* access to food and water.
Instruments: a scale for weighing animals; a microtome; an automatic biochemical analyzer; an inverted microscope
Reagents: compound 1, the positive drug GFT505, tetrachloromethane (purchased from Shanghai Aladdin Biochemical Technology Co., Ltd.), and sunflower seed oil (purchased from Shanghai Yuanye Biotech Co., Ltd.).

### Experimental procedures:

### 1. Animal grouping and modeling

After 1 week of adaptive feeding, the mice were randomized into 5 groups according to the body weight: a control group (Oil), a model group (CCl₄), a positive drug GFT505 (10 mg/kg) group (CCl₄ + GFT505), a compound 1 low-dose (0.03 mg/kg) group (CCl₄ + 1 low dose), and a compound 1 high-dose (0.1 mg/kg) group (CCl₄ + 1 high dose). The mice were all fed food and water normally, and the modeling lasted for 3 weeks. The mice in the model group and each of the administration groups were injected twice a week with a 25% CCl₄ oil solution at a dose of 2 mL/kg, and the mice in the control group were injected with an oil solvent with the same volume.

### 2. Administration

The administration was started at the same time of molding. The mice in the CCl₄ + GFT505 group were intragastrically administered GFT505 (10 mg/kg) every day; the mice in the CCl₄ + 1 low dose group were intragastrically administered compound 1 (0.03 mg/kg) every day; the mice in the CCl₄ + 1 high dose group were intragastrically administered compound 1 (0.1 mg/kg) every day; the mice in the control group and the model group were intragastrically administered control solvents with the same volume every day. After 3 weeks of administration, the mice were all fed food and water normally. Each group of mice was weighed every day, and their body weight, hair, feces, and activities were carefully observed and recorded.

### 3. Sampling

Twenty-four hours after the sixth injection of CCl₄, the mice were dissected for sampling. Blood was collected from the orbit, the mice were euthanized, and the liver was collected. The right lobular tissue of the liver was fixed with 4% paraformaldehyde and used for sectioning followed by HE and sirius red staining. Part of liver tissues were divided into 3 parts and snap-frozen in liquid nitrogen for subsequent assays of other indexes.

### 4. Liver tissue sectioning

The pre-treated tissue was sent to Wuhan Servicebio Technology Co., Ltd. for preparing HE-stained sections and sirius red-stained sections.

### 5. Assay of hydroxyproline in liver tissue.

The liver tissue stored at -80 °C was taken and placed in liquid nitrogen, and about 200 mg of liver tissue was rapidly cut off. Hydroxyproline in the liver tissue was assayed according to the method in the product's instruction (Beijing Solarbio Science & Technology Co., Ltd., BC0255).

### 6. Experimental results

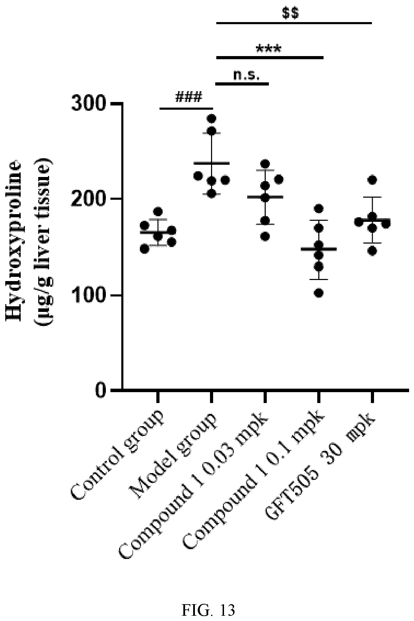

The results in FIG. 13 show that tetrachloromethane modeling could significantly increase the hydroxyproline content in the liver. Moreover, the administration of compound 1 could dose-dependently reduce the hydroxyproline content in the liver of the model mice, and the effect of compound 1 at a dose of 0.1 mg/kg was superior to that of GFT505 at 30 mg/kg.

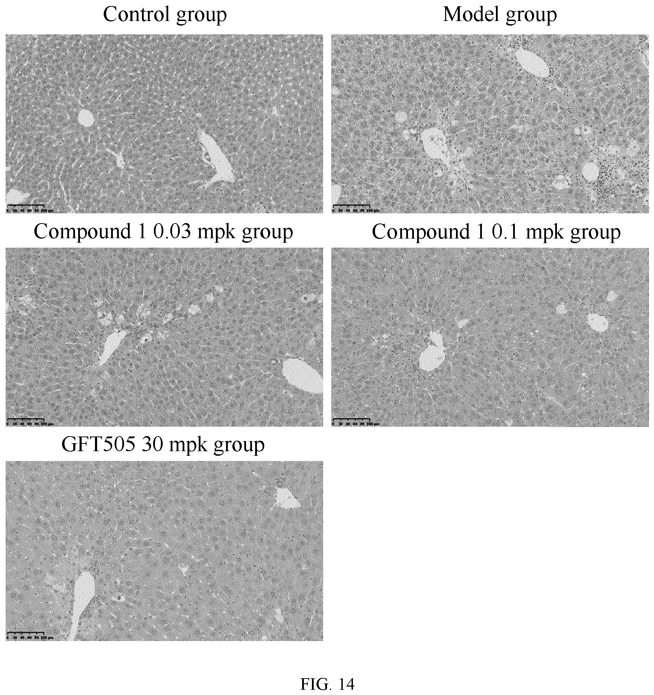

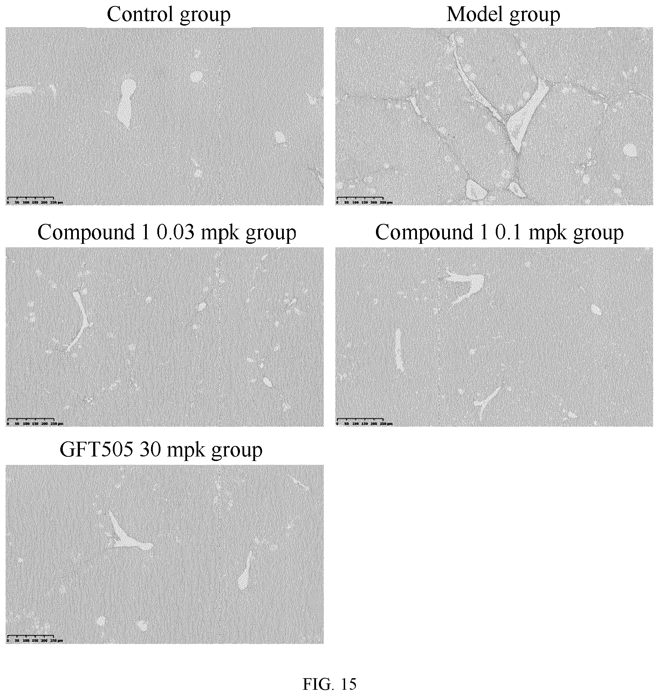

The anti-hepatic fibrosis effect of compound 1 was observed through a method in pathological research. The HE staining results (FIG. 14) show that compound 1 could reduce inflammatory infiltration in the mouse liver. Sirius red staining results (FIG. 15) show that compound 1 could significantly reduce collagen deposition in the mouse liver. It is noted that the improving effect of compound 1 at a dose of 0. 1 mg/kg on the collagen deposition in the mouse liver was superior to that of GFT505 at 10 mg/kg.

In conclusion, compound 1 had a protective effect on the hepatic fibrosis mouse model, suggesting that compound 1 has therapeutic effects on hepatic fibrosis-associated diseases and can be used for preparing medicaments for the prevention and treatment of hepatic fibrosis-associated diseases, liver cirrhosis, and other diseases. Other compounds of the present invention also have similar effects.

### Example 133

### Eutectic Structure of Complex of Compound 1 with PPAR6 Protein

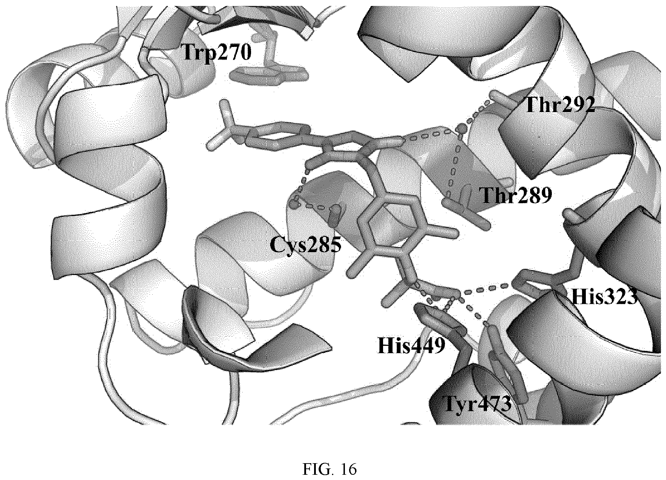

A plasmid expressing a protein comprising a PPARδ-LBD region (see Journal of Medicinal Chemistry 2022, 65, 2571-2592) was transformed into *E. coil* BL21. After culture and amplification, IPTG was added at 4 °C to induce protein expression. After *E. coil* was lysed, the supernatant was collected and purified via a nickel column. The purified protein was dissolved in a solution of 20 mM Tris, 150 mM NaCl, and 10% glycerol, pH 8.0. To the protein solution at a concentration of 7 mg/mL was added a solution of compound 1 in DMSO at a final concentration of 2 mM. An eutectic complex of compound 1 and the PPARδ protein was grown at 16 °C in a crystalline solvent that was a mixed solvent of 0.5 M sodium citrate, 19% PEG3350, and 20% glycerol, pH 5.5. The crystals were snap-frozen in liquid nitrogen for data collection. X-ray diffraction data were collected at beam line BL02U of Shanghai Synchrotron Radiation Facility with the help of an X-ray crystallography facility platform at the National Center for Protein Sciences (Tsinghua University). The data were processed with HKL2000 and solved by molecular replacement using the Phenix program, and the search model was PDB with the code of 3SP9. Modeling and refinement were performed using coot software and PHENIX software. The experimental results are shown in FIG. 16. The results indicate that the binding pattern of compound 1 to the PPAR6 protein is similar to that of an endogenous ligand fatty acid, and the carboxylic acid moiety of compound 1 has interactions through hydrogen bonds with His323, His449, and Tyr473 of the protein. Unlike other small molecular ligands, the hydantoin ring moiety of compound 1 can form interactions through hydrogen bonds with Cys285, Thr289, and Thr292 of the PPAR6 protein, respectively, via multiple "water molecule bridges". The interactions of this particular agonist with the PPARδ protein may be an important reason for the potent agonistic activity and high selectivity of compound 1.

### Example 134

### Tablet

Compound 1 (50 g) obtained in Example 1, hydroxypropyl methylcellulose E (150 g), starch (200 g), an appropriate amount of povidone K30, and magnesium stearate (1 g) were mixed, followed by granulating and tableting. In addition, the compounds prepared in Examples 1-123 can be prepared into capsules, powders, granules, pills, injections, syrups, oral liquids, inhalants, ointments, suppositories, patches, and the like by adding different pharmaceutical excipients according to conventional formulation methods of Pharmacopoeia (2015 Edition).

## Claims

1. A hydantoin compound of formula (I) or a pharmaceutically acceptable salt thereof, wherein: 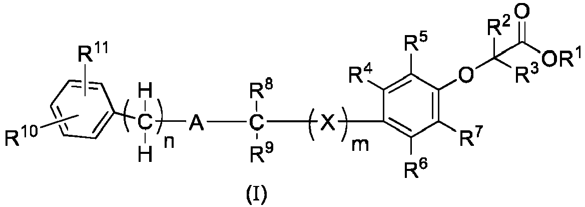
A is selected from 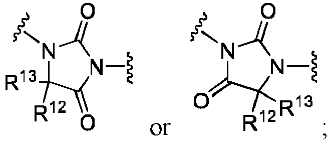
R¹ is selected from H, linear or branched C1-C6 alkyl, C3-C6 cycloalkyl, (CH₂)ₚOR¹⁴, or (CH₂)_{q}NR¹⁵, wherein p = any integer from 2 to 6; q = any integer from 2 to 6; R¹⁴ and R¹⁵ are each independently selected from H, R¹⁶, or C(O)R¹⁷, wherein R¹⁶ and R¹⁷ are each independently selected from linear or branched C1-C6 alkyl or C3-C6 cycloalkyl;
R² and R³ are each independently selected from H or linear or branched C1-C4 alkyl; or R² and R³, together with the carbon atom to which they are bonded, form a 3- to 6-membered cycloalkyl ring;
R⁴, R⁵, R⁶, and R⁷ are each independently selected from H, halogen, OR¹⁸, hydroxy, linear or branched C1-C4 alkyl, trifluoromethyl, methylthio, trifluoromethoxy, trifluoromethylthio, C3-C6 cycloalkyl, cycloalkenyl, heterocycloalkyl, heterocycloalkenyl, alkynyl, phenyl, substituted phenyl, heteroaryl, substituted heteroaryl, fused aryl, or substituted fused aryl; or at least two of substituents in R⁴, R⁵, R⁶, and R⁷, together with the atoms to which they are linked, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹⁸ is selected from linear or branched C1-C4 alkyl, hydroxyalkyl, alkoxyalkyl, alkoxyalkoxyalkyl, C3-C6 cycloalkyl, or alkynylalkoxyalkyl;
X is selected from CH₂, O, or S;
m is selected from any integer from 0 to 4;
n is selected from any integer from 0 to 2;
R⁸ and R⁹ are each independently selected from H, deuterium, linear or branched C1-C4 alkyl, or halogen; or R⁸ and R⁹, together with the carbon atom to which they are bonded, form a 3- to 6-membered cycloalkyl ring;
R¹⁰ and R¹¹ are independently selected from H, hydroxy, halogen, cyano, linear or branched C1-C4 alkyl, trifluoromethyl, methylthio, trifluoromethoxy, trifluoromethylthio, alkylsulfonyl, alkoxy, cycloalkyl, cycloalkenyl, heterocycloalkyl, heterocycloalkenyl, alkynyl, phenyl, substituted phenyl, phenoxy, substituted phenyloxy, heteroaryl, substituted heteroaryl, fused aryl, or substituted fused aryl, wherein the substituted phenyl, substituted phenyloxy, substituted heteroaryl, or substituted fused aryl may be independently substituted with 1 to 2 of the following substituents: halogen, hydroxy, cyano, linear or branched C1-C4 alkyl, trifluoromethyl, methylthio, trifluoromethoxy, trifluoromethylthio, or alkylsulfonyl; or R¹⁰ and R¹¹, together with the atoms to which they are linked, may form a substituted or unsubstituted benzene ring, a substituted or unsubstituted heteroaromatic ring, a substituted or unsubstituted cycloalkane ring, a substituted or unsubstituted heterocycloalkane ring, or a substituted or unsubstituted heterocycloalkene ring;
R¹² and R¹³ are each independently selected from H, deuterium, and linear or branched C1-C4 alkyl; or R¹² and R¹³, together with the carbon atom to which they are bonded, form a 3- to 6-membered cycloalkyl ring.

2. The hydantoin compound or the pharmaceutically acceptable salt thereof according to claim 1, wherein,
A is preferably selected from 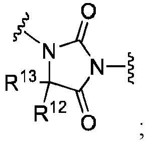
R¹ is selected from H, linear or branched C1-C4 alkyl, acetamidoethyl, or (CH₂)ₚOR¹⁴, wherein p = any integer from 2 to 6; R¹⁴ is selected from linear or branched C1-C4 alkyl;
R² and R³ are each independently selected from H or linear or branched C1-C4 alkyl; or R² and R³, together with the carbon atom to which they are bonded, form a 3- to 6-membered cycloalkyl ring;
R⁴, R⁵, R⁶, and R⁷ are each independently selected from H, halogen, trifluoromethyl, trifluoromethoxy, trifluoromethylthio, OR¹⁸, and linear or branched C1-C4 alkyl;
R¹⁸ is selected from linear or branched C1-C4 alkyl;
X is selected from CH₂;
m is selected from any integer from 0 to 2;
n is selected from 0 or 1;
R⁸ and R⁹ are each independently selected from H, deuterium, linear or branched C1-C4 alkyl, and halogen; or R⁸ and R⁹, together with the carbon atom to which they are bonded, form a 3-to 6-membered cycloalkyl ring;
R¹⁰ and R¹¹ are independently selected from H, halogen, cyano, linear or branched C1-C4 alkyl, trifluoromethyl, methylthio, trifluoromethoxy, trifluoromethylthio, methylsulfonyl, ethylsulfonyl, linear or branched C1-C4 alkoxy, C3-C6 cycloalkyloxy, C3-C6 cycloalkyl, phenyl, substituted phenyl, phenoxy, and substituted phenyloxy, wherein the substituted phenyl or substituted phenyloxy may independently be substituted with 1 to 2 of the following substituents: halogen, cyano, linear or branched C1-C4 alkyl, trifluoromethyl, methylthio, trifluoromethoxy, trifluoromethylthio, or methylsulfonyl;
R¹² and R¹³ are each independently selected from H, deuterium, and linear or branched C1-C4 alkyl.

3. The hydantoin compound or the pharmaceutically acceptable salt thereof according to claim 1, wherein the compound further comprises a prodrug, a deuterated compound, or a solvate thereof.

4. The hydantoin compound or the pharmaceutically acceptable salt thereof according to claim 1, wherein the compound or the pharmaceutically acceptable salt thereof is any one of the compounds shown as follows:
| Compound No. | Structure | Name |
|---|---|---|
| 1 | 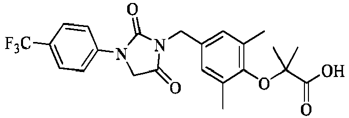 | 2-(4-((2,5-Dioxo-3-(4-(trifluoromethyl)phenyl)imid azolidin-1-yl)methyl)-2,6-dimethylphenoxy)-2-methylpropionic acid |
| 2 | 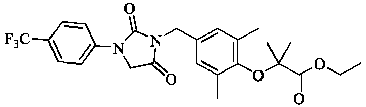 | Ethyl 2-(4-((2,5-dioxo-3-(4-(trifluoromethyl)phenyl)imid azolidin-1-yl)methyl)-2,6-dimethylphenoxy)-2-methylpropionate |
| 3 | 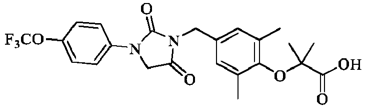 | 2-(4-((2,5-Dioxo-3-(4-(trifluoromethoxy)phenyl)im idazolidin-1-yl)methyl)-2,6-dimethylphenoxy)-2-methylpropionic acid |
| 4 | 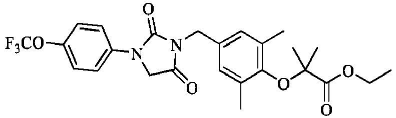 | Ethyl 2-(4-((2,5-dioxo-3-(4-(trifluoromethoxy)phenyl)im idazolidin-1-yl)methyl)-2,6-dimethylphenoxy)-2-methylpropionate |
| 5 | 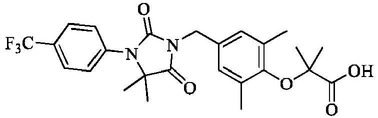 | 2-(4-((4,4-Dimethyl-2,5-dioxo-3-(4-(trifluoromethyl)phenyl)imid azolin-1-yl)methyl)-2,6-dimethylphenoxy)-2-methylpropionic acid |
| 6 | 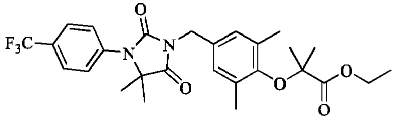 | Ethyl 2-(4-((4,4-dimethyl-2,5-dioxo-3-(4-(trifluoromethyl)phenyl)imid azolin-1-yl)methyl)-2,6-dimethylphenoxy)-2-methylpropionate |
| 7 | 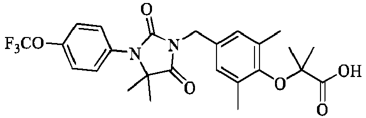 | 2-(4-((4,4-Dimethyl-2,5-dioxo-3-(4-(trifluoromethyl)phenyl)imid azolin-1-yl)methyl)-2,6-dimethylphenoxy)-2-methylpropionic acid |
| 8 | 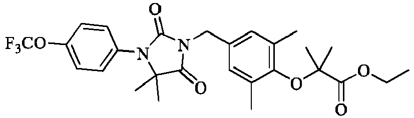 | Ethyl 2-(4-((4,4-dimethyl-2,5-dioxo-3-(4-(trifluoromethoxy)phenyl)im idazolin-1-yl)methyl)-2,6-dimethylphenoxy)-2-methylpropionate |
| 9 | 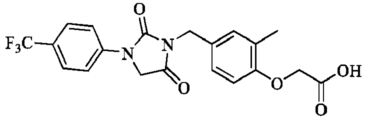 | 2-(4-((2,5-Dioxo-3-(4-(trifluoromethyl)phenyl)imid azolidin-1-yl)methyl)-2-methylphenoxy)acetic acid |
| 10 | 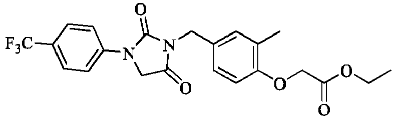 | Ethyl 2-(4-((2,5-dioxo-3-(4-(trifluoromethyl)phenyl)imid azolidin-1-yl)methyl)-2-methylphenoxy)acetate |
| 11 | 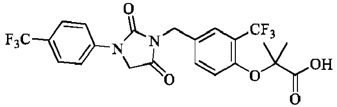 | 2-(4-((2,5-Dioxo-3-(4-(trifluoromethyl)phenyl)imid azolidin-1-yl)methyl)-2-(trifluoromethyl)phenoxy)-2-methylpropionic acid |
| 12 | 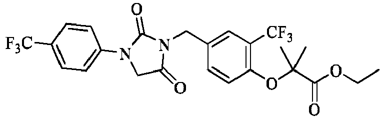 | Ethyl 2-(4-((2,5-dioxo-3-(4-(trifluoromethyl)phenyl)imid azolidin-1-yl)methyl)-2-(trifluoromethyl)phenoxy)-2-methylpropionate |
| 13 | 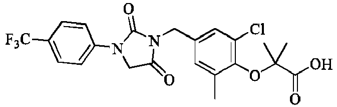 | 2-(2-Chloro-4-((2, 5-dioxo-3 - (4-(trifluoromethyl)phenyl)imid azolidin-1-yl)methyl)-6-methylphenoxy)-2-methylpropionic acid |
| 14 | 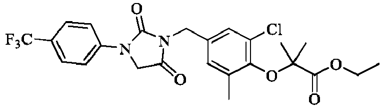 | Ethyl 2-(2-chloro-4-((2,5-dioxo-3-(4-(trifluoromethyl)phenyl)imid azolidin-1-yl)methyl)-6-methylphenoxy)-2-methylpropionate |
| 15 | 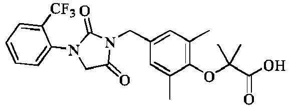 | 2-(4-((2,5-Dioxo-3-(2-(trifluoromethyl)phenyl)imid azolidin-1-yl)methyl)-2,6-dimethylphenoxy)-2-methylpropionic acid |
| 16 | 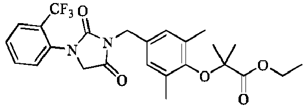 | Ethyl 2-(4-((2,5-dioxo-3-(2-(trifluoromethyl)phenyl)imid azolidin-1-yl)methyl)-2,6-dimethylphenoxy)-2-methylpropionate |
| 17 | 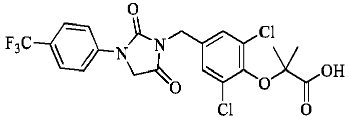 | 2-(2,6-Dichloro-4-((2,5-dioxo-3-(4-(trifluoromethyl)phenyl)imid azolidin-1-yl)methyl)phenoxy)-2-methylpropionic acid |
| 18 | 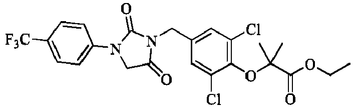 | Ethyl 2-(2,6-dichloro-4-((2,5-dioxo-3-(4- (trifluoromethyl)phenyl)imid azolidin-1- yl)methyl)phenoxy)-2-methylpropionate |
| 19 | 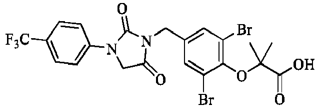 | 2-(2,6-Dibromo-4-((2,5-dioxo-3-(4-(trifluoromethyl)phenyl)imid azolidin-1-yl)methyl)phenoxy)-2-methylpropionic acid |
| 20 | 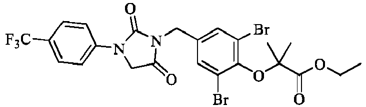 | Ethyl 2-(2,6-dibromo-4-((2,5-dioxo-3-(4-(trifluoromethyl)phenyl)imid azolidin-1- yl)methyl)phenoxy)-2-methylpropionate |
| 21 | 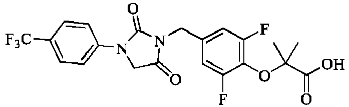 | 2-(4-((2,5-Dioxo-3-(4-(trifluoromethyl)phenyl)imid azolin-1-yl)methyl)-2,6- difluorophenoxy)-2- methylpropionic acid |
| 22 | 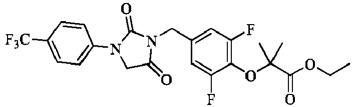 | Ethyl 2-(4-((2,5-dioxo-3-(4-(trifluoromethyl)phenyl)imid azolin-1-yl)methyl)-2,6- difluorophenoxy)-2- methylpropionate |
| 23 | 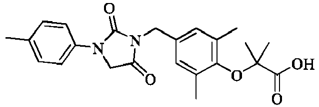 | 2-(4-((2,5-Dioxo-3-(p-tolyl)imidazolin-1- yl)methyl)-2,6- dimethylphenoxy)-2-methylpropionic acid |
| 24 | 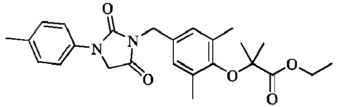 | Ethyl 2-(4-((2,5-dioxo-3-(p-tolyl)imidazolin-1- yl)methyl)-2,6- dimethylphenoxy)-2-methylpropionate |
| 25 | 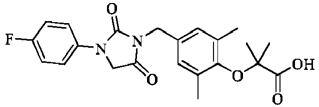 | 2-(4-((3 -(4-Fluorophenyl)-2,5-dioxoimidazolin-1-yl)methyl)-2,6- dimethylphenoxy)-2-methylpropionic acid |
| 26 | 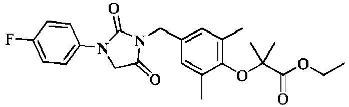 | Ethyl 2-(4-((3-(4-fluorophenyl)-2,5- dioxoimidazolin-1- yl)methyl)-2,6-dimethylphenoxy)-2-methylpropionate |
| 27 | 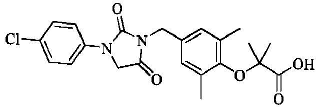 | 2-(4-((3 -(4-Chlorophenyl)-2, 5-dioxoimidazolin-1-yl)methyl)-2,6- dimethylphenoxy)-2-methylpropionic acid |
| 28 | 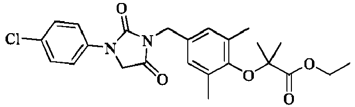 | Ethyl 2-(4-((3-(4-chlorophenyl)-2,5-dioxoimidazolin-1- yl)methyl)-2,6-dimethylphenoxy)-2-methylpropionate |
| 29 | 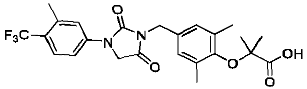 | 2-(2,6-Dimethyl-4-((3-(3-methyl-4-(trifluoromethyl)phenyl)-2,5-dioxoimidazolin-1-yl)methyl)phenoxy)-2-methylpropionic acid |
| 30 | 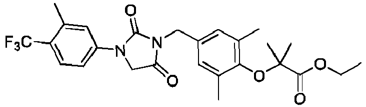 | Ethyl 2-(2,6-dimethyl-4-((3-(3-methyl-4-(trifluoromethyl)phenyl)-2,5-dioxoimidazolin-1-yl)methyl)phenoxy)-2-methylpropionate |
| 31 | 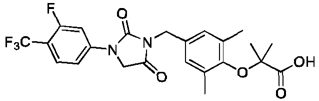 | 2-(2,6-Dimethyl-4-((3-(3-fluoro-4-(trifluoromethyl)phenyl)-2,5-dioxoimidazolin-1-yl)methyl)phenoxy)-2-methylpropionic acid |
| 32 | 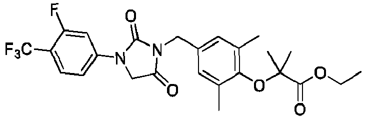 | Ethyl 2-(2,6-dimethyl-4-((3-(3-chloro-4-(trifluoromethyl)phenyl)-2,5-dioxoimidazolin-1-yl)methyl)phenoxy)-2-methylpropionate |
| 33 | 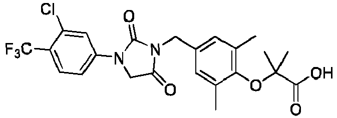 | 2-(2,6-Dimethyl-4-((3-(3-chloro-4-(trifluoromethyl)phenyl)-2,5-dioxoimidazolin-1-yl)methyl)phenoxy)-2-methylpropionic acid |
| 34 | 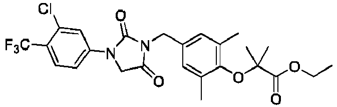 | Ethyl 2-(2,6-dimethyl-4-((3-(3-chloro-4-(trifluoromethyl)phenyl)-2,5-dioxoimidazolin-1-yl)methyl)phenoxy)-2-methylpropionate |
| 35 | 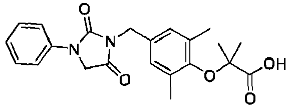 | 2-(4-((2,5-Dioxo-3- phenylimidazolin-1-yl)methyl)-2,6-dimethylphenoxy)-2-methylpropionic acid |
| 36 | 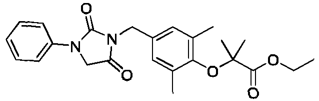 | Ethyl 2-(4-((2,5-dioxo-3-phenylimidazolin-1-yl)methyl)-2,6-dimethylphenoxy)-2-methylpropionate |
| 37 | 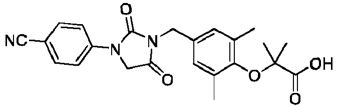 | 2-(4-((3 -(4-Cyanobenzene)-2,5-dioxoimidazolin-1-yl)methyl)-2,6-dimethylphenoxy)-2-methylpropionic acid |
| 38 | 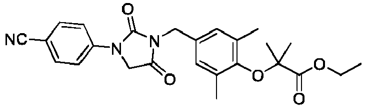 | Ethyl 2-(4-((3-(4-cyanobenzene)-2,5-dioxoimidazolin-1-yl)methyl)-2,6-dimethylphenoxy)-2-methylpropionate |
| 39 | 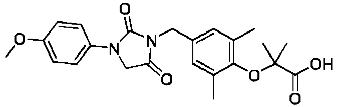 | 2-(4-((3-(4-Methoxyphenyl)-2,5-dioxoimidazolin-1-yl)methyl)-2,6-dimethylphenoxy)-2-methylpropionic acid |
| 40 | 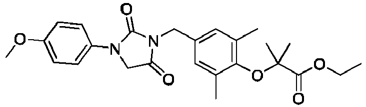 | Ethyl 2-(4-((3-(4-methoxyphenyl)-2,5-dioxoimidazolin-1-yl)methyl)-2,6-dimethylphenoxy)-2-methylpropionate |
| 41 | 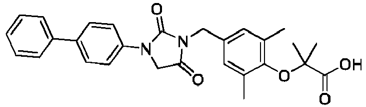 | 2-(4-((3-(4-Biphenyl-2,5-dioxoimidazolin-1-yl)methyl)-2,6-dimethylphenoxy)-2-methylpropionic acid |
| 42 | 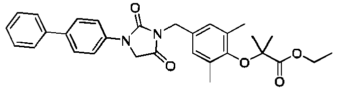 | Ethyl 2-(4-((3-(4-biphenyl-2,5-dioxoimidazolin-1-yl)methyl)-2,6-dimethylphenoxy)-2-methylpropionate |
| 43 | 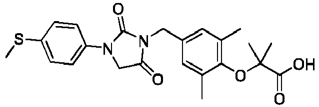 | 2-(4-((3-(4-Methylthiophenyl)-2,5- dioxoimidazolin-1-yl)methyl)-2,6- dimethylphenoxy)-2- methylpropionic acid |
| 44 | 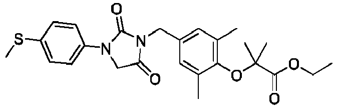 | Ethyl 2-(4-((3-(4-methylthiophenyl)-2,5- dioxoimidazolin-1-yl)methyl)-2,6-dimethylphenoxy)-2-methylpropionate |
| 45 | 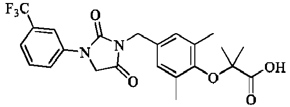 | 2-(4-((2,5-Dioxo-3-(3-(trifluoromethyl)phenyl)imid azolin-1-yl)methyl)-2,6- dimethylphenoxy)-2-methylpropionic acid |
| 46 | 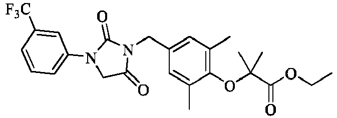 | Ethyl 2-(4-((2,5-dioxo-3-(3- (trifluoromethyl)phenyl)imid azolin-1-yl)methyl)-2,6-dimethylphenoxy)-2- methylpropionate |
| 47 | 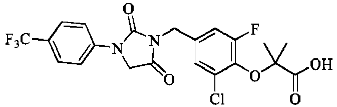 | 2-(2-Chloro-4-((2,5-dioxo-3- (4- (trifluoromethyl)phenyl)imid azolin-1-yl)methyl)-6-fluorophenoxy)-2-methylpropionic acid |
| 48 | 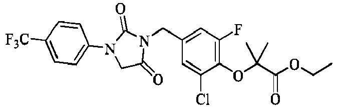 | Ethyl 2-(2-chloro-4-((2,5-dioxo-3-(4-(trifluoromethyl)phenyl)imid azolin-1-yl)methyl)-6-fluorophenoxy)-2-methylpropionate |
| 49 | 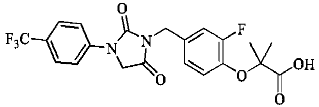 | 2-(4-((2,5-Dioxo-3-(4- (trifluoromethyl)phenyl)imid azolin-1-yl)methyl)-2-fluorophenoxy)-2-methylpropionic acid |
| 50 | 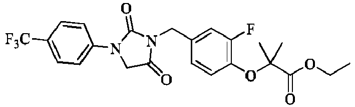 | Ethyl 2-(4-((2,5-dioxo-3-(4- (trifluoromethyl)phenyl)imid azolin-1-yl)methyl)-2-fluorophenoxy)-2-methylpropionate |
| 51 | 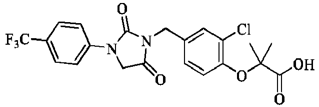 | 2-(4-((2,5-Dioxo-3-(4- (trifluoromethyl)phenyl)imid azolin-1-yl)methyl)-2-chlorophenoxy)-2-methylpropionic acid |
| 52 | 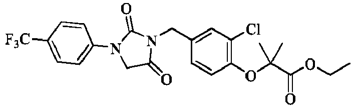 | Ethyl 2-(4-((2,5-dioxo-3-(4- (trifluoromethyl)phenyl)imid azolin-1-yl)methyl)-2-chlorophenoxy)-2-methylpropionate |
| 53 | 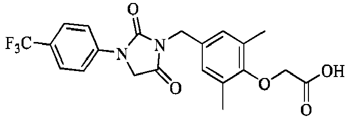 | 2-(4-((2,5-Dioxo-3-(4-(trifluoromethyl)phenyl)imid azolin-1-yl)methyl)-2,6-dimethylphenoxy)acetic acid |
| 54 | 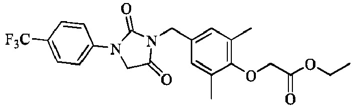 | Ethyl 2-(4-((2,5-dioxo-3-(4-(trifluoromethyl)phenyl)imid azolin-1-yl)methyl)-2,6-dimethylphenoxy)acetate |
| 55 | 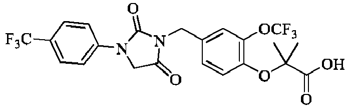 | 2-(4-((2,5-Dioxo-3-(4-(trifluoromethyl)phenyl)imid azolin-1-yl)methyl)-2-(trifluoromethoxy)phenoxy)-2-methylpropionic acid |
| 56 | 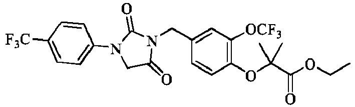 | Ethyl 2-(4-((2,5-dioxo-3-(4-(trifluoromethyl)phenyl)imid azolin-1-yl)methyl)-2-(trifluoromethoxy)phenoxy)-2-methylpropionate |
| 57 | 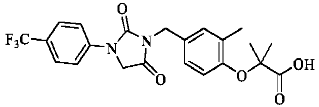 | 2-(4-((2,5-Dioxo-3-(4-(trifluoromethyl)phenyl)imid azolin-1-yl)methyl)-2-methylphenoxy)-2-methylpropionic acid |
| 58 | 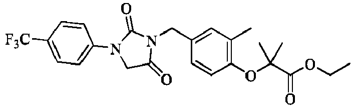 | Ethyl 2-(4-((2,5-dioxo-3-(4-(trifluoromethyl)phenyl)imid azolin-1-yl)methyl)-2-methylphenoxy)-2-methylpropionate |
| 59 | 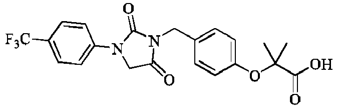 | 2-(4-((2,5-Dioxo-3-(4-(trifluoromethyl)phenyl)imid azolin-1-yl)methyl)phenoxy)-2-methylpropionic acid |
| 60 | 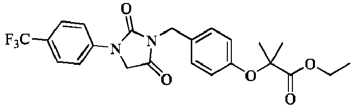 | Ethyl 2-(4-((2,5-dioxo-3-(4-(trifluoromethyl)phenyl)imid azolin-1-yl)methyl)phenoxy)-2-methylpropionate |
| 61 | 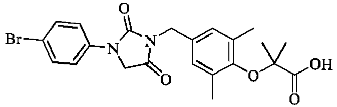 | 2-(4-((3-(4-Bromophenyl)-2,5-dioxoimidazolin-1-yl)methyl)-2,6-dimethylphenoxy)-2-methylpropionic acid |
| 62 | 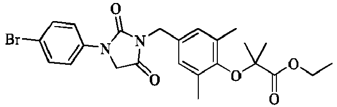 | Ethyl 2-(4-((3-(4-bromophenyl)-2,5-dioxoimidazolin-1-yl)methyl)-2,6-dimethylphenoxy)-2-methylpropionate |
| 63 | 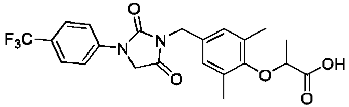 | 2-(4-((2,5-Dioxo-3-(4-(trifluoromethyl)phenyl)imid azolin-1-yl)methyl)-2,6-dimethylphenoxy)propionic acid |
| 64 | 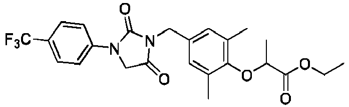 | Ethyl 2-(4-((2,5-dioxo-3-(4-(trifluoromethyl)phenyl)imid azolin-1-yl)methyl)-2,6-dimethylphenoxy)propionate |
| 65 | 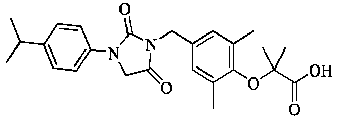 | 2-(4-((3-(4-Isopropylphenyl)-2,5-dioxoimidazolin-1-yl)methyl)-2,6-dimethylphenoxy)-2-methylpropionic acid |
| 66 | 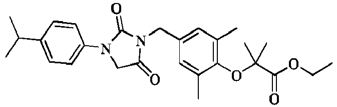 | Ethyl 2-(4-((3-(4-isopropylphenyl)-2,5- dioxoimidazolin-1-yl)methyl)-2,6-dimethylphenoxy)-2-methylpropionate |
| 67 | 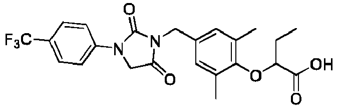 | 2-(4-((2,5-Dioxo-3-(4-(trifluoromethyl)phenyl)imid azolin-1-yl)methyl)-2,6-dimethylphenoxy)butyric acid |
| 68 | 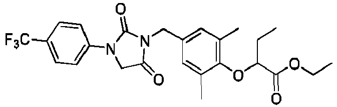 | Ethyl 2-(4-((2,5-dioxo-3-(4-(trifluoromethyl)phenyl)imid azolin-1-yl)methyl)-2,6-dimethylphenoxy)butyrate |
| 69 | 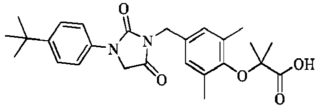 | 2-(4-((3-(4-(*tert-*Butyl)phenyl)-2,5-dioxoimidazolin-1-yl)methyl)-2,6-dimethylphenoxy)-2-methylpropionic acid |
| 70 | 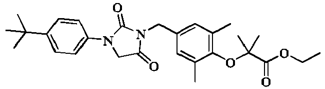 | Ethyl 2-(4-((3-(4-(*tert-*butyl)phenyl)-2,5-dioxoimidazolin-1-yl)methyl)-2,6-dimethylphenoxy)-2-methylpropionate |
| 71 | 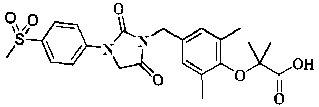 | 2-(2,6-Dimethyl-4-((3-(4-(methylsulfonyl)phenyl)-2,5-dioxoimidazolin-1-yl)methyl)phenoxy)-2-methylpropionic acid |
| 72 | 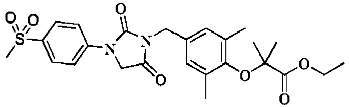 | Ethyl 2-(2,6-dimethyl-4-((3-(4-(methylsulfonyl)phenyl)-2,5-dioxoimidazolin-1-yl)methyl)phenoxy)-2-methylpropionate |
| 73 | 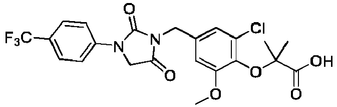 | 2-(2-Chloro-4-((2,5-dioxo-3- (4-(trifluoromethyl)phenyl)imid azolin-1-yl)methyl)-6- methoxyphenoxy)-2- methylpropionic acid |
| 74 | 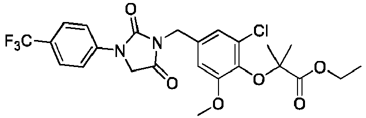 | Ethyl 2-(2-chloro-4-((2,5-dioxo-3-(4- (trifluoromethyl)phenyl)imid azolin-1-yl)methyl)-6-methoxyphenoxy)-2-methylpropionate |
| 75 | 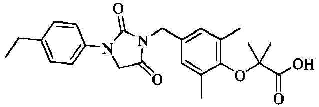 | 2-(4-(3-(4-Ethylphenyl)-2,5-dioxoimidazolidin-1-yl)methyl)-2,6-dimethylphenoxy-2-methylpropionic acid |
| 76 | 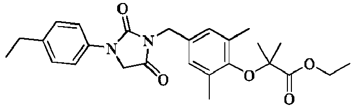 | Ethyl 2-(4-(3-(4-ethylphenyl)-2,5-dioxoimidazolidin-1-yl)methyl)-2,6-dimethylphenoxy-2- methylpropionate |
| 77 | 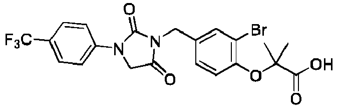 | 2-(2-Bromo-4-((2,5-dioxo-3-(4-trifluoromethyl)phenyl)imid azolidin-1-yl)methylphenoxy)-2-methylpropionic acid |
| 78 | 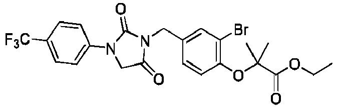 | Ethyl 2-(2-bromo-4-((2,5-dioxo-3-(4-trifluoromethyl)phenyl)imid azolidin-1-yl)methylphenoxy)-2-methylpropionate |
| 79 | 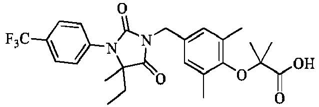 | 2-(4-((4-Ethyl-4-methyl-2,5-dioxo-3-(4-(trifluoromethyl)phenyl)imid azolin-1-yl)methyl)-2,6-dimethylphenoxy)-2-methylpropionic acid |
| 80 | 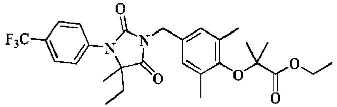 | Ethyl 2-(4-((4-ethyl-4-methyl-2,5-dioxo-3-(4-(trifluoromethyl)phenyl)imid azolin-1-yl)methyl)-2,6-dimethylphenoxy)-2-methylpropionate |
| 81 | 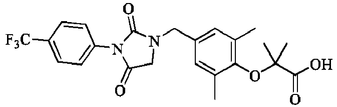 | 2-(4-((2,4-Dioxo-3-(4-(trifluoromethyl)phenyl)imid azolidin-1-yl)methyl)-2,6-dimethylphenoxy)-2-methylpropionic acid |
| 82 | 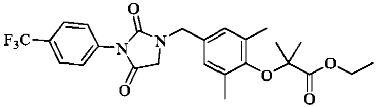 | Ethyl 2-(4-((2,4-dioxo-3-(4-(trifluoromethyl)phenyl)imid azolidin-1-yl)methyl)-2,6- dimethylphenoxy)-2-methylpropionate |
| 83 | 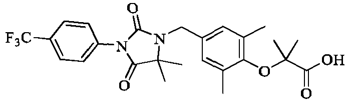 | 2-(4-((4,4-Dimethyl-2,5-dioxo-3-(4- (trifluoromethoxy)phenyl)im idazolidin-1-yl)methyl)-2,6-dimethylphenoxy)-2-methylpropionic acid |
| 84 | 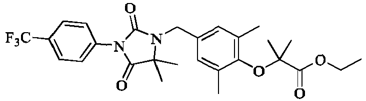 | Ethyl 2-(4-((4,4-dimethyl-2,5-dioxo-3-(4- (trifluoromethoxy)phenyl)im idazolidin-1-yl)methyl)-2,6-dimethylphenoxy)-2- methylpropionate |
| 85 | 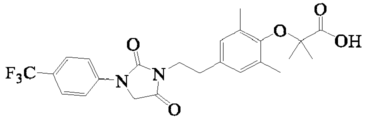 | 2-(4-(2-(2,5-Dioxo-3-(4-(trifluoromethyl)phenyl)imid azolidin-1-yl)ethyl)-2,6-dimethylphenoxy)-2-methylpropionic acid |
| 86 | 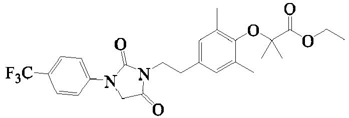 | Ethyl 2-(4-(2-(2,5-dioxo-3-(4-(trifluoromethyl)phenyl)imid azolidin-1-yl)ethyl)-2,6- dimethylphenoxy)-2-methylpropionate |
| 87 | 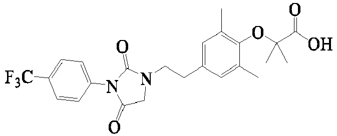 | 2-(4-(2-(2,4-Dioxo-3-(4- (trifluoromethyl)phenyl)imid azolidin-1-yl)ethyl)-2,6-dimethylphenoxy)-2-methylpropionic acid |
| 88 | 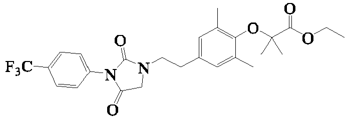 | Ethyl 2-(4-(2-(2,4-dioxo-3-(4-(trifluoromethyl)phenyl)imid azolidin-1-yl)ethyl)-2,6- dimethylphenoxy)-2- methylpropionate |
| 89 | 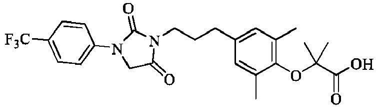 | 2-(4-(3-(2,5-Dioxo-3-(4-(trifluoromethyl)phenyl)imid azolidin-1-yl)propyl)-2,6-dimethylphenoxy)-2- methylpropionic acid |
| 90 | 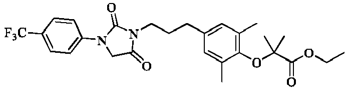 | Ethyl 2-(4-(3-(2,5-dioxo-3-(4-(trifluoromethyl)phenyl)imid azolidin-1-yl)propyl)-2,6-dimethylphenoxy)-2- methylpropionate |
| 91 | 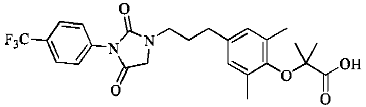 | 2-(4-(3-(2,4-Dioxo-3-(4- (trifluoromethyl)phenyl)imid azolidin-1-yl)propyl)-2,6- dimethylphenoxy)-2- methylpropionic acid |
| 92 | 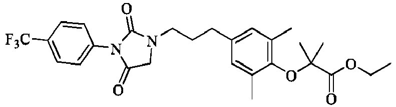 | Ethyl 2-(4-(3-(2,4-dioxo-3-(4-(trifluoromethyl)phenyl)imid azolidin-1-yl)propyl)-2,6- dimethylphenoxy)-2- methylpropionate |
| 93 | 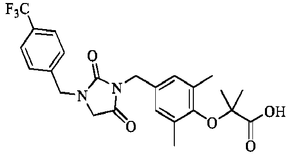 | 2-(4-((2,5-Dioxo-3-(4- (trifluoromethyl)phenyl)imid azolidin-1-yl)methyl)-2,6- dimethylphenoxy)-2- methylpropionic acid |
| 94 | 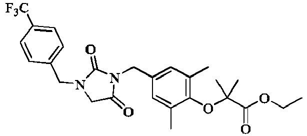 | Ethyl 2-(4-((2,5-dioxo-3-(4-(trifluoromethyl)phenyl)imid azolidin-1-yl)methyl)-2,6-dimethylphenoxy)-2- methylpropionate |
| 95 | 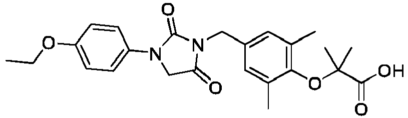 | 2-(4-(3-(4-Ethoxyphenyl)-2,5-dioxoimidazolidin-1-yl)methyl)-2,6-dimethylphenoxy)-2-methylpropionic acid |
| 96 | 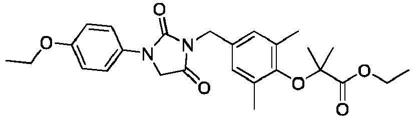 | Ethyl 2-(4-(3-(4-ethoxyphenyl)-2,5-dioxoimidazolidin-1-yl)methyl)-2,6-dimethylphenoxy)-2-methylpropionate |
| 97 | 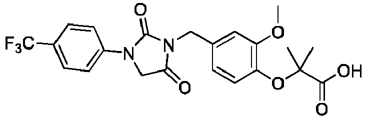 | 2-(4-(2,5-Dioxo-3-(4- trifluoromethylphenyl)imida zolidin-1-yl)methyl)-2-methoxyphenoxy)-2-methylpropionic acid |
| 98 | 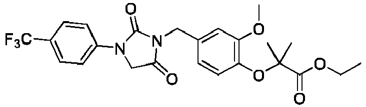 | Ethyl 2-(4-(2,5-dioxo-3-(4- trifluoromethylphenyl)imida zolidin-1-yl)methyl)-2-methoxyphenoxy)-2-methylpropionate |
| 99 | 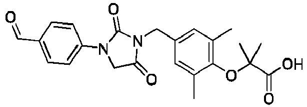 | 2-(4-(3-(4-Formylphenyl)-2,5-dioxoimidazolidin-1-yl)methyl)-2,6-dimethylphenoxy)-2-methylpropionic acid |
| 100 | 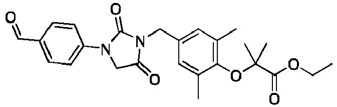 | Ethyl 2-(4-(3-(4-formylphenyl)-2,5-dioxoimidazolidin-1-yl)methyl)-2,6- dimethylphenoxy)-2- methylpropionate |
| 101 | 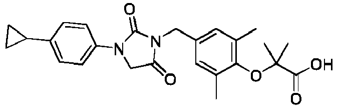 | 2-(3-(4-(Cyclopropylphenyl)-2,5-dioxoimidazolidin-1-yl)methyl)-2,6- dimethylphenoxy)-2-methylpropionic acid |
| 102 | 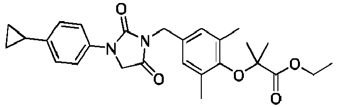 | Ethyl 2-(4-(3-(4-cyclopropylphenyl)-2,5-dioxoimidazolidin-1-yl)methyl)-2,6-dimethylphenoxy)-2-methylpropionate |
| 103 | 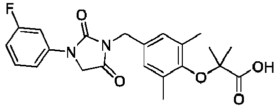 | 2-(4-(3-Fluorophenyl)-2,5-dioxoimidazolidin-1-yl)methyl)-2,6-dimethylphenoxy-2-methylpropionic acid |
| 104 | 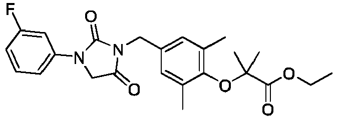 | Ethyl 2-(4-(3-(3-fluorophenyl)-2,5-dioxoimidazolidin-1-yl)methyl)-2,6-dimethylphenoxy)-2-methylpropionate |
| 105 | 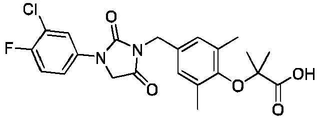 | 2-(3-Chloro-4-fluorophenyl)-2,5-dioxoimidazolidin-1-yl)methyl-2,6-dimethylphenoxy-2-methylpropionic acid |
| 106 | 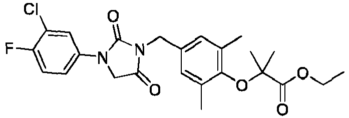 | Ethyl 2-(4-(3-chloro-4-fluorophenyl)-2,5-dioxoimidazolin-1-yl)methyl)-2,6-dimethylphenoxy-2-methylpropionate |
| 107 | 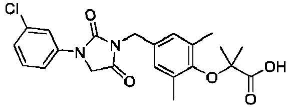 | 2-(4-((3-(3-Chlorophenyl)-2,5-dioxoimidazolidin-1-yl)methyl)-2,6-dimethylphenoxy-2-methylpropionic acid |
| 108 | 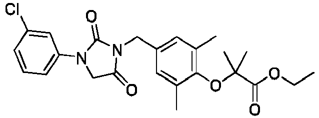 | Ethyl 2-(4-(3-chlorophenyl)-2,5-dioxoimidazolidin-1-yl)methyl)-2,6-dimethylphenoxy)-2-methylpropionate |
| 109 | 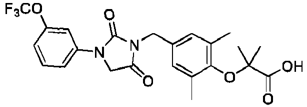 | 2-(4-(2,5-Dioxo-3-(3- trifluoromethoxyphenyl)imi dazolidin-1-yl)methyl)-2,6-dimethylphenoxy)-2-methylpropionic acid |
| 110 | 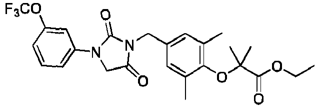 | Ethyl 2-(4-(2,5-dioxo-3-(3-(trifluoromethoxyphenyl)imi dazolidin-1-yl)methyl)-2,6-dimethylphenoxy)-2-methylpropionate |
| 111 | 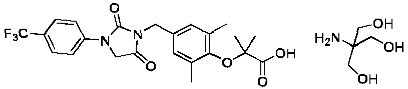 | Tromethamine 2-(4-(2,5-Dioxo-3-(4-trifluoromethylphenyl)imida zolidin-1-yl)methyl)-2,6-dimethylphenoxy)-2-methylpropionate |
| 112 | 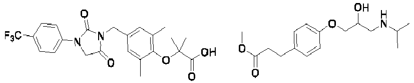 | Esmolol 2-(4-(2,5-dioxo-3-(4-(trifluoromethyl)phenyl)imid azolidin-1-yl)methyl)-2,6-dimethylphenoxy)-2-methylpropionate |
| 113 | 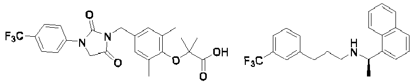 | Cinacalcet 2-(4-(2,5-dioxo-3-(4-trifluoromethylphenyl)imida zolidin-1-yl)methyl)-2,6-dimethylphenoxy)-2-methylpropionate |
| 114 | 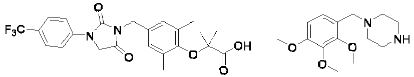 | Trimetazidine 2-(4-(2,5-dioxo-3-(4-trifluoromethylphenyl)imida zolin-1-yl)methyl)-2,6-dimethylphenoxy)-2-methylpropionate |
| 115 | 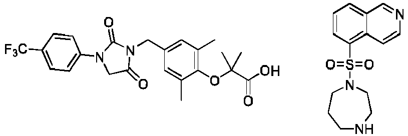 | Fasudil 2-(4-((2,5-dioxo-3-(4-(trifluoromethyl)phenyl)imid azolidinoxy-1-yl)methyl)-2,6-dimethylphenoxy)-2-methylpropionate |
| 116 | 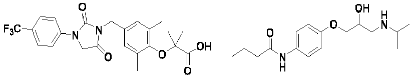 | Acebutolol 2-(2,5-dioxo-3-(4-(trifluoromethyl)phenyl)imid azolidin-1-yl)methyl)-2,6-dimethylphenoxy)-2-methylpropionate |
| 117 | 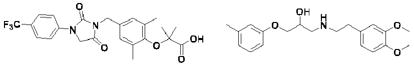 | Bevantolol 2-(4-(2,5-dioxo-3-(4-(trifluoromethyl)phenyl)imid azolidin-1-yl)methyl)-2,6-dimethylphenoxy)-2-methylpropionate |
| 118 | 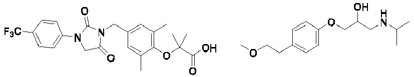 | Metoprolol 2-(4-(2,5-dioxo-3-(4-(trifluoromethyl)phenyl)imid azolidin-1-yl)methyl)-2,6-dimethylphenoxy)-2-methylpropionate |
| 119 | 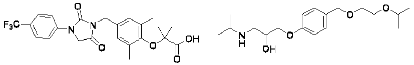 | Bisoprolol 2-(4-((2,5-dioxo-3-(4-(trifluoromethyl)phenyl))imidazolin-1-yl)methyl)-2,6-dimethylphenoxy)-2-methylpropionate |
| 120 | 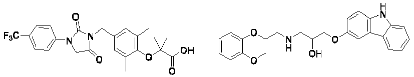 | Carvedilol 2-(4-(2,5-dioxo-3-(4-(trifluoromethyl)phenyl)imid azolidin-1-yl)methyl)-2,6-dimethylphenoxy)-2-methylpropionate |
| 121 | 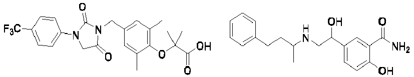 | Labetalol 2-(4-(2,5-dioxo-3-(4-(trifluoromethyl)phenyl)imid azolidin-1-yl)methyl)-2,6-dimethylphenoxy)-2-methylpropionate |
| 122 | 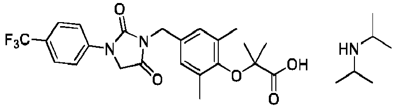 | Diisopropylamine 2-(4-(2,5-dioxo-3-(4-(trifluoromethyl)phenyl)imid azolidin-1-yl)methyl)-2,6-dimethylphenoxy-2-methylpropionate |
| 123 | 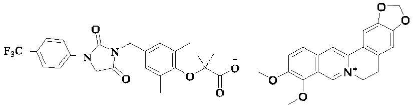 | Berberine 2-(4,5-dioxo-3-(4-(trifluoromethylphenyl)imid azolidin-1-yl)methyl)-2,6-dimethylphenoxy)-2-methylpropionate |

5. Use of the hydantoin compound or the pharmaceutically acceptable salt, the prodrug, the deuterated compound, or the solvate thereof according to any one of claims 1-4 in the preparation of a PPARα/δ dual agonist.

6. Use of the hydantoin compound or the pharmaceutically acceptable salt, the prodrug, the deuterated compound, or the solvate thereof according to any one of claims 1-4 in the preparation of a medicament for preventing or treating a disease mediated by PPARα and/or PPARδ.

7. The use according to claim 6, wherein the disease mediated by PPARα and/or PPARδ comprises metabolic diseases, cardiovascular and cerebrovascular diseases, inflammatory diseases, autoimmune diseases, organ fibrosis diseases, neurodegenerative diseases, secondary diseases caused by pathogen infections, mitochondrial dysfunction and disorder diseases, or tumors.

8. A salt of a hydantoin compound, wherein the salt of the hydantoin compound comprises a salt formed by the hydantoin compound according to any one of claims 1-4 with a metal ion or a pharmaceutically acceptable amine or an ammonium ion.

9. A pharmaceutical composition for preventing or treating a disease mediated by PPARα and/or PPARδ, comprising the hydantoin compound or the pharmaceutically acceptable salt, the prodrug, the deuterated compound, or the solvate thereof according to any one of claims 1-4 as an active ingredient, and a pharmaceutically acceptable carrier.

10. The pharmaceutical composition according to claim 9, wherein the pharmaceutical composition is a capsule, a powder, a tablet, a granule, a pill, an injection, a syrup, an oral liquid, an inhalant, an ointment, a suppository, or a patch.
